# EUROPEAN PATENT APPLICATION

(11) **EP 4 759 802 A1**
(43) Date of publication of application: **17.06.2026**
(21) Application number: 24872750.5
(22) Date of filing: 28.08.2024
(51) Int. Cl.: C07D 209/88, C07D 409/12, C07D 405/12, C07D 403/12, H10K 85/60

(54) **COMPOUND FOR ORGANIC ELECTRIC ELEMENT, ORGANIC ELECTRIC ELEMENT USING SAME, AND ELECTRONIC DEVICE HAVING SAME**

(30) Priority: 27.09.2023 KR 20230130846
(71) Applicant: Duk San Neolux Co., Ltd., Chungcheongnam-do 31027 (KR)
(72) Inventor: YUN, Bu Yong, Cheonan-si, Chungcheongnam-do 31027 (KR); LEE, Se Hoon, Cheonan-si, Chungcheongnam-do 31027 (KR); LEE, Hyung Dong, Cheonan-si, Chungcheongnam-do 31027 (KR)
(74) Representative: Isarpatent
(86) International application number: PCT/KR2024/012899
(87) International publication number: WO 2025/071042

(57) **Abstract**

The present invention provides a novel compound that can improve the luminous efficiency, stability, and lifespan of the element, a composition comprising the same and an organic electronic element using the same, and an electronic device thereof.

## Description

### BACKGROUND

### [Technical Field]

The present invention relates to compounds for organic electronic elements, organic electronic elements using the same, and an electronic device thereof.

### [Background Art]

In general, organic light emitting phenomenon refers to a phenomenon that converts electric energy into light energy by using an organic material. An organic electronic element using an organic light emitting phenomenon usually has a structure including an anode, a cathode, and an organic material layer interposed therebetween. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer etc.

A material used as an organic material layer in an organic electronic element may be classified into a light emitting material and a charge transport material, such as a hole injection material, a hole transport material, an electron transport material, an electron injection material etc. depending on its function. And the light emitting material can be classified into a high molecular weight type and a low molecular weight type according to the molecular weight, and it can be classified into a fluorescent material derived from a singlet excited state of an electron and a phosphorescent material derived from a triplet excited state of an electron depending on the light emission mechanism. Further, the light emitting material may be divided into blue, green, and red light emitting materials and yellow and orange light emitting materials necessary for realizing a better natural color according to the emission color.

However, when only one material is used as a light emitting material, due to intermolecular interaction, the maximum emission wavelength shifts to a longer wavelength, and there are problems in that the color purity is lowered or the device efficiency is reduced due to the emission attenuation effect, therefore in order to increase color purity and increase luminescence efficiency through energy transfer, a host/dopant system may be used as a light emitting material. The principle is that when a small amount of a dopant having a smaller energy band gap than that of the host forming the emitting layer is mixed in the emitting layer, excitons generated in the emitting layer are transported to the dopant to emit light with high efficiency. Here, since the wavelength of the host moves to the wavelength band of the dopant, light having a desired wavelength can be obtained according to the type of dopant used.

Currently, the portable display market is a large-area display, and the size thereof is increasing, and thus, more power consumption than the power consumption required for the existing portable display is required. Therefore, power consumption has become a very important factor for a portable display having a limited power supply such as a battery, and the problem of efficiency and lifetime must also be solved.

Efficiency, lifespan, and driving voltage are interrelated. As efficiency increases, the driving voltage decreases relatively. As the driving voltage decreases, the crystallization of organic materials due to Joule heating generated during operation decreases, which results in a tendency for the lifespan to increase. However, the efficiency cannot be maximized simply by improving the organic material layer. This is because, when the energy level and T1 value between each organic material layer, and the intrinsic properties (mobility, interfacial properties, etc.) of materials are optimally combined, long lifetime and high efficiency can be achieved at the same time.

Therefore, while delaying the penetration and diffusion of metal oxide from the anode electrode (ITO) into the organic layer, which is one of the causes of shortening the lifetime of the organic electronic element, it should have stable characteristics against Joule heating generated during device driving, and OLED devices are mainly formed by a deposition method, and it is necessary to develop a material that can withstand a long time during deposition, that is, a material with strong heat resistance.

That is, in order to fully exhibit the excellent characteristics of an organic electronic element, it should be preceded that the material constituting the organic material layer in the device, such as a hole injection material, a hole transport material, a light emitting material, an electron transport material, an electron injection material, etc., is supported by a stable and efficient material. But the development of a stable and efficient organic material layer material for an organic electronic element has not yet been sufficiently made. Therefore, the development of new materials is continuously required, and in particular, the development of a host material for the emitting layer is urgently needed.

### [DETAILED DESCRIPTION OF THE INVENTION]

### [Summary]

In order to solve the problems of the above-mentioned background technology, the present invention has discovered a compound with a novel structure, and also discovered that when the compound is applied to an organic electronic element, the luminescence efficiency, stability, and lifetime of the element can be greatly improved.

Accordingly, the purpose of the present invention is to provide a novel compound, an organic electronic element using the same, and an electronic device thereof.

### [Technical Solution]

The present invention provides a compound represented by Formula 1.

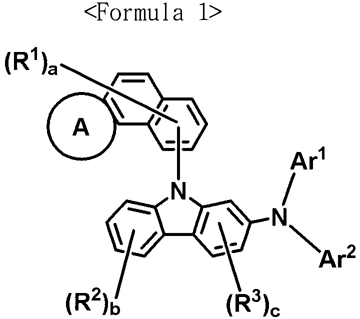

In another aspect, the present invention provides a composition for a phosphorescent light-emitting layer of an organic electric element comprising a mixture of a compound represented by Formula 1 and a compound represented by Formula 2.

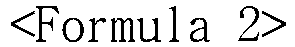

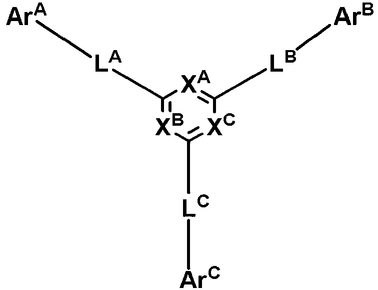

In another aspect, the present invention provides an organic electronic element comprising a compound represented by Formula 1 or a material for an organic electric element and an electronic device thereof.

### [Effects of the Invention]

By using the compound according to the present invention, high luminescence efficiency, low driving voltage and high heat resistance of the element can be achieved, and color purity and lifetime of the element can be greatly improved.

### [BRIEF DESCRIPTION OF THE DRAWINGS]

FIG.1 to 3 are exemplary views of an organic electroluminescent device according to the present invention.
FIG.4 is a Formula according to one aspect of the present invention.

| | | | |
|---|---|---|---|
| 100, 200, 300: | organic electronic element | 110 : | the first electrode |
| 120 : | hole injection layer | 130 : | hole transport layer |
| 140 : | emitting layer | 150 : | electron transport layer |
| 160 : | electron injection layer | 170 : | second electrode |
| 180 : | light efficiency enhancing Layer | 210 : | buffer layer |
| 220 : | emitting auxiliary layer | 320 : | first hole injection layer |
| 330 : | first hole transport layer | 340 : | first emitting layer |
| 350 : | first electron transport layer | 360 : | first charge generation layer |
| 361 : | second charge generation layer | 420 : | second hole injection layer |
| 430 : | second hole transport layer | 440 : | second emitting layer |
| 450 : | second electron transport layer | CGL : | charge generation layer |
| ST1 : | first stack | ST2 : | second stack |

### [DETAILED DESCRIPTION]

Hereinafter, some embodiments of the present invention will be described in detail. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used to define an essence, order or sequence of a corresponding component but used merely to distinguish the corresponding component from other component(s). It should be noted that if a component is described as being "connected", "coupled", or "connected" to another component, the component may be directly connected or connected to the other component, but another component may be "connected ", " coupled" or "connected" between each component.

As used in the specification and the accompanying claims, unless otherwise stated, the following is the meaning of the term as follows.

Unless otherwise stated, the term "halo" or "halogen" , as used herein, includes fluorine(F), bromine(Br), chlorine(Cl), or iodine(I).

Unless otherwise stated, the term "alkyl" or "alkyl group" , as used herein, has a single bond of 1 to 60 carbon atoms, 1 to 30 carbon atoms, 1 to 25 carbon atoms, 1 to 18 carbon atoms, or 1 to 12 carbon atoms, and means saturated aliphatic functional radicals including a linear alkyl group, a branched chain alkyl group, a cycloalkyl group (alicyclic), an cycloalkyl group substituted with a alkyl or an alkyl group substituted with a cycloalkyl. Unless otherwise stated, the term "alkenyl" or "alkynyl" , as used herein, has double or triple bonds of 2 to 60 carbon atoms, 2 to 30 carbon atoms, 2 to 25 carbon atoms, 2 to 18 carbon atoms, 2 to 12 carbon atoms, but is not limited thereto, and includes a linear or a branched chain group.

Unless otherwise stated, the term "cycloalkyl" , as used herein, means alkyl forming a ring having 3 to 60 carbon atoms, 3 to 30 carbon atoms, 3 to 25 carbon atoms, 3 to 18 carbon atoms, 3 to 12 carbon atoms, but is not limited thereto.

Unless otherwise stated, the term "alkoxyl group" , "alkoxy group" or "alkyloxy group" , as used herein, means an alkyl group bonded to oxygen radical, but is not limited thereto, and has 1 to 60 carbon atoms, 1 to 30 carbon atoms, 1 to 25 carbon atoms, 1 to 18 carbon atoms, or 1 to 12 carbon atoms.

Unless otherwise stated, the term "aryloxyl group" or "aryloxy group" , as used herein, means an aryl group bonded to oxygen radical, but is not limited thereto, and has 6 to 60 carbon atoms, 6 to 30 carbon atoms, 6 to 25 carbon atoms, 6 to 18 carbon atoms, or 6 to 12 carbon atoms.

Unless otherwise specified, the terms "aryl group" and "arylene group" used in the present invention have 6 to 60 carbon atoms, 6 to 30 carbon atoms, 6 to 25 carbon atoms, 6 to 18 carbon atoms, or 6 to 12 carbon atoms, respectively, but are not limited thereto. In the present invention, an aryl group or arylene group refers to an aromatic group of a single ring or multiple rings, and comprises an aromatic ring formed by the bonding or reaction of adjacent substituents. For example, the aryl group may be phenyl, biphenyl, a fluorene group or a spirofluorene group.

The prefix "aryl" or "ar" means a radical substituted with an aryl group. For example, an arylalkyl may be an alkyl substituted with an aryl, and an arylalkenyl may be an alkenyl substituted with aryl, and a radical substituted with an aryl has a number of carbon atoms as defined herein.

Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heterocyclic group" , as used herein, contains one or more heteroatoms, and has 2 to 60 carbon atoms, 2 to 30 carbon atoms, 2 to 25 carbon atoms, 2 to 18 carbon atoms, 2 to 12 carbon atoms, and comprises any one of a single ring or multiple ring, and may include heteroaliphatic ring and heteroaromatic ring. Also, the heterocyclic group may also be formed in conjunction with an adjacent group.

Unless otherwise stated, the term "heteroatom" , as used herein, represents at least one of N, O, S, P, or Si.

Additionally, "heterocyclic group" means a single ring, ring aggregate, fused multiple ring system, spiro compound, etc. containing a heteroatom. Also, compounds containing heteroatom groups such as SO₂, P=O, etc. instead of carbon forming a ring, such as the compounds below, can also be included in the heterocyclic group. For example, a "heterocyclic group" includes the following compound.

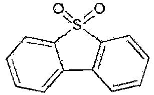

The term "aliphatic ring group" used in the present invention refers to cyclic hydrocarbons excluding aromatic hydrocarbons, and includes single rings, ring aggregates, fused multiple ring systems, spiro compounds, etc., and means a ring having 3 to 60 carbon atoms, 3 to 30 carbon atoms, 3 to 25 carbon atoms, 3 to 18 carbon atoms, 3 to 12 carbon atoms, but is not limited thereto. For example, even when benzene, an aromatic ring, and cyclohexane, a non-aromatic ring, are fused, it is an aliphatic ring.

Unless otherwise stated, the term "fluorenyl group" , "fluorenylene group" or "fluorentriyl group" as used herein, means a monovalent, divalent or trivalent functional group, in which R, R' and R" are all hydrogen in the following structures, and the term "substituted fluorenyl group" , "substituted fluorenylene group" or "substituted fluorentriyl group" means that at least one of the substituents R, R' and R" is a substituent other than hydrogen, and include those in which R and R' are bonded to each other to form a spiro compound together with the carbon to which they are bonded. In this specification, fluorenyl group, fluorenylene group, and fluorenetriyl group may all be referred to as fluorene groups, regardless of valence.

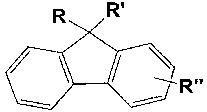

The term "spiro compound" used in the present invention has a 'spiro union', and a spiro union means a connection formed by 2 rings sharing only one atom. At this time, the atom shared between the 2 rings is called a 'spiro atom', and depending on the number of spiro atoms contained in a compound, they are called 'monospiro-', 'dispiro-', and 'trispiro-' compounds, respectively.

Unless otherwise stated, the term "aliphatic" as used herein means an aliphatic hydrocarbon having 1 to 60 carbon atoms, 1 to 30 carbon atoms, 1 to 25 carbon atoms, 1 to 18 carbon atoms or 1 to 12 carbon atoms, and "aliphatic ring" means an aliphatic hydrocarbon ring having 3 to 60 carbon atoms, 3 to 30 carbon atoms, 3 to 25 carbon atoms, 3 to 18 carbon atoms or 3 to 12 carbon atoms.

Unless otherwise stated, the term "ring" , as used herein, means an aliphatic ring having 3 to 60 carbon atoms, 3 to 30 carbon atoms, 3 to 25 carbon atoms, 3 to 18 carbon atoms or 3 to 12 carbon atoms; or an aromatic ring having 6 to 60 carbon atoms, 6 to 30 carbon atoms, 6 to 25 carbon atoms, 6 to 18 carbon atoms, or 6 to 12 carbon atoms; or a heterocyclic having 2 to 60 carbon atoms, 2 to 30 carbon atoms, 2 to 25 carbon atoms, 2 to 18 carbon atoms, 2 to 12 carbon atoms, or a fused ring formed by the combination thereof, and includes a saturated or unsaturated ring.

Other hetero compounds or hetero radicals other than the above-mentioned hetero compounds include, but are not limited thereto, one or more heteroatoms.

Also, unless expressly stated, as used herein, "substituted" in the term "substituted or unsubstituted" means substituted with one or more substituents selected from the group consisting of deuterium, halogen, an amino group, a nitrile group, a nitro group, a C₁-C₂₀ alkyl group, a C₁-C₂₀ alkoxyl group, a C₁-C₂₀ alkylamine group, a C₁-C₂₀ alkylthiopen group, a C₆-C₂₀ arylthiopen group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₆-C₂₀ arylalkenyl group, a silane group, a boron group, a germanium group, and a C₂-C₂₀ heterocyclic group, but is not limited to these substituents.

In this specification, the 'group name' corresponding to the aryl group, arylene group, heterocyclic group, etc., as examples of each symbol and its substituent, may be written as the 'name of the group reflecting the valence', but is written as the 'parent compound name'. For example, in the case of 'phenanthrene', a type of aryl group, the name of the group may be written by distinguishing the valence, such as the monovalent 'group' is 'phenanthryl' and the divalent group is 'phenanthrylene', but may be written as 'phenanthrene', which is the name of the parent compound, regardless of the valence. Similarly, in the case of pyrimidine, it can be written as 'pyrimidine' regardless of the valence, or it can be written as the 'name of the group' of the valence, such as pyrimidineyl group in the case of monovalent group, pyrimidineylene in the case of divalent group, etc. Additionally, in this specification, when describing compound names or substituent names, numbers or alphabets indicating positions may be omitted. For example, pyrido[4,3-d]pyrimidine to pyridopyrimidine, benzofuro[2,3-d]pyrimidine to benzofuropyrimidine, 9,9-dimethyl-9H-fluorene can be described as dimethylfluorene, etc. Therefore, both benzo[g]quinoxaline and benzo[f]quinoxaline can be described as benzoquinoxaline.

Also, unless there is an explicit explanation, the formula used in the present invention is the same as the definition of the substituent by the exponent definition of the following formula.

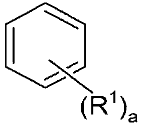

Here, when a is an integer of 0, the substituent R¹ is absent, when a is an integer of 1, the sole substituent R¹ is linked to any one of the carbon constituting the benzene ring, when a is an integer of 2 or 3, each is combined as follows, where R¹ may be the same or different from each other, when a is an integer of 4 to 6, it is bonded to the carbon of the benzene ring in a similar manner, while the indication of the hydrogen bonded to the carbon forming the benzene ring is omitted.

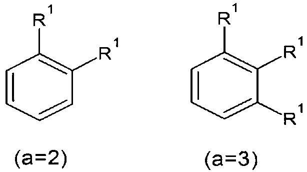

Unless otherwise expressly stated, the terms "ortho", "meta", and "para" used in the present invention refer to the substitution positions of all substituents, and the ortho position refers to a compound in which the position of the substituent is immediately adjacent, for example, when benzene is used, it means 1 or 2 position, and the meta position is the next substitution position of the neighbor substitution position, when benzene as an example stands for 1 or 3 position, and the para position is the next substitution position of the meta position, which means 1 and 4 position when benzene is taken as an example. A more detailed example of the substitution position is as follows, and it can be confirmed that the ortho-, and meta- position are substituted by non-linear type and para- positions are substituted by linear type.

### [Example of ortho-position]

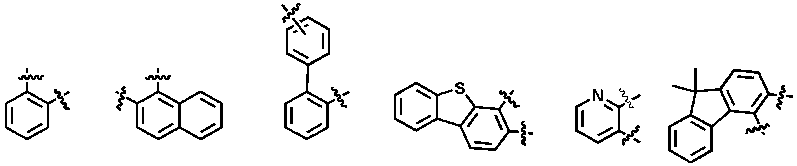

### [Example of meta-position]

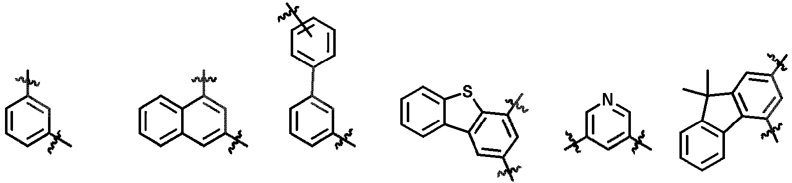

### [Example of para-position]

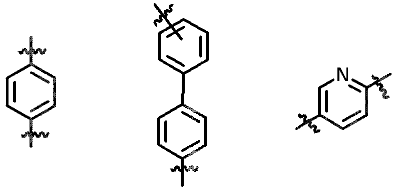

The term "composition" as used in the present invention is intended to be broadly interpreted to include not only compounds but also solutions, dispersions, liquid and solid mixtures (mixtures, admixtures). The composition of the present invention may contain the compound of the present invention alone, or may contain 2 or more different compounds in combination, or may contain the compound in combination with 2 or more other compounds. In other words, the composition may comprise a compound corresponding to Formula 1 alone, may comprise a mixture of 2 or more compounds of Formula 1, or may comprise a mixture of a compound of Formula 1 and a compound not corresponding to the present invention. Wherein, the compound not corresponding to the present invention may be a single compound or 2 or more compounds. Here, when the compound is contained in a combination of 2 or more other compounds, the other compounds may be already known compounds of each organic material layer or compounds to be developed in the future. Wherein, the compound contained in the organic material layer may be composed of only the same type of compound, but may also be a mixture of 2 or more types of heterogeneous compounds represented by Formula 1.

Hereinafter, a compound according to one aspect of the present invention, a composition for a phosphorescent light-emitting layer of an organic electric element and an organic electronic element including the same will be described.

The present invention provides a compound represented by Formula 1. 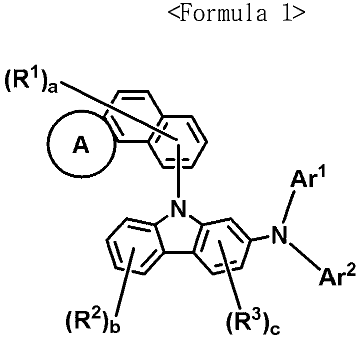 wherein:
Ring A is a C₁₀ aryl group,
R¹, R² and R³ are independently the same or different from each other, and are independently selected from the group consisting of hydrogen; deuterium;
a C₆-C₆₀ aryl group; a fluorenyl group; a C₂-C₆₀ heterocyclic group including at least one heteroatom of O, N, S, Si or P; a C₁-C₅₀ alkyl group; a C₂-C₂₀ alkenyl group; a C₂-C₂₀ alkynyl group; a C₁-C₃₀ alkoxyl group; and a C₆-C₃₀ aryloxy group; or a plurality of adjacent R² and R³ are may be bonded to each other to form an aromatic ring,

Wherein when R¹, R² and R³ are an aryl group, preferably an C₆-C₃₀ aryl group, more preferably an C₆-C₂₅ aryl group, an C₆-C₁₈ aryl group or an C₆-C₁₂ aryl group, such as phenyl, biphenyl, terphenyl, naphthalene, phenanthrene, chrysene, etc.

Wherein when R¹, R² and R³ are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.

Wherein when R¹, R² and R³ are an alkyl group, preferably a C₁-C₃₀ alkyl group, more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.

Wherein when R¹, R² and R³ are an alkoxyl group, preferably a C₁-C₂₅ alkoxyl group, more preferably a C₁-C₁₈ alkoxyl group, or a C₁-C₁₂ alkoxyl group,

Wherein when R¹, R² and R³ are an aryloxy group, preferably an C₆-C₂₅ aryloxy group, more preferably an C₆-C₁₈ aryloxy group, or an C₆-C₁₂ aryloxy group.

Ar¹ and Ar² are independently selected from the group consisting of a C₆-C₆₀ aryl group; a fluorenyl group; a C₂-C₆₀ heterocyclic group including at least one heteroatom of O, N, S, Si or P; and a fused ring group of a C₃-C₆₀ aliphatic ring and a C₆-C₆₀ aromatic ring;

Wherein when Ar¹ and Ar² are an aryl group, preferably an C₆-C₃₀ aryl group, more preferably an C₆-C₂₅ aryl group, an C₆-C₁₈ aryl group or an C₆-C₁₂ aryl group, such as phenyl, biphenyl, terphenyl, naphthalene, phenanthrene, chrysene, etc.

Wherein when Ar¹ and Ar² are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.

Wherein when Ar¹ and Ar² are a fused ring group, preferably a fused ring group of a C₃-C₃₀ aliphatic ring and a C₆-C₃₀ aromatic ring, more preferably a fused ring group of a C₃-C₂₅ aliphatic ring and a C₆-C₂₅ aromatic ring.
a is an integer of 0 to 11, b is an integer of 0 to 4, c is an integer of 0 to 3,
wherein, the aryl group and heterocyclic group may be further substituted with one or more substituents selected from the group consisting of deuterium, a C₁-C₂₀ alkyl group; a C₆-C₂₀ aryl group; a C₆-C₂₀ aryl group substituted with deuterium; a C₂-C₂₀ heterocyclic group.

Also, Formula 1 is represented by any one of the following Formulas 3 to 10. 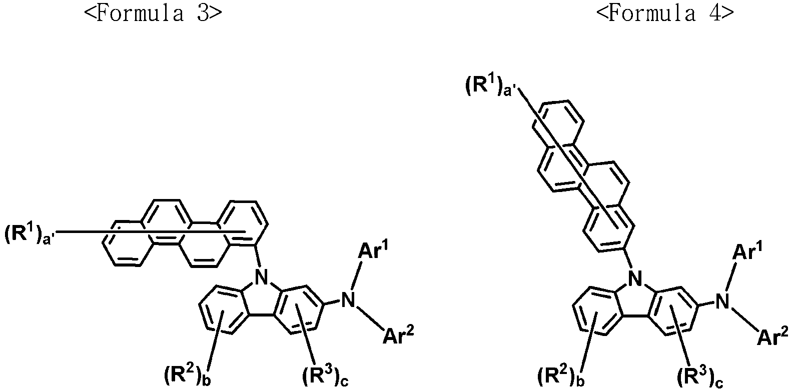 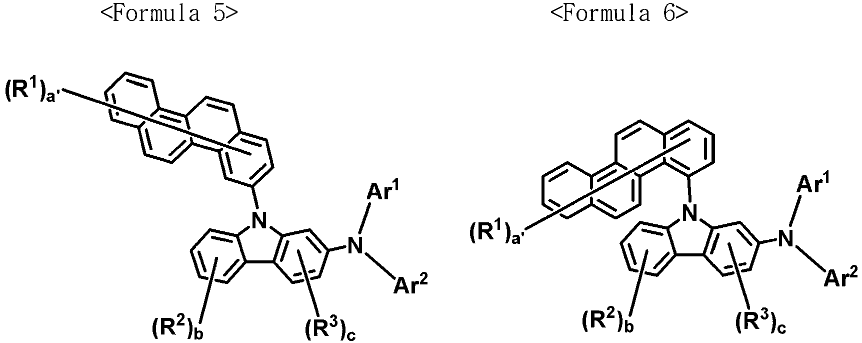 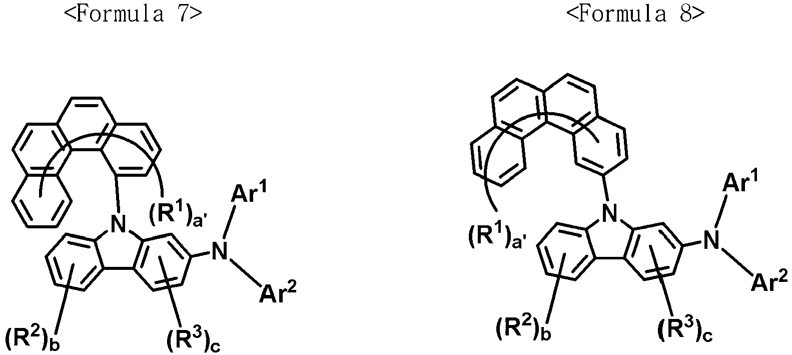 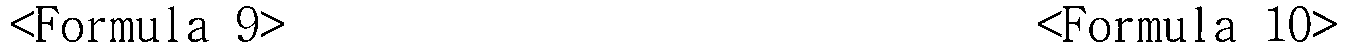 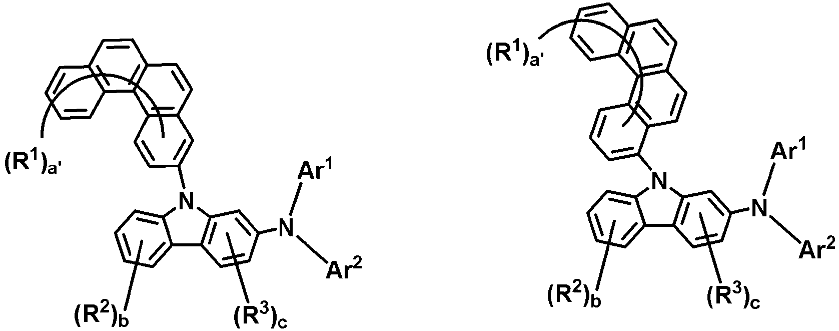 wherein:
R¹, R², R³, Ar¹, Ar², b and c are the same as defined above,
a' is an integer of 0 to 11.

Also, Ar¹ and Ar² are represented by any one of the following Formulas a-1 to a-15. 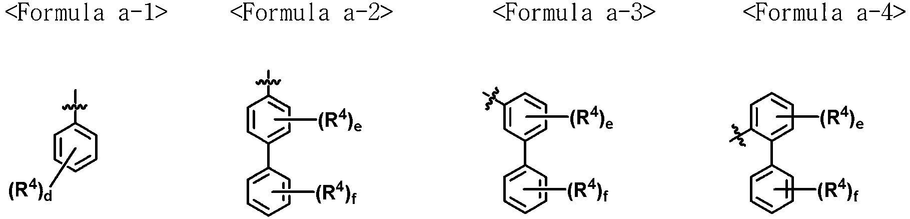 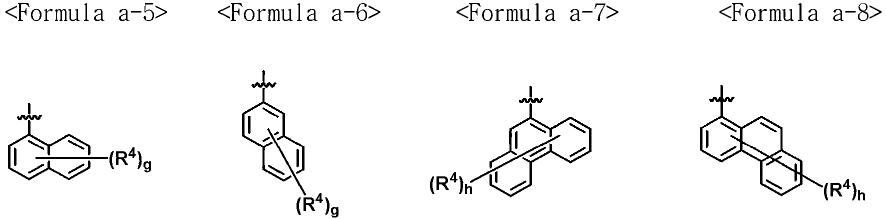 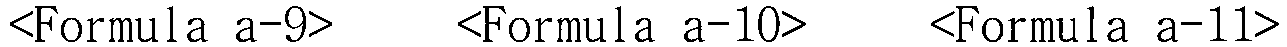 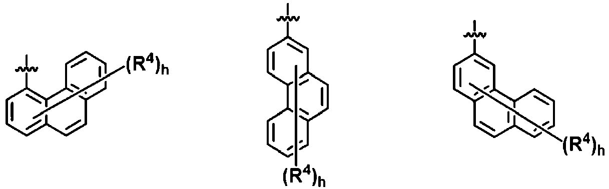 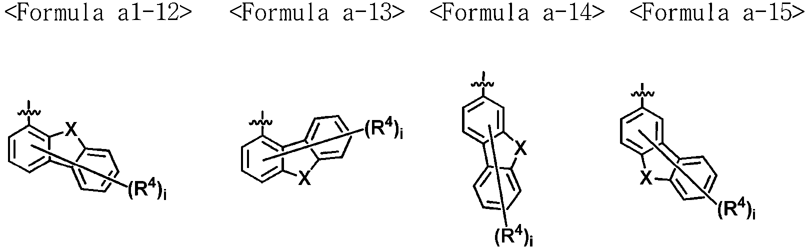 wherein:
R⁴ is independently the same or different from each other and is independently selected from the group consisting of deuterium; a C₆-C₂₀ aryl group; and a C₆-C₂₀ aryl group substituted with deuterium; or a plurality of adjacent groups thereof may be bonded to each other to form a ring,
d and f are each independently an integer of 0 to 5, e is an integer of 0 to 4, g and i are each independently an integer of 0 to 7, h is an integer of 0 to 9,
X is O, S, NR' or CR'R",
R' and R" are independently selected from the group consisting of hydrogen; deuterium; a C₁-C₂₀ alkyl group; a C₆-C₆₀ aryl group; and a C₆-C₂₀ aryl group substituted with deuterium; a C₂-C₆₀ heterocyclic group including at least one heteroatom of O, N, S, Si or P; or R' and R" may be bonded to each other to form a ring, 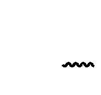 indicates the position to be bonded.

Specifically, the compound of Formula 1 may be any one of the following compounds P-1 to P-128, but is not limited thereto.

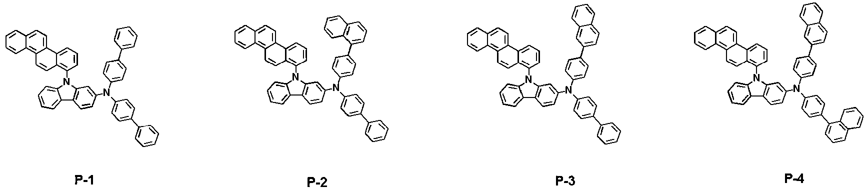

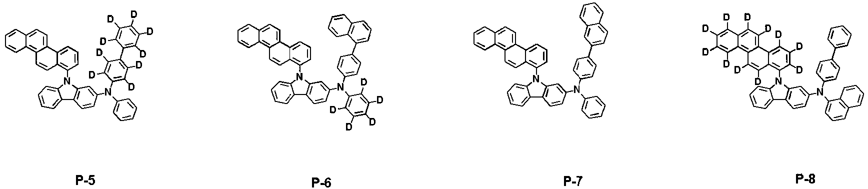

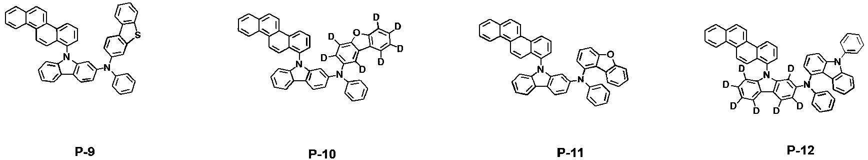

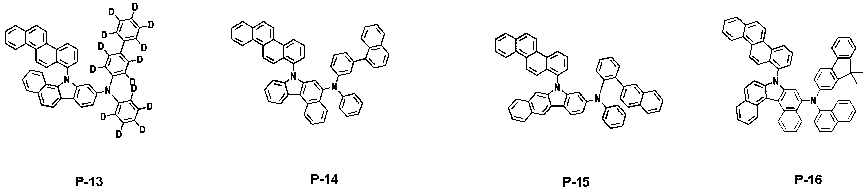

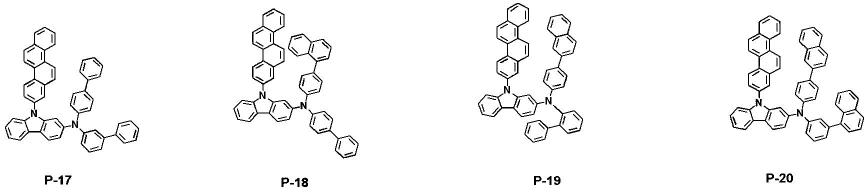

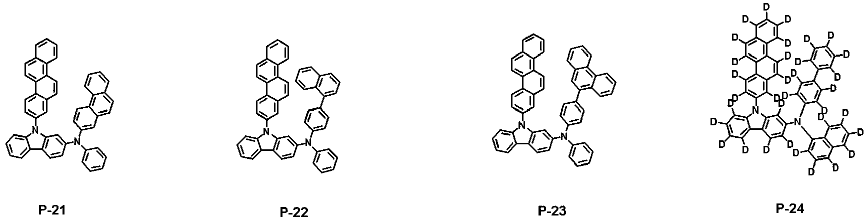

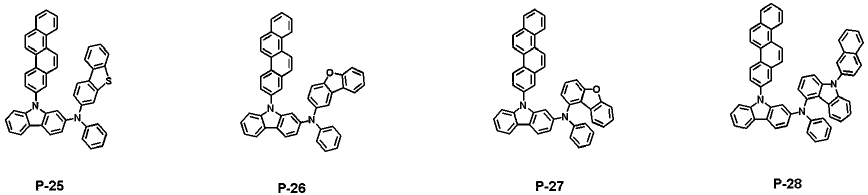

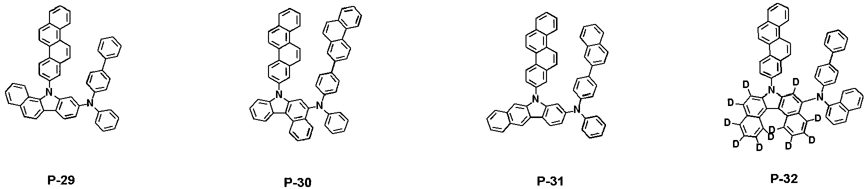

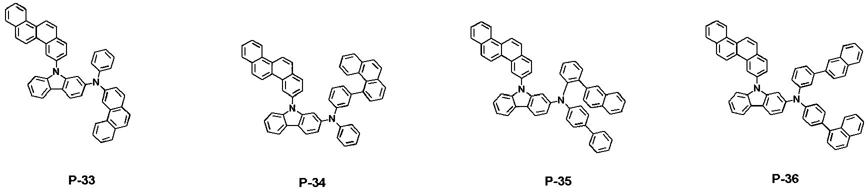

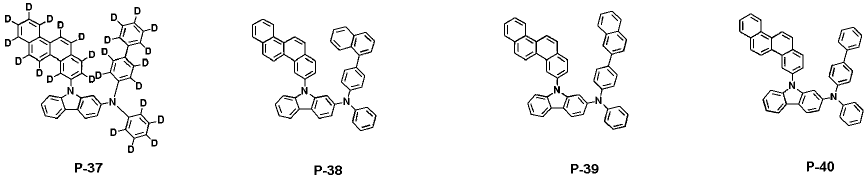

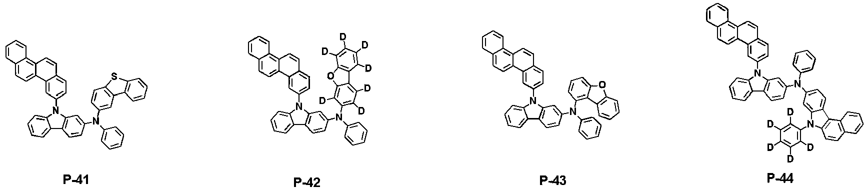

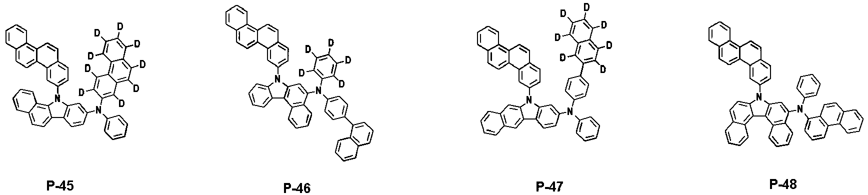

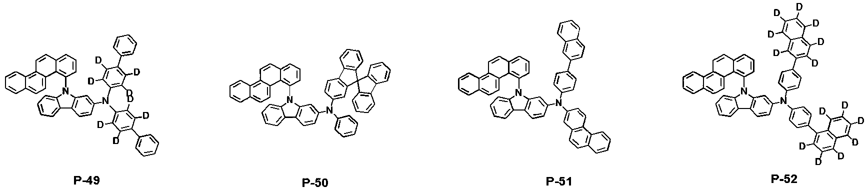

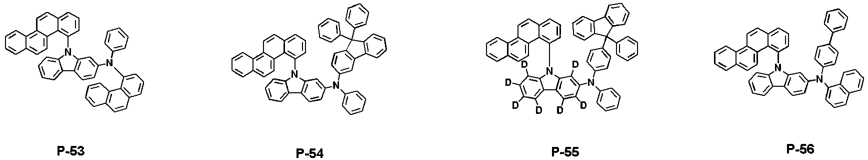

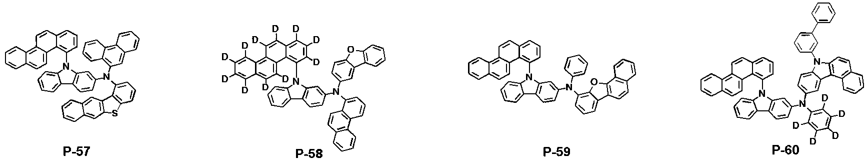

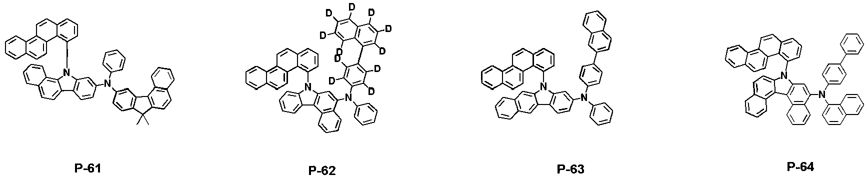

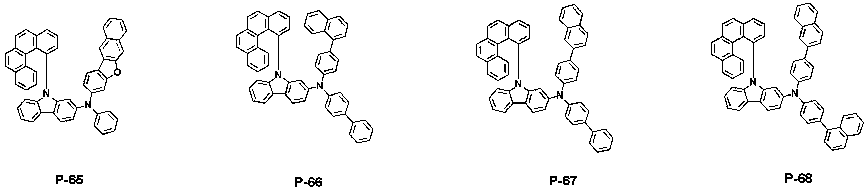

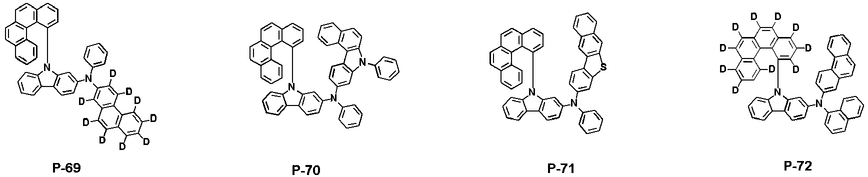

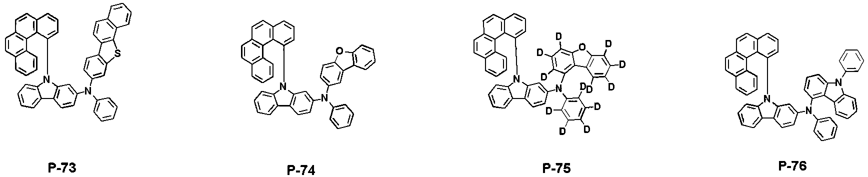

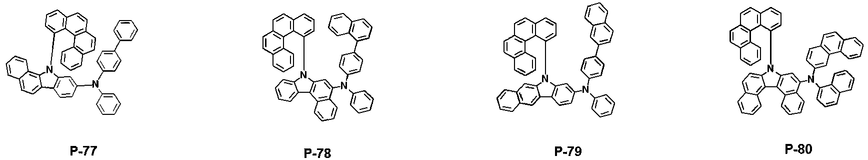

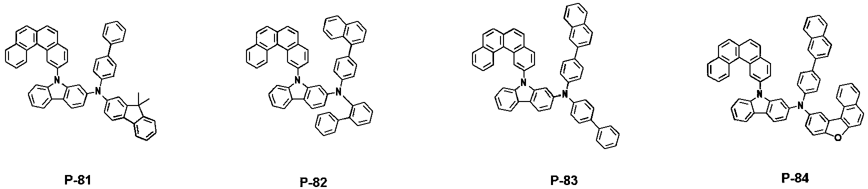

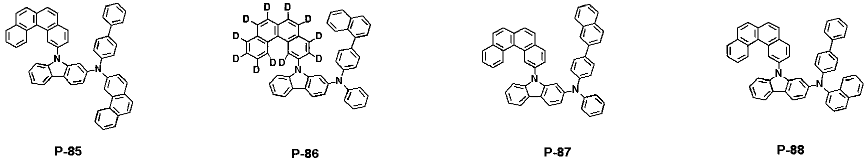

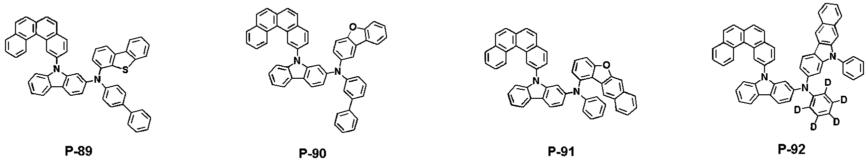

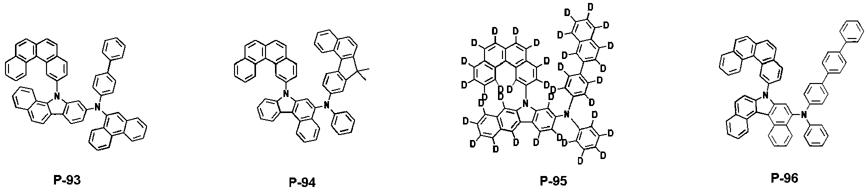

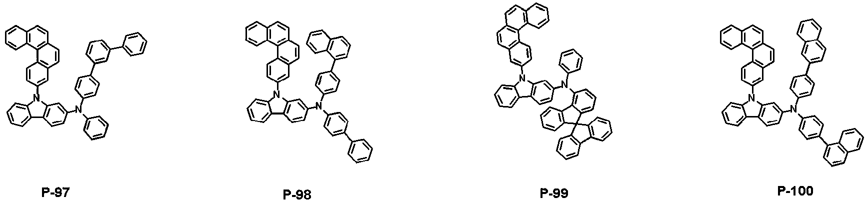

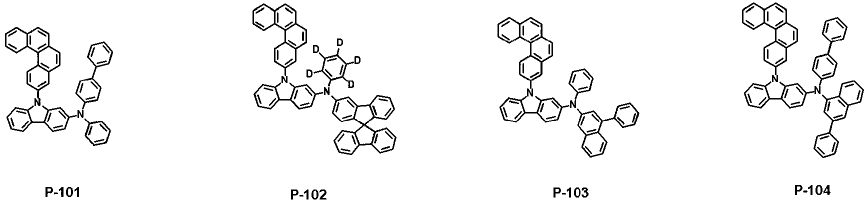

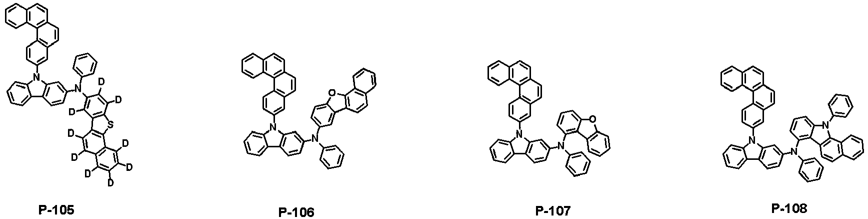

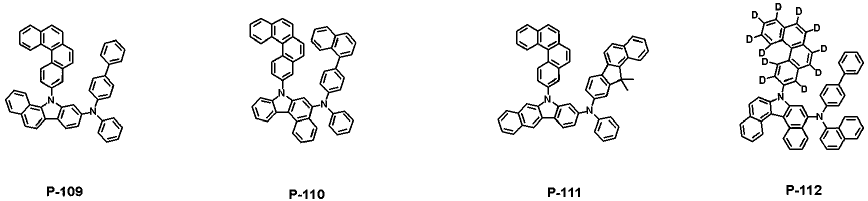

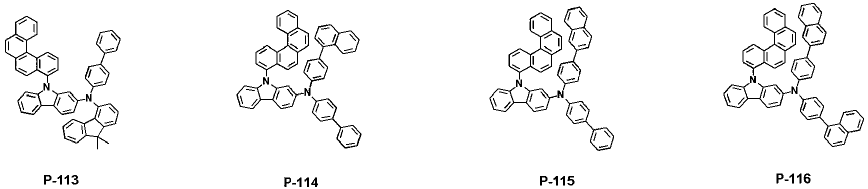

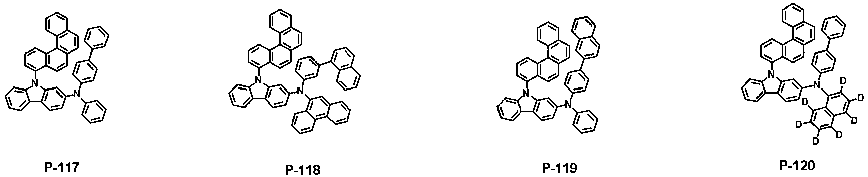

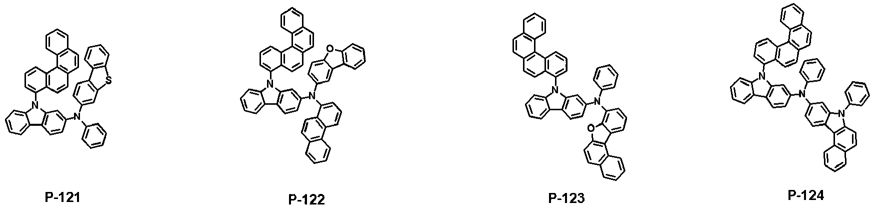

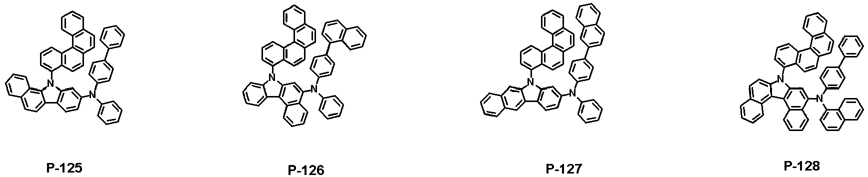

Additionally, in another aspect, the present invention provides a composition for a phosphorescent light-emitting layer of an organic electronic element comprising a mixture of a compound represented by Formula 1 and a compound represented by Formula 2. 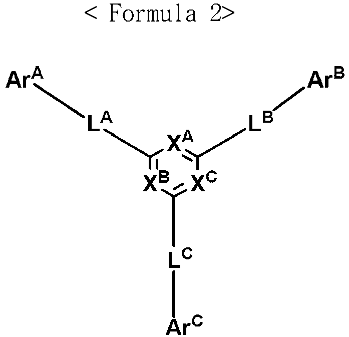 wherein:
X^{A}, X^{B} and X^{C} are each independently CR^{a} or N,

However, at least one of X^{A}, X^{B} and X^{C} is N,
Ar^{A}, Ar^{B} and Ar^{C} are independently selected from the group consisting of a C₆-C₆₀ aryl group; a fluorenyl group; a C₂-C₆₀ heterocyclic group including at least one heteroatom of O, N, S, Si or P; a C₃-C₆₀ cycloalkyl group; a fused ring group of a C₃-C₆₀ aliphatic ring and a C₆-C₆₀ aromatic ring;
Wherein when Ar^{A}, Ar^{B} and Ar^{C} are an aryl group, preferably a C₆-C₃₀ aryl group, more preferably a C₆-C₂₅ aryl group, a C₆-C₁₈ aryl group or a C₆-C₁₂ aryl group, such as phenyl, biphenyl, terphenyl, naphthalene, phenanthrene, chrysene, etc.

Wherein when Ar^{A}, Ar^{B} and Ar^{C} are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.

Wherein when Ar^{A}, Ar^{B} and Ar^{C} are an cycloalkyl group, preferably a C₃-C₃₀ cycloalkyl group, more preferably a C₃-C₂₅ cycloalkyl group, a C₃-C₁₈ cycloalkyl group or a C₃-C₁₂ cycloalkyl group, such as a cyclobutane, cyclopentane, cyclohexane, bicycloheptane, adamantyl etc.

Wherein when Ar^{A}, Ar^{B} and Ar^{C} are a fused ring group, preferably a fused ring group of a C₃-C₃₀ aliphatic ring and a C₆-C₃₀ aromatic ring, more preferably a fused ring group of a C₃-C₂₅ aliphatic ring and a C₆-C₂₅ aromatic ring.

L^{A}, L^{B} and L^{C} are independently selected from the group consisting of a single bond; a C₆-C₆₀ arylene group; a fluorenylene group; a C₂-C₆₀ heterocyclic group including at least one heteroatom of O, N, S, Si or P; and a fused ring group of a C₃-C₆₀ aliphatic ring and a C₆-C₆₀ aromatic ring;
Wherein when L^{A}, L^{B} and L^{C} are an arylene group, preferably a C₆-C₃₀ arylene group, more preferably a C₆-C₂₅ arylene group, a C₆-C₁₈ arylene group or a C₆-C₁₂ arylene group, such as phenylene, biphenylene, naphthylene, terphenylene, anthracenylene, etc.

Wherein when L^{A}, L^{B} and L^{C} are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.

Wherein when L^{A}, L^{B} and L^{C} are a fused ring group, preferably a fused ring group of a C₃-C₃₀ aliphatic ring and a C₆-C₃₀ aromatic ring, more preferably a fused ring group of a C₃-C₂₅ aliphatic ring and a C₆-C₂₅ aromatic ring.

The R^{a} is hydrogen; or deuterium;
wherein the aryl group, arylene group, heterocyclic group, fluorenyl group, fluorenylene group, cycloalkyl group and fused ring group may be substituted with one or more substituents selected from the group consisting of deuterium; halogen; silane group; siloxane group; boron group; germanium group; a cyano group; a nitro group; a C₁-C₂₀ alkylthio group; a C₁-C₂₀ alkoxyl group; ; a C₆-C₂₀ aryloxy group a C₁-C₂₀ alkyl group; a C₂-C₂₀ alkenyl group; a C₂-C₂₀ alkynyl group; a C₆-C₂₀ aryl group; a C₆-C₂₀ aryl group substituted with deuterium; a fluorenyl group; a C₂-C₂₀ heterocyclic group; a C₃-C₂₀ aliphatic ring; a C₇-C₂₀ arylalkyl group; a C₆-C₂₀ arylalkenyl group; and a C₇-C₂₀ alkylaryl group; and the hydrogen of these substituents may be further substituted with one or more deuterium or a C₆-C₂₀ aryl group; and the substituents may be bonded to each other to form a saturated or unsaturated ring, wherein the term 'ring' means a C₃-C₆₀ aliphatic ring or a C₆-C₆₀ aromatic ring or a C₂-C₆₀ heterocyclic group or a fused ring formed by the combination thereof.

Additionally, the present invention provides a composition for a phosphorescent light-emitting layer of an organic electronic element, wherein at least one of the Ar^{A}, Ar^{B} and Ar^{C} is represented by any one of Formulae Ar-a to Ar-d. 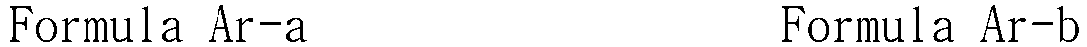 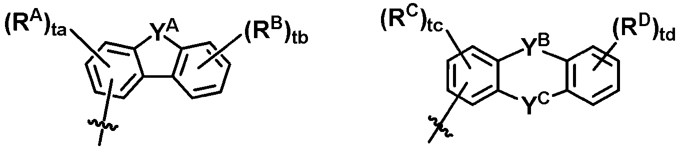 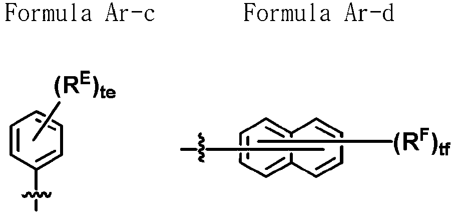 wherein:
Y^{A}, Y^{B} and Y^{C} are each independently O, S, NR^{1A} or CR^{1B}R^{1C},
R^{A}, R^{B}, R^{C}, R^{D}, R^{E}, R^{F}, R^{1A}, R^{1B} and R^{1C} are independently the same or different from each other and are independently selected from the group consisting of hydrogen; deuterium; halogen; a cyano group; a C₆-C₂₀ aryl group; a fluorenyl group; a C₂-C₂₀ heterocyclic group including at least one heteroatom of O, N, S, Si or P; a C₁-C₂₀ alkyl group; a C₂-C₂₀ alkenyl group; a C₂-C₂₀ alkynyl group;
a C₁-C₂₀ alkoxyl group; and a C₆-C₂₀ aryloxy group; or a plurality of adjacent groups thereof may be bonded to each other to form a ring,
ta and tc are each independently an integer of 0 to 3, tb and td are each independently an integer of 0 to 4, te is an integer of 0 to 5, tf is an integer of 0 to 7, 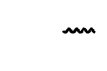 indicates the position to be bonded.

Formula Ar-a is represented by any one of the following Formulas Ar-a-1 to Ar-a-4. 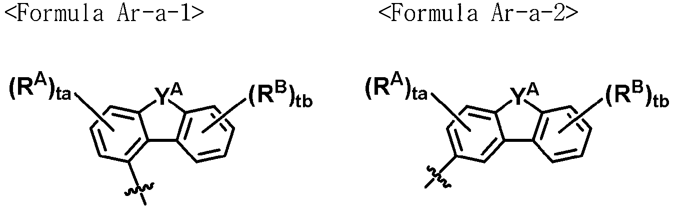 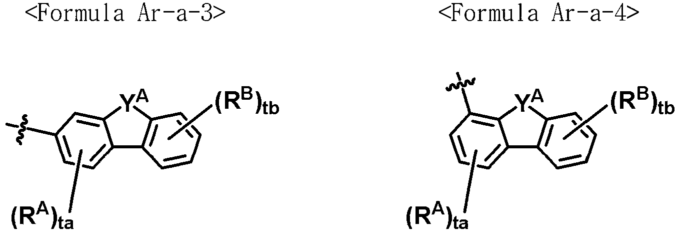 wherein:
R^{A}, R^{B}, Y^{A}, ta, tb and 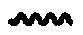 are the same as defined in Ar-a.

Formula Ar-b is represented by the following Formula Ar-b-1 or Formula Ar-b-2. 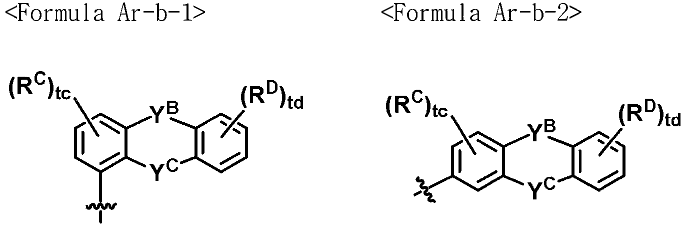 wherein:
R^{C}, R^{D}, Y^{B}, Y^{C}, tc, td and 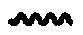 are the same as defined in Ar-b.

Formula Ar-d is represented by the following Formula Ar-d-1 or Formula Ar-d-2. 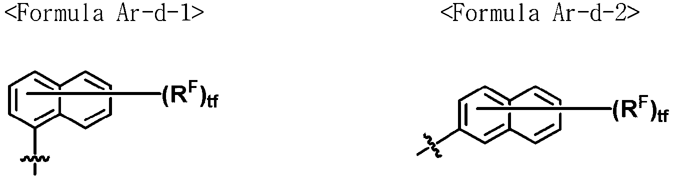 wherein:
R^{F}, tf and 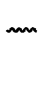 are the same as defined in Ar-d.
L^{A}, L^{B} and L^{C} of Formula 2 may be represented by a single bond or any one of the following Formulas b-1 to b-13. 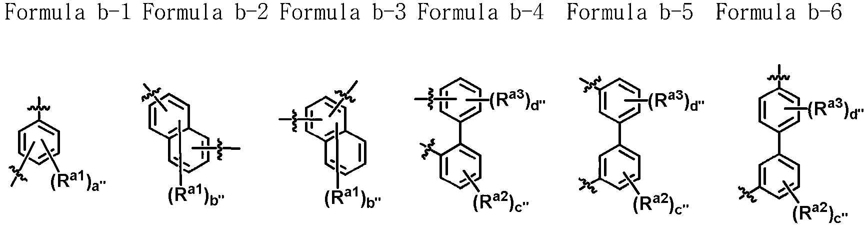 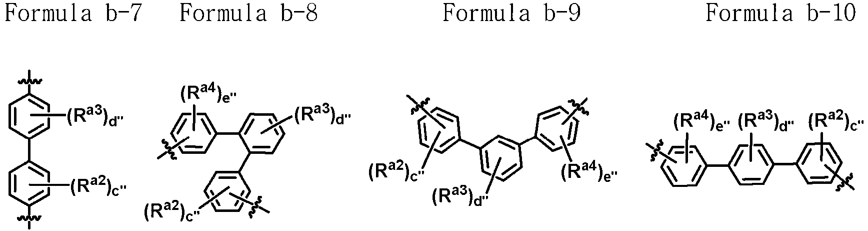 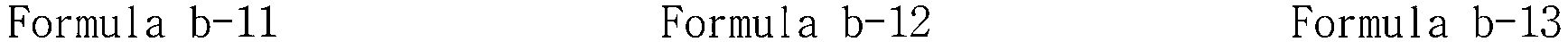 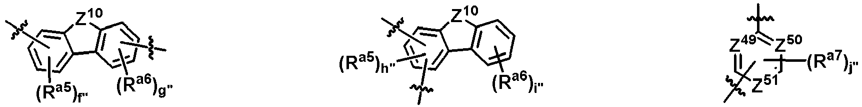 wherein:
   Z¹⁰ is O, S, NR^{1D} or CR^{1E}R^{1F},
   a" , c" , d" , e" and i" are independently an integer of 0 to 4, b" is an integer of 0 to 6, f" and g" are independently an integer of 0 to 3, h" is an integer of 0 to 2, j" is an integer of 0 to 1,
   R^{a1}, R^{a2}, R^{a3}, R^{a4}, R^{a5}, R^{a6}, R^{a7}, R^{1D}, R^{1E} and R^{1F} are independently the same or different from each other and are independently selected from the group consisting of hydrogen; deuterium; a C₆-C₂₀ aryl group; a fluorenyl group; a C₂-C₂₀ heterocyclic group including at least one heteroatom of O, N, S, Si or P; a C₁-C₅₀ alkyl group; a C₂-C₂₀ alkenyl group; a C₂-C₂₀ alkynyl group; a C₁-C₂₀ alkoxyl group; and a C₆-C₂₀ aryloxy group; or a plurality of adjacent groups thereof may be bonded to each other to form a ring,
   Z⁴⁹, Z⁵⁰ and Z⁵¹ are independently CR^{1G} or N,
   However, at least one of Z⁴⁹, Z⁵⁰ and Z⁵¹ is N,
   R^{1G} is the same as the definition of R^{A}, 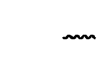 indicates the position to be bonded.

Specifically, the compound of Formula 2 may be any one of the following compounds N-1 to N-276, but is not limited thereto.

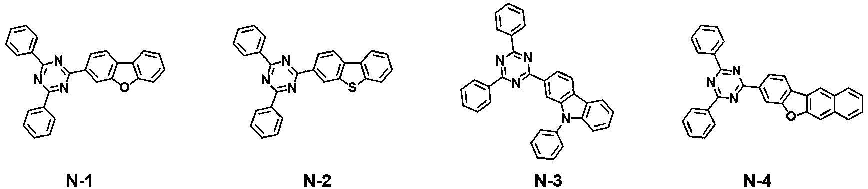

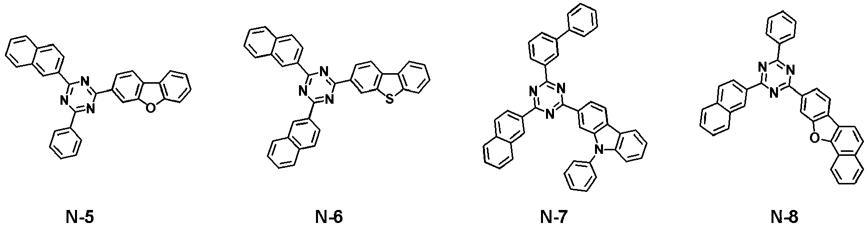

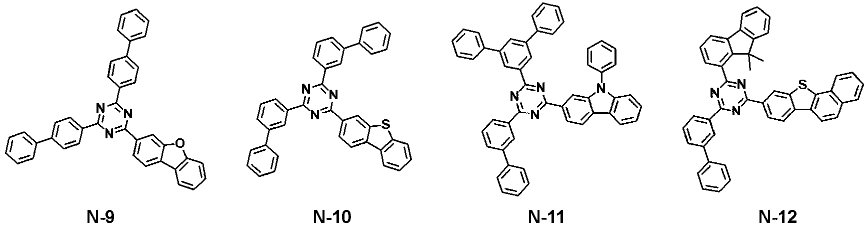

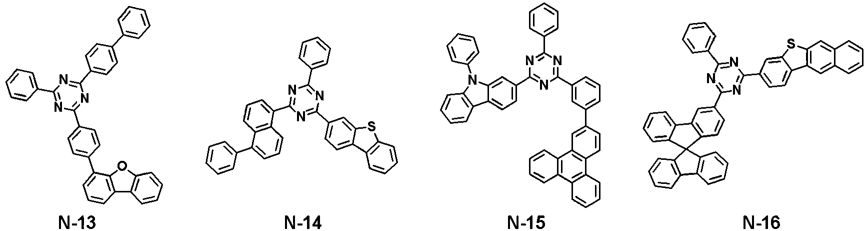

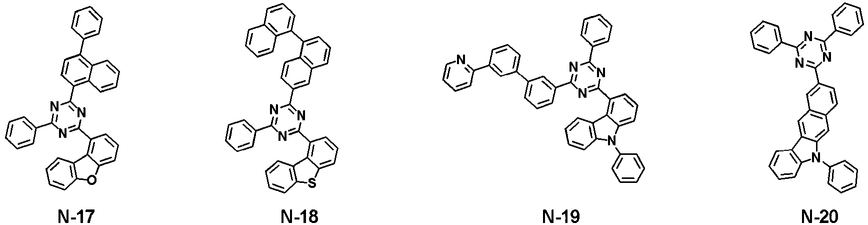

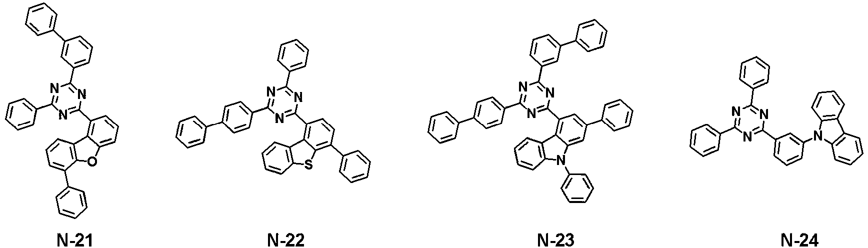

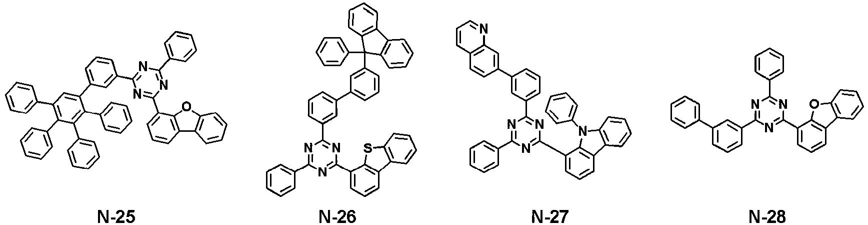

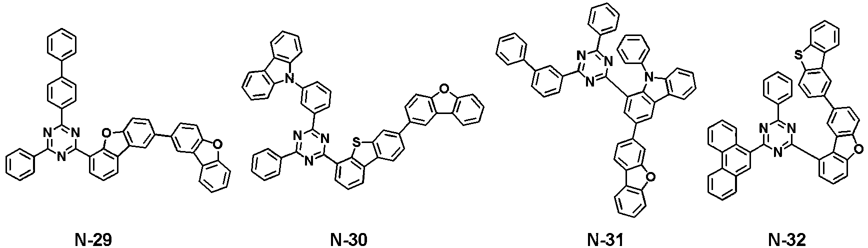

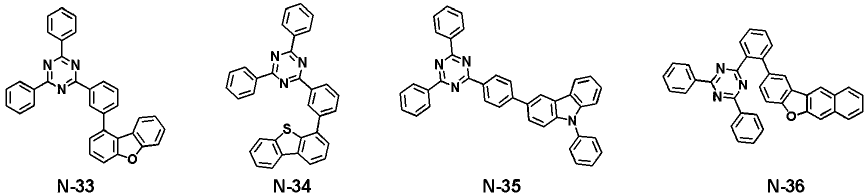

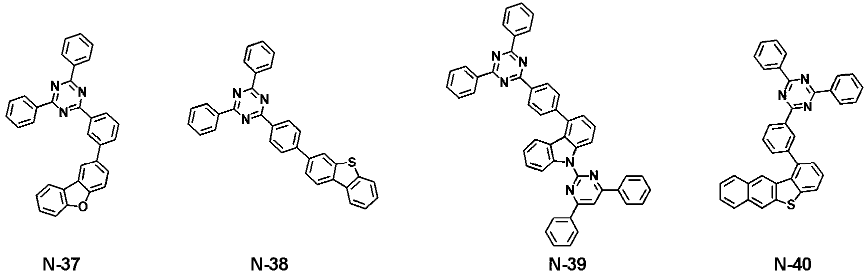

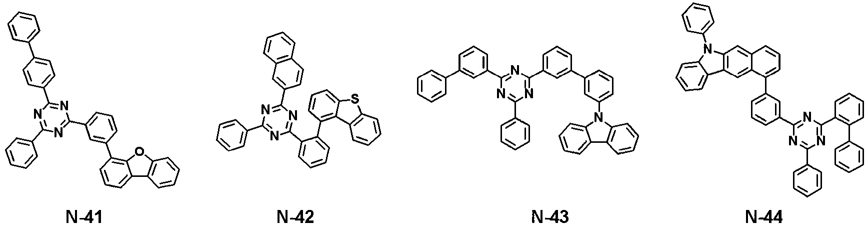

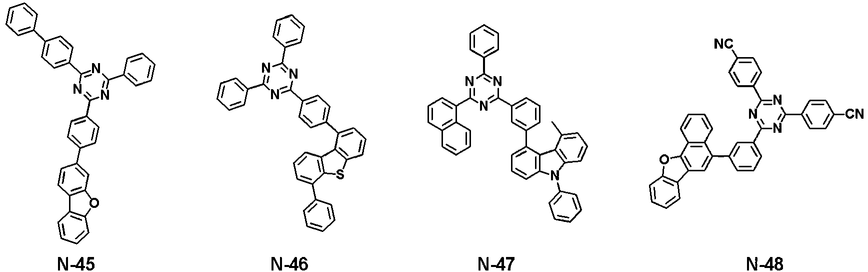

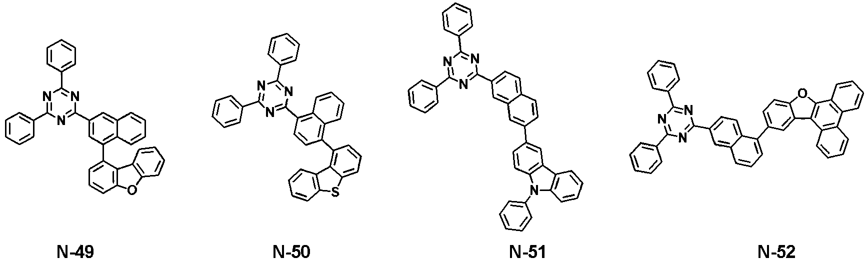

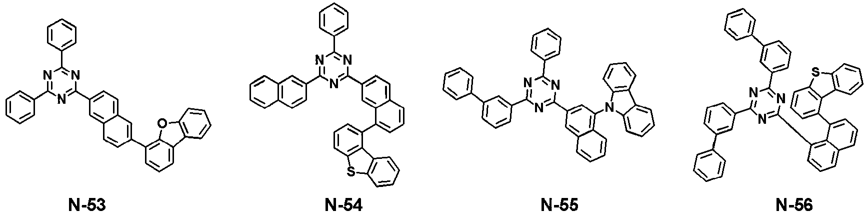

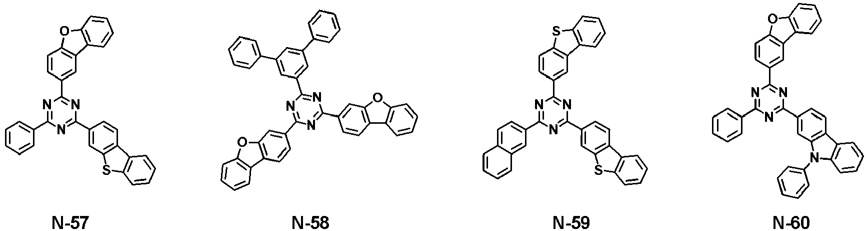

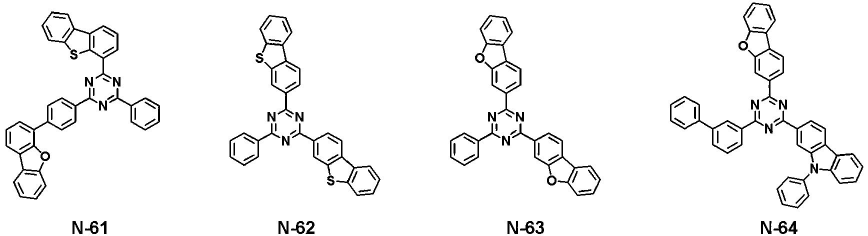

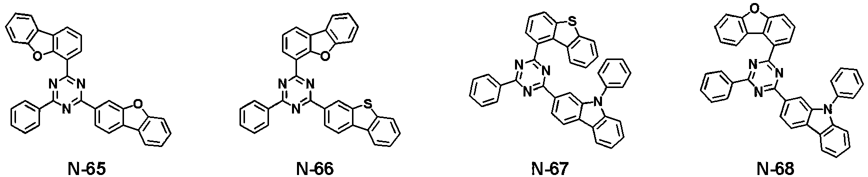

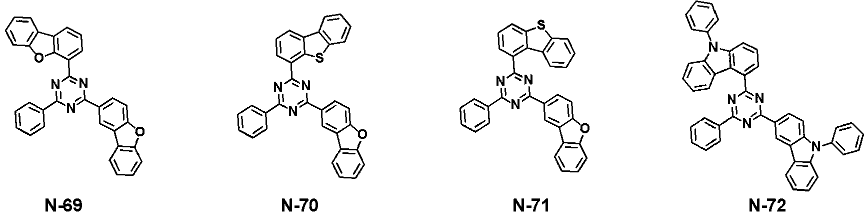

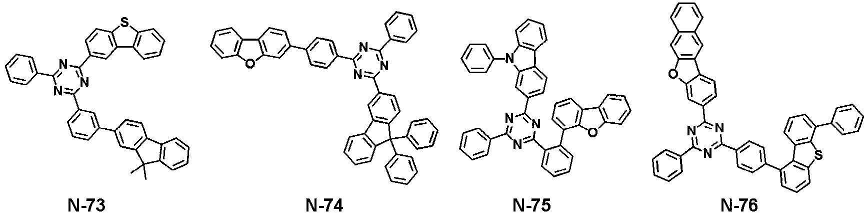

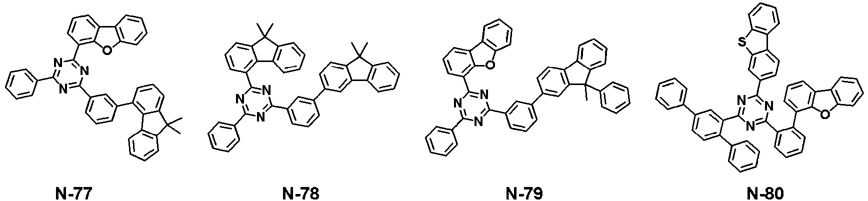

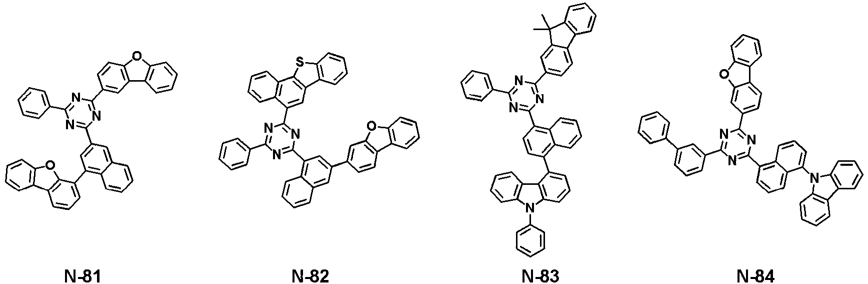

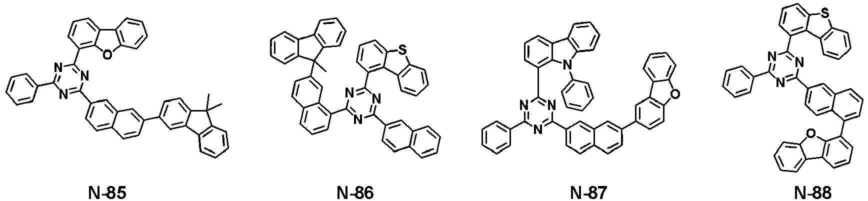

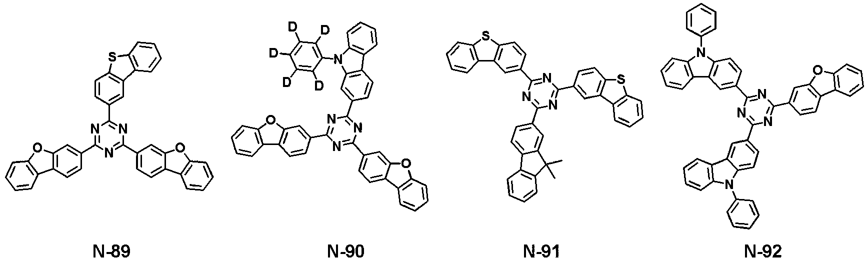

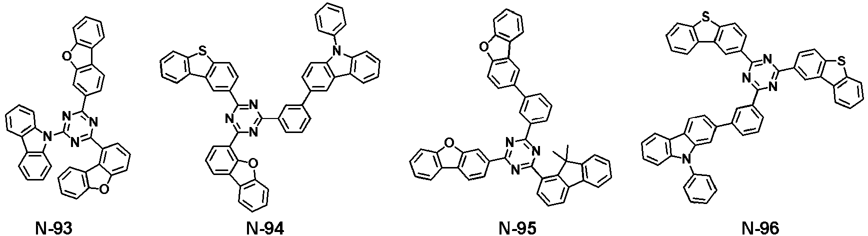

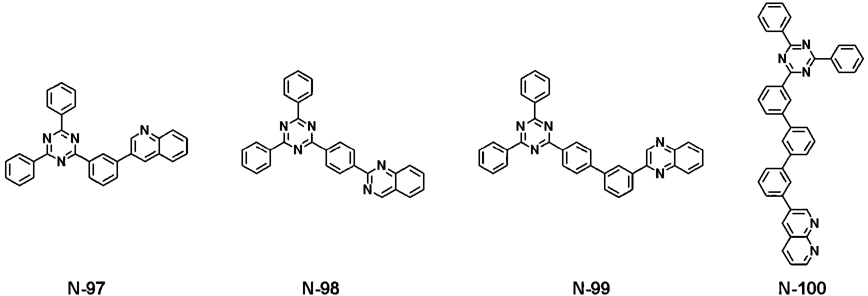

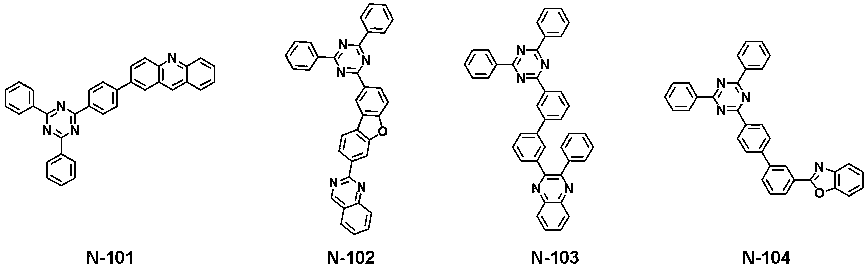

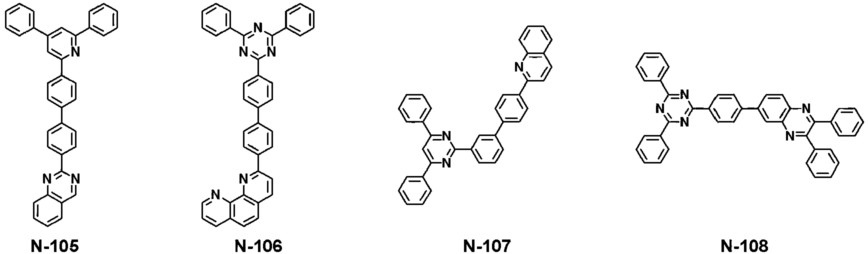

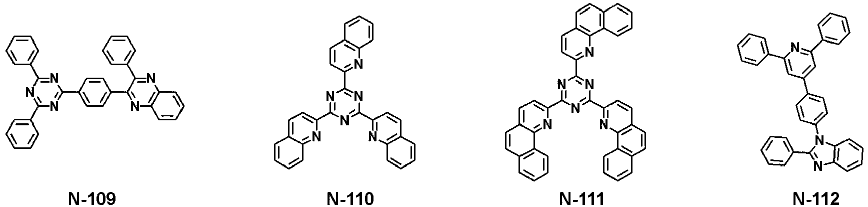

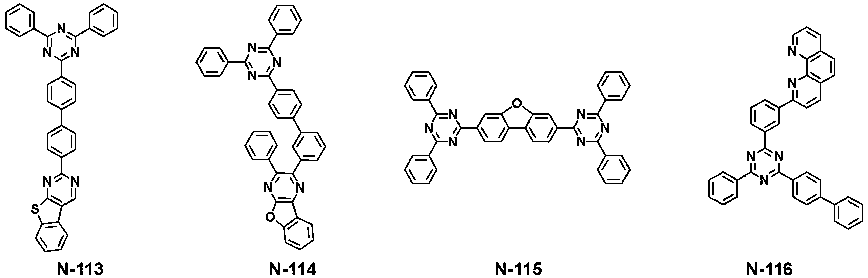

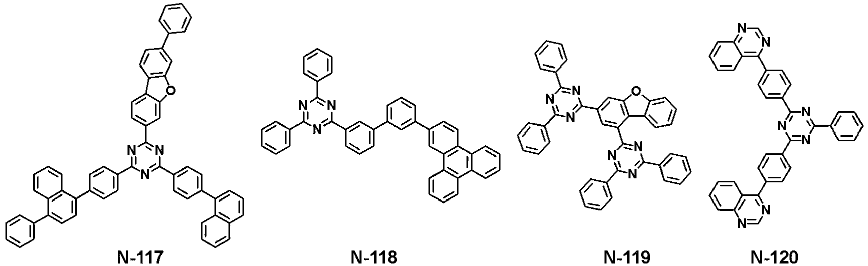

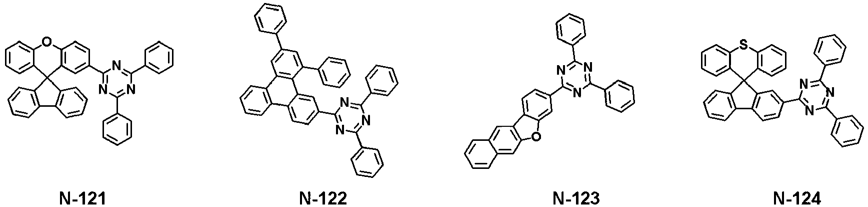

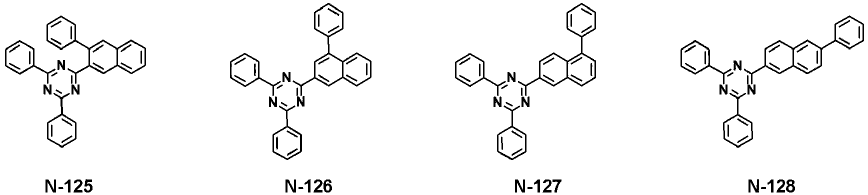

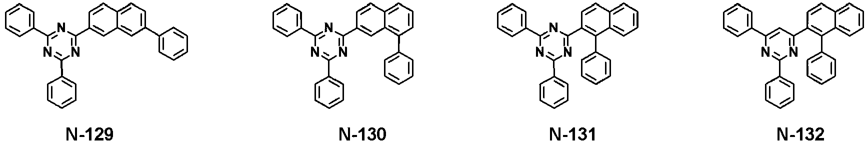

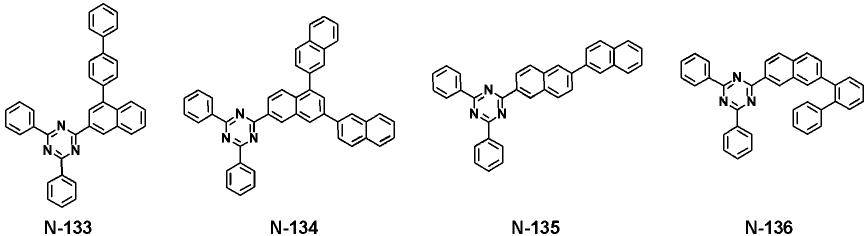

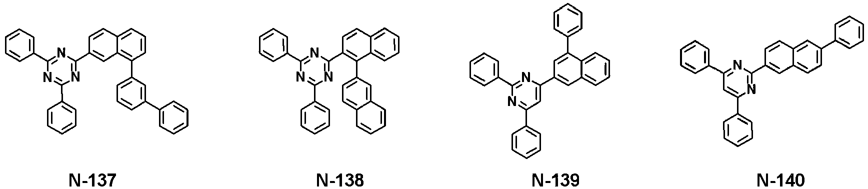

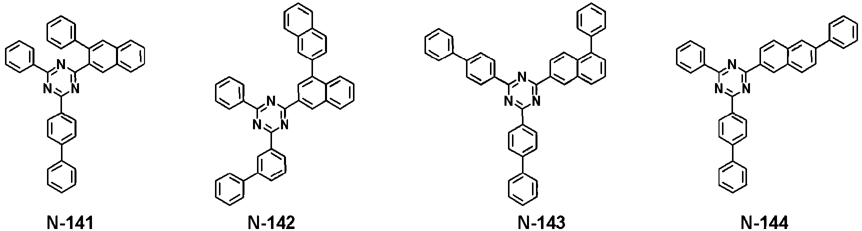

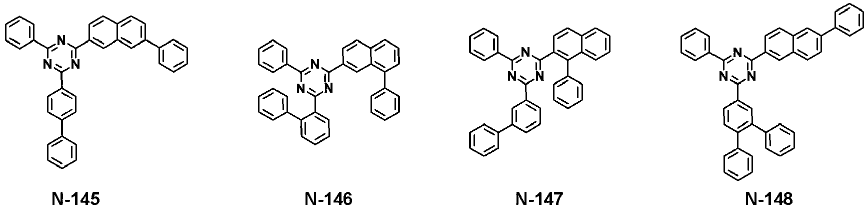

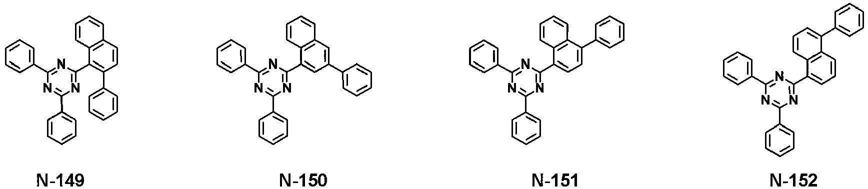

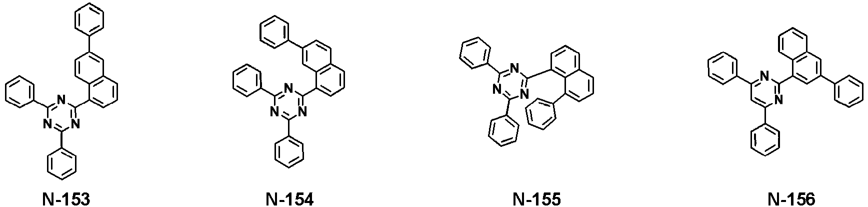

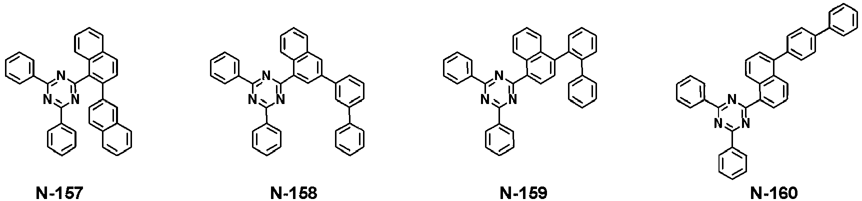

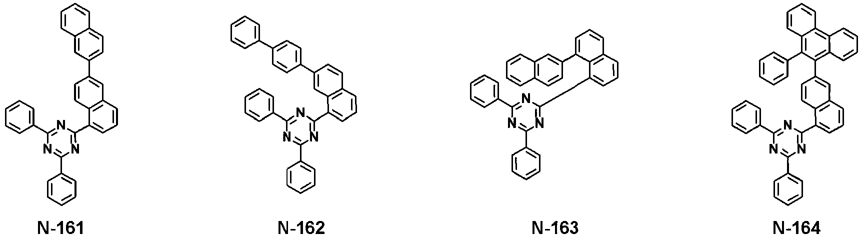

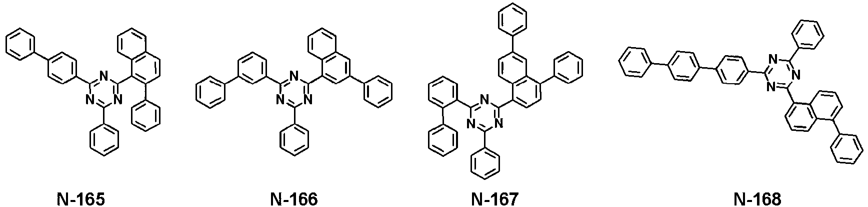

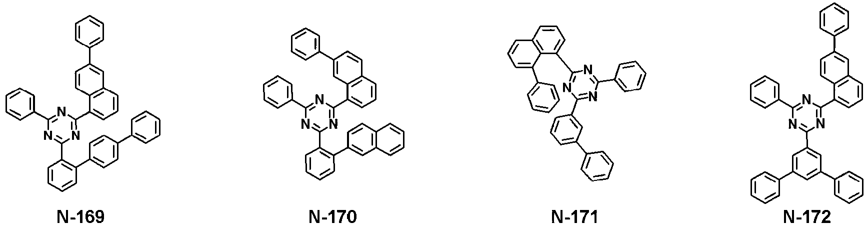

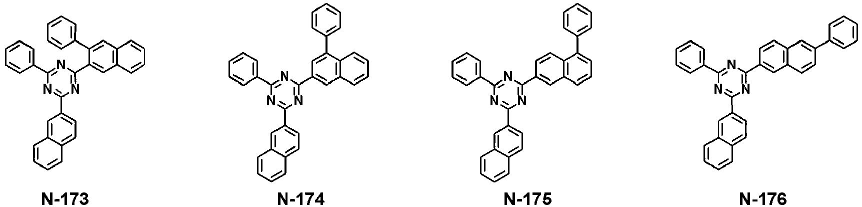

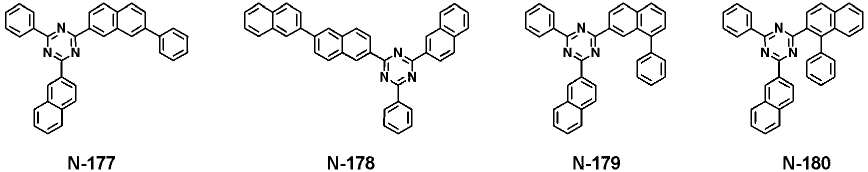

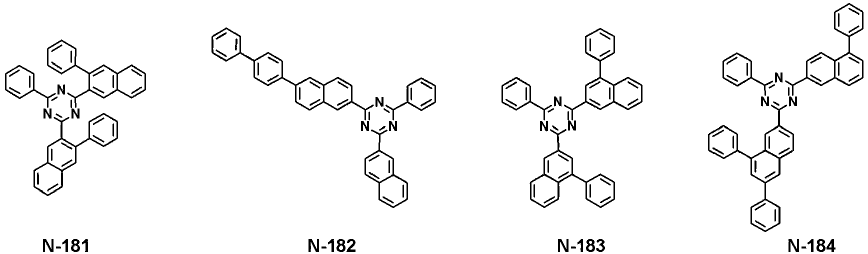

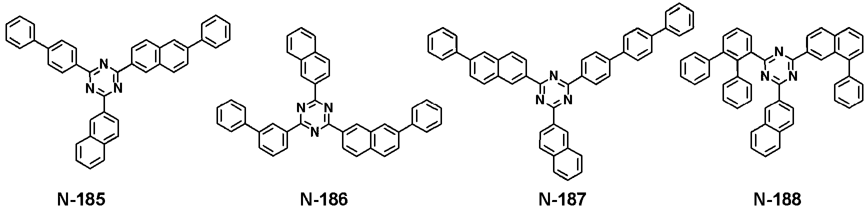

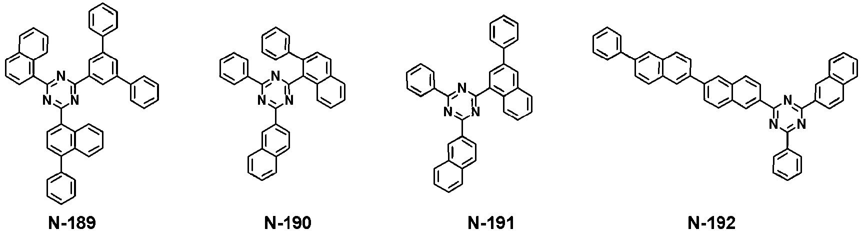

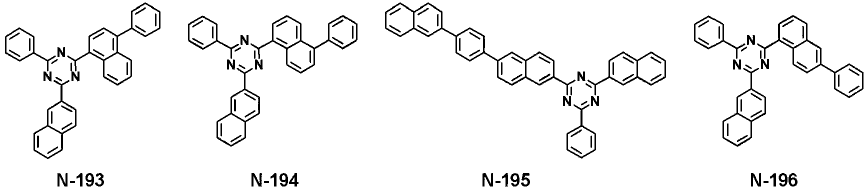

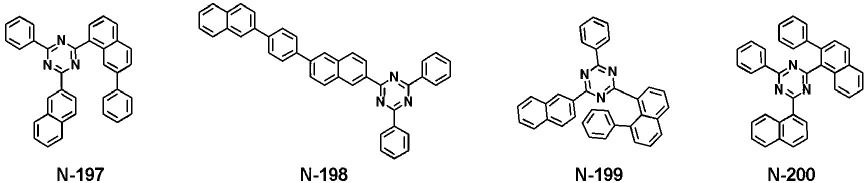

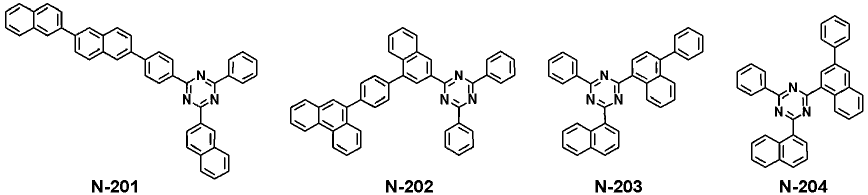

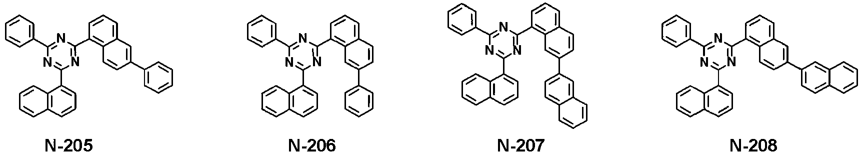

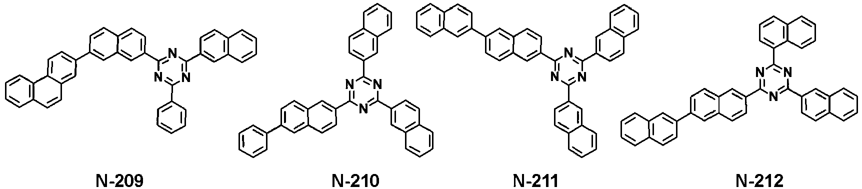

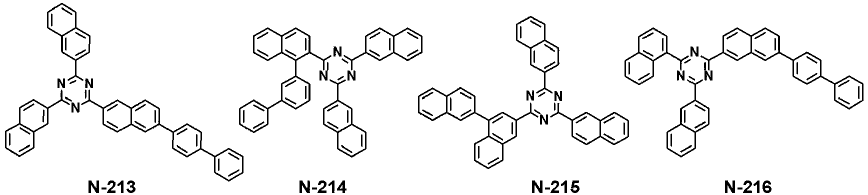

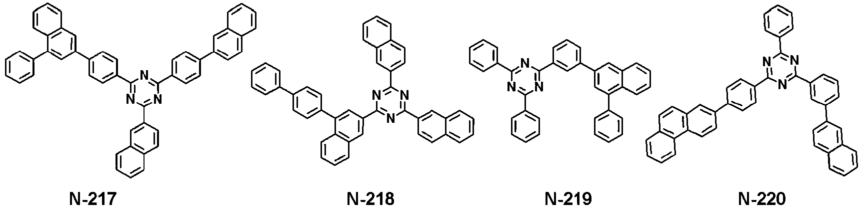

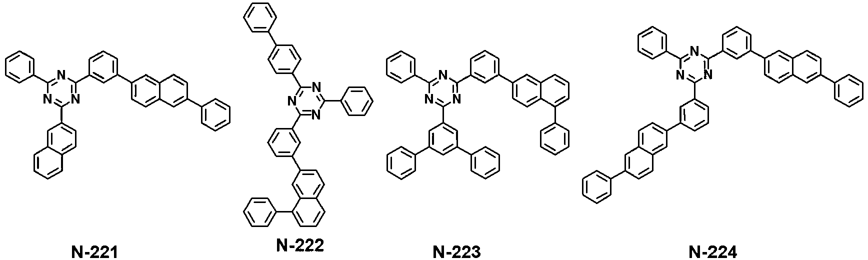

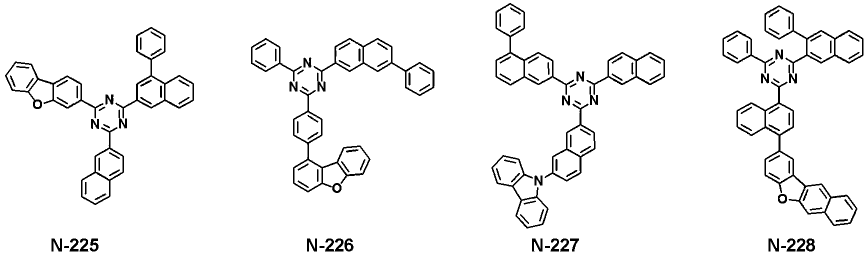

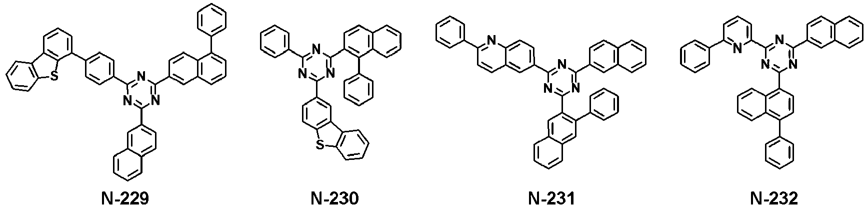

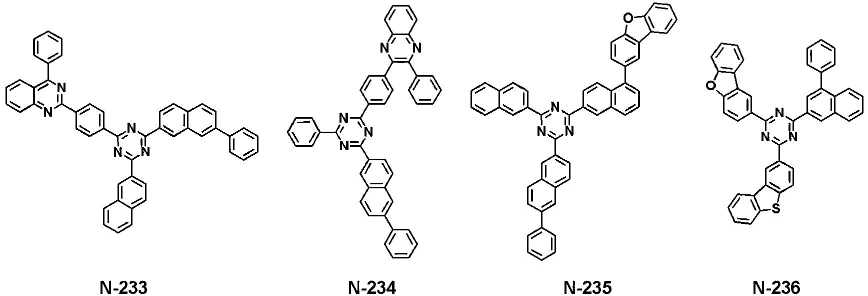

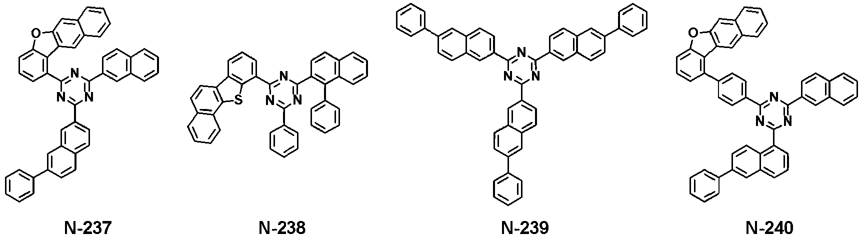

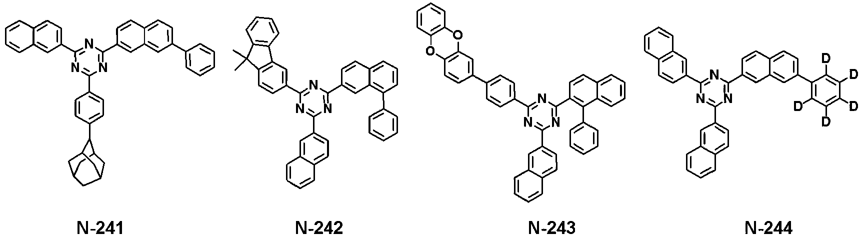

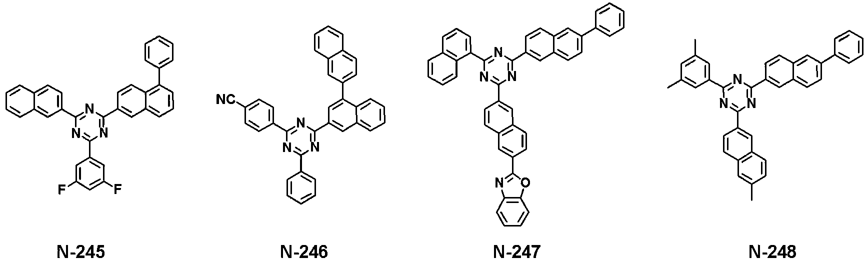

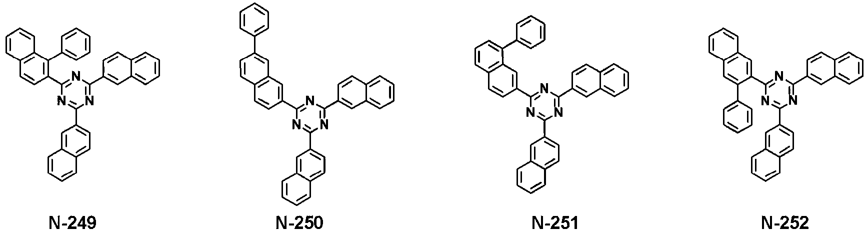

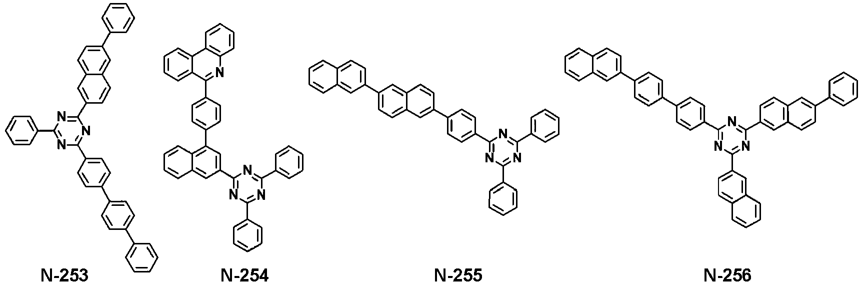

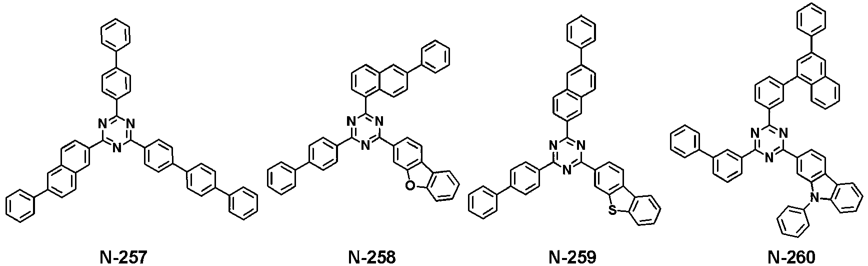

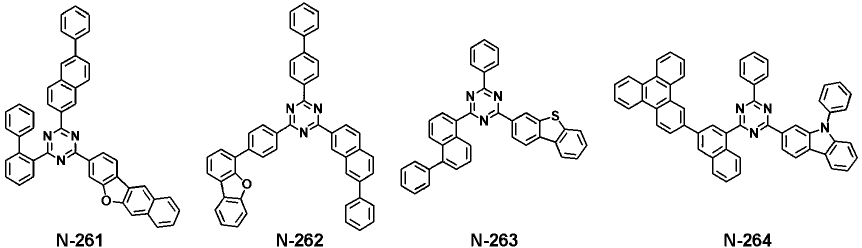

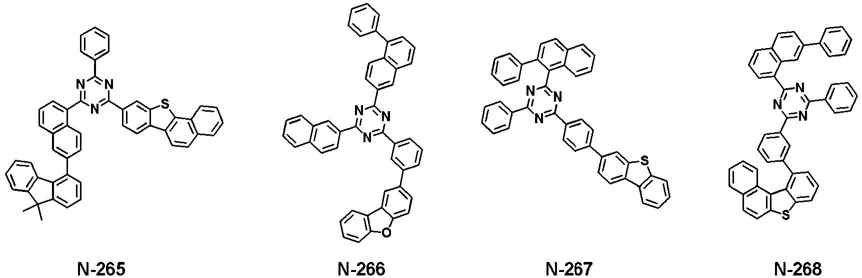

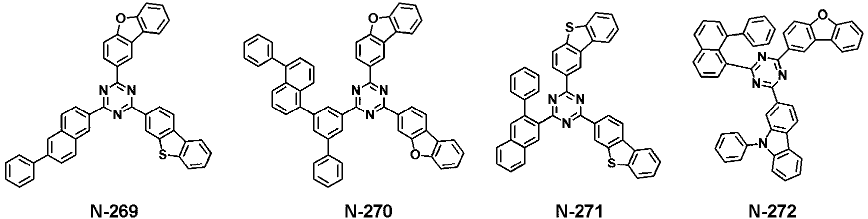

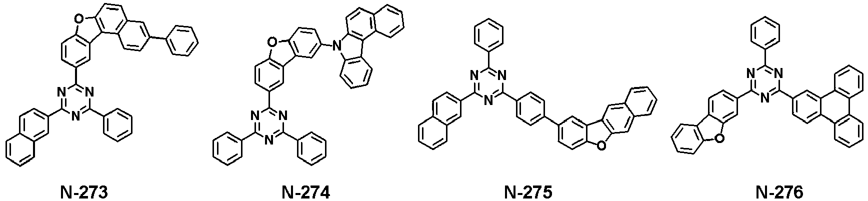

Preferably, the composition for the phosphorescent light-emitting layer of the organic electronic element may be a host for the emitting layer.

Additionally, in another aspect, the present invention provides an organic electronic element comprising a first electrode, a second electrode and an organic material layer between the first electrode and the second electrode; wherein the organic material layer comprises a composition for a phosphorescent light-emitting layer of the organic electronic element, wherein the phosphorescent light-emitting layer comprises a compound represented by Formula 1, or a mixture of a compound represented by Formula 1 and a compound represented by Formula 2.

In another aspect, the present invention provides a method for reusing a compound of Formula 1 comprising:
recovering a crude organic light emitting material comprising the compound of Formula 1 from a deposition apparatus used in the process for depositing the organic emitting material to prepare an organic light emitting device;
removing impurities from the crude organic light emitting material;
recovering the organic light emitting material after the impurities are removed; and
purifying the recovered organic light emitting material to have a purity of 99.9% or higher.

The step of removing impurities from the crude organic light emitting material recovered from the deposition apparatus may preferably comprise performing a pre-purification process to obtain a purity of 98% or more by recrystallization in a recrystallization solvent.

The recrystallization solvent may be preferably a polar solvent having a polarity index (PI) of 5.5 to 7.2.

The recrystallization solvent may be preferably a polar solvent having a polarity index (PI) of 5.5 to 7.2.

The recrystallization solvent may preferably be used by mixing a polar solvent having a polarity value of 5.5 to 7.2 and a non-polar solvent having a polarity value of 2.0 to 4.7.

When a mixture of a polar solvent and a non-polar solvent is used, the recrystallization solvent may be used in an amount of 15% (v/v) or less of the non-polar solvent compared to the polar solvent.

The recrystallization solvent is preferably a single solvent of N-Methylpyrrolidone (NMP); or a polar solvent mixed any one selected from the group consisting of 1,3-Dimethyl-2-imidazolidinone, 2-pyrrolidone, N,N-Dimethyl formamide, Dimethyl acetamide, and Dimethyl sulfoxide to the N-Methylpyrrolidone; or alone; or mixed non-polar solvents; selected from the group consisting of Toluene, Dichloromethane (DCM), Dichloroethane (DCE), Tetrahydrofuran (THF), Chloroform, Ethyl acetate and Butanone; or a mixture of a polar solvent and a non-polar solvent.

The pre-purification process may comprise a step of precipitating crystals of by cooling to 0° C. to 5° C. after dissolving the crude organic light emitting material recovered from the deposition apparatus in a polar solvent at 90° C. to 120° C.

The pre-purification process may comprise a step of precipitating crystals by cooling to 35° C to 40° C, adding a non-polar solvent, and then cooling to 0° C to 5° C. after dissolving the crude organic light emitting material recovered from the deposition apparatus in a polar solvent at 90° C. to 120° C.

The pre-purification process may comprise a step of precipitating crystals while concentrating the solvent and removing the non-polar solvent, after dissolving the crude organic light emitting material recovered from the deposition apparatus in a non-polar solvent.

The pre-purification process may comprise a step of recrystallizing again with a non-polar solvent after recrystallizing first with a polar solvent.

The step of purifying the recovered impurities to a purity of 99.9% or higher may comprise performing an adsorption separation process to adsorb and remove impurities by adsorbing on the adsorbent.

The adsorbent may be activated carbon, silica gel, alumina, or a material for known adsorption purposes.

The step of purifying the recovered impurities to a purity of 99.9% or higher may comprise performing sublimation purification.

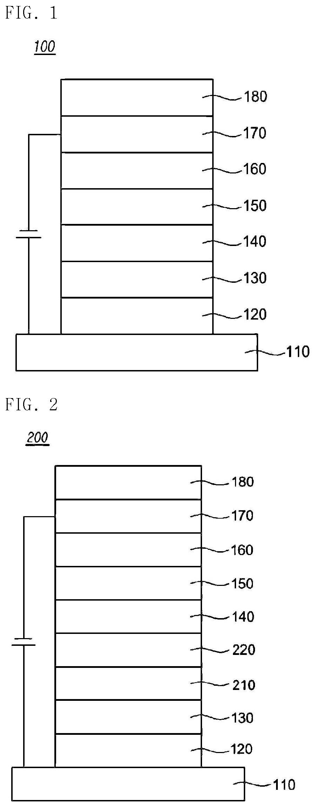

Referring to FIG. 1, the organic electronic element (100) according to one embodiment of the present invention comprises a first electrode (110), a second electrode (120), and organic material layer comprising a single compound or 2 or more compounds represented by Formula 1 between the first electrode (110) and the second electrode (170). Here, the first electrode may be an anode or positive electrode, and the second electrode may be a cathode or a negative electrode, and in the case of an inverted organic electronic element, the first electrode may be a cathode, and the second electrode may be an anode.

The organic material layer may sequentially comprise a hole injection layer (120), a hole transport layer (130), an emitting layer (140), an electron transport layer(150), and an electron injection layer (160) on the first electrode(110). Here, the remaining layers except the emitting layer (140) may not be formed. The organic material layer may further comprise a hole blocking layer, an electron blocking layer, an emitting-auxiliary layer (220), a buffer layer (210), etc., and the electron transport layer (150), etc. may serve as a hole blocking layer (see FIG. 2).

Also, the organic electronic element according to an embodiment of the present invention may further include a protective layer or a light efficiency enhancing layer (180). Wherein the light efficiency enhancing layer is formed on one of both surfaces of the first electrode that is not in contact with the organic material layer or on one of both surfaces of the second electrode that is not in contact with the organic material layer. The compound or material for an organic electronic element according to one embodiment of the present invention applied to the organic material layer may be used as a material for a hole injection layer (120), a hole transport layer (130), an emitting-auxiliary layer (220), an electron transport auxiliary layer, an electron transport layer (150), an electron injection layer (160), a host or dopant of an emitting layer (140), or the light efficiency enhancing layer. Preferably, for example, a composition for a phosphorescent light-emitting layer of an organic electronic element comprising a compound according to Formula 1 of the present invention, or a mixture of a compound represented by Formula 1 and a compound represented by Formula 2 can be used as a host material of the emitting layer.

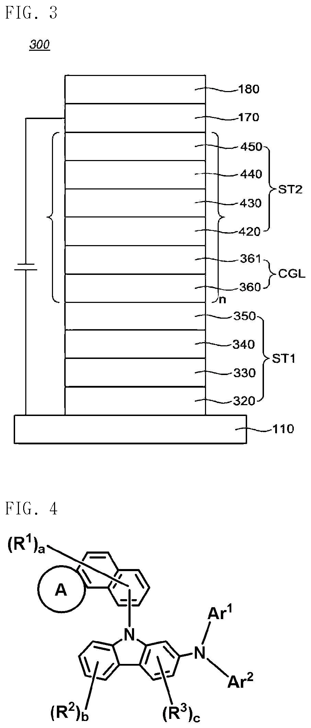

The organic material layer may comprise 2 or more stacks comprising a hole transport layer, an emitting layer and an electron transport layer sequentially formed on the anode, and may further comprise a charge generation layer formed between the 2 or more stacks (see FIG. 3).

Otherwise, even if the same core is used, the band gap, the electrical characteristics, the interface characteristics, etc. may vary depending on which substituent is bonded at which position, therefore the choice of core and the combination of sub-substituents associated therewith is also very important, and in particular, when the optimal combination of energy levels and T1 values, and unique properties of materials(mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer(120), the hole transport layer(130), the emitting layer(140), the electron transport layer(150), and the electron injection layer(160) is formed thereon, and then a material that can be used as a cathode is deposited thereon.

Also, the present invention provides the organic electronic element wherein the organic material layer is formed by one of a spin coating process, a nozzle printing process, an inkjet printing process, a slot coating process, a dip coating process or a roll-to-roll process, and the organic material layer comprises the compound or material for organic electronic elements as an electron transport material.

As another specific example, a compound of the same or different types represented by Formula 1 is mixed and used in the organic material layer. Preferably, the organic material layer comprises an emitting layer, and may comprise a composition for a phosphorescent light-emitting layer of an organic electronic element comprising a compound represented by Formula 1 or a mixture of a compound represented by Formula 1 and a compound represented by Formula 2 as the emitting layer.

Additionally, the present invention provides a composition for a phosphorescent light-emitting layer of an organic electronic element comprising a compound represented by Formula 1, or a mixture of a compound represented by Formula 1 and a compound represented by Formula 2, and provides an organic electronic element comprising the composition.

Also, the present invention also provides an electronic device comprising a display device comprising the organic electronic element; and a control unit for driving the display device.

According to another aspect, the present invention provides a display device wherein the organic electronic element is at least one of an OLED, an organic solar cell, an organic photo conductor, an organic transistor (organic TFT) and an element for monochromic or white illumination. Here, the electronic device may be a wired/wireless communication terminal which is currently used or will be used in the future, and covers all kinds of electronic devices including a mobile communication terminal such as a cellular phone, a personal digital assistant(PDA), an electronic dictionary, a point-to-multipoint(PMP), a remote controller, a navigation unit, a game player, various kinds of TVs, and various kinds of computers.

Hereinafter, Synthesis examples of the compound represented by Formula 1 and Formula 2 of the present invention, and preparation examples of the organic electronic element of the present invention will be described in detail by way of example, but are not limited to the following examples.

### [Synthesis Example]

The compound (final products) represented by Formula 1 according to the present invention is synthesized by reacting Sub 1 and Sub 2 as in Reaction Scheme 1, but is not limited thereto.

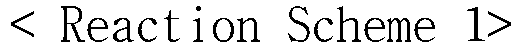

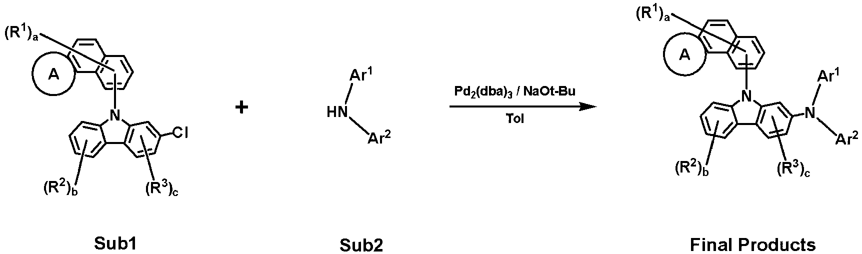

### I. Synthesis of Sub1

Sub1 of the reaction scheme 1 is synthesized by the reaction path of the following reaction scheme 2, but is not limited thereto.

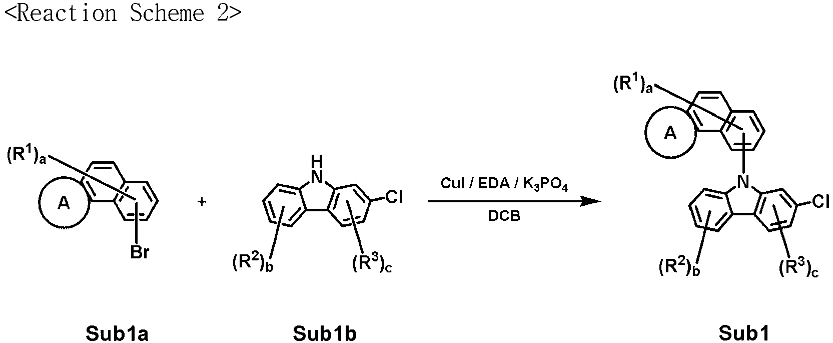

### 1. Synthesis example of Sub1-1

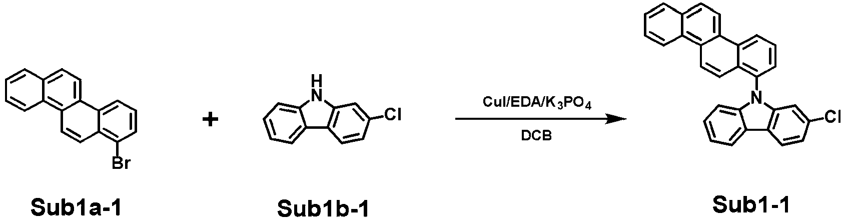

Sub1a-1 (25.0 g, 81.38 mmol) was dissolved in DCB (Dichlorobenzene) (200 mL) in a round-bottom flask, Sub1b-1 (18.0 g, 89.52 mmol), K₃PO₄ (34.5 g, 162.77 mmol), CuI (3.1 g, 16.28 mmol), EDA (Ethylenediamine) (1.96 g, 32.55 mmol) were added, and stirred at 230° C. After the reaction was completed, the mixture was extracted with CH₂Cl₂ and water, the organic layer was dried over MgSO₄ and concentrated. Afterwards, the generated compound was recrystallized after applying a silica gel column to obtain 21.9 g of the product (yield 63%).

### 2. Synthesis example of Sub1-9

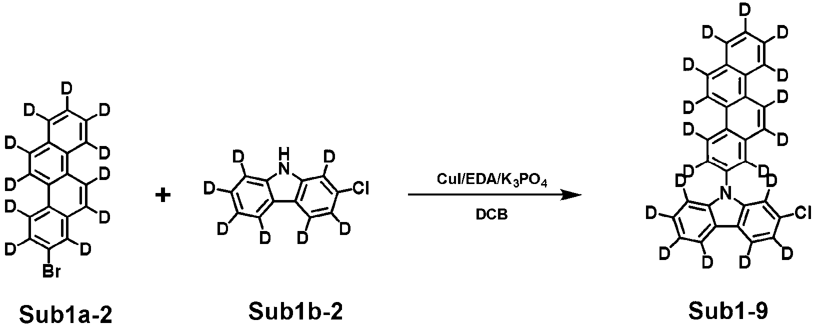

Sub1a-2 (20.0 g, 62.84 mmol) was dissolved in DCB (Dichlorobenzene) (157 mL) in a round-bottom flask, Sub1b-2 (14.4 g, 69.13 mmol), K₃PO₄ (26.6 g, 125.68 mmol), CuI (2.39 g, 12.57 mmol), EDA (Ethylenediamine) (1.51 g, 25.14 mmol) were added, and stirred at 230° C. After the reaction was completed, the mixture was extracted with CH₂Cl₂ and water, the organic layer was dried over MgSO₄ and concentrated. Afterwards, the generated compound was recrystallized after applying a silica gel column to obtain 15.9 g of the product (yield 57%).

### 3. Synthesis example of Sub1-16

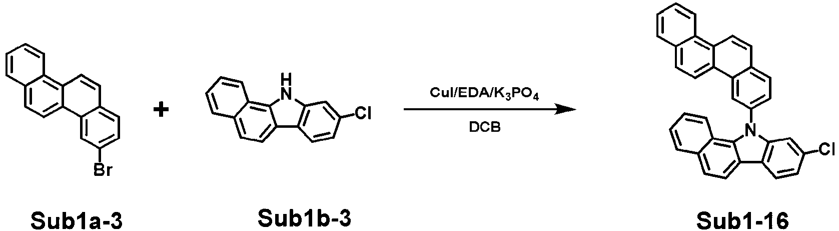

Sub1a-3 (18.0 g, 58.60 mmol) was dissolved in DCB (Dichlorobenzene) (146 mL) in a round-bottom flask, Sub1b-3 (16.2 g, 64.46 mmol), K₃PO₄ (24.8 g, 117.19 mmol), CuI (2.23 g, 11.72 mmol), EDA (Ethylenediamine) (1.41 g, 23.44 mmol) were added, and stirred at 230° C. After the reaction was completed, the mixture was extracted with CH₂Cl₂ and water, the organic layer was dried over MgSO₄ and concentrated, and the resulting compound was recrystallized using a silicagel column to obtain 16.80 g of the product (yield: 60%).

### 4. Synthesis example of Sub1-32

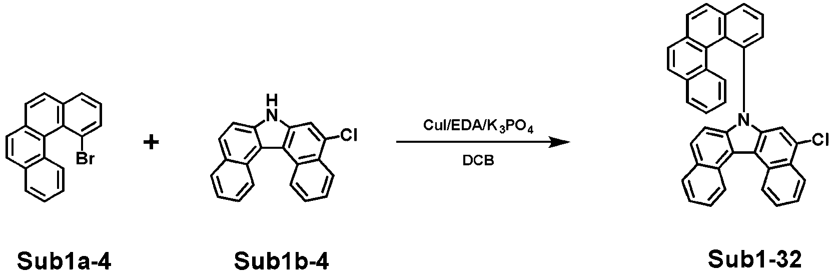

Sub1a-4 (21.0 g, 68.36 mmol) was dissolved in DCB (Dichlorobenzene) (170 mL) in a round-bottom flask, Sub1b-4 (22.7 g, 75.20 mmol), K₃PO₄ (29.0 g, 136.72 mmol), CuI (2.6 g, 13.67 mmol), EDA (Ethylenediamine) (1.64 g, 27.34 mmol) were added, and stirred at 230° C. After the reaction was completed, the mixture was extracted with CH₂Cl₂ and water, the organic layer was dried over MgSO₄ and concentrated. Afterwards, the generated compound was recrystallized after applying a silica gel column to obtain 23.8 g of the product (yield 66%).

### 5. Synthesis example of Sub1-42

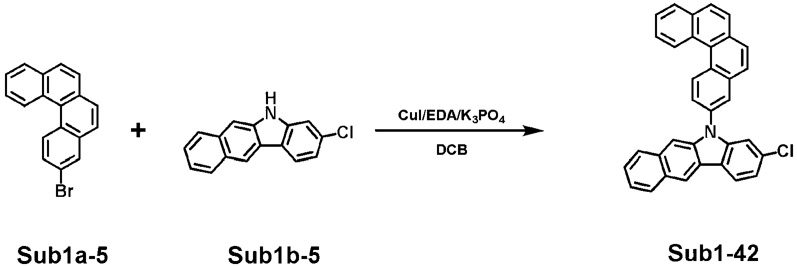

Sub1a-5 (25.0 g, 81.38 mmol) was dissolved in DCB (Dichlorobenzene) (203 mL) in a round-bottom flask, Sub1b-5 (22.5 g, 89.52 mmol), K₃PO₄ (34.5 g, 162.7 mmol), CuI (3.1 g, 16.28 mmol), EDA (Ethylenediamine) (1.9 g, 32.55 mmol) were added, and stirred at 230° C. After the reaction was completed, the mixture was extracted with CH₂Cl₂ and water, the organic layer was dried over MgSO₄ and concentrated. Afterwards, the generated compound was recrystallized after applying a silica gel column to obtain 26.0 g of the product (yield 67%).

Compounds belonging to Sub1 may be, but are not limited to, the compounds below, and Table 1 shows the FD-MS (Field Desorption-Mass Spectrometry) values of compounds belonging to Sub1.

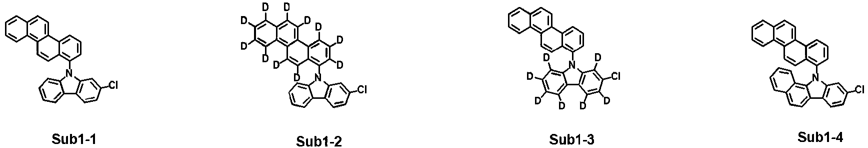

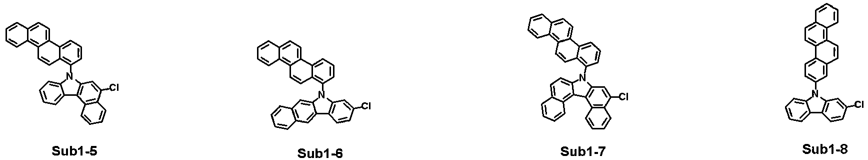

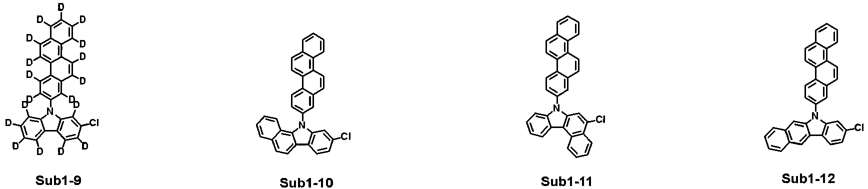

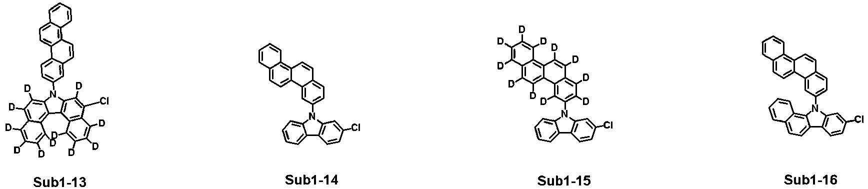

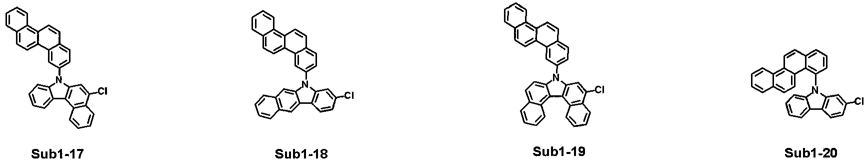

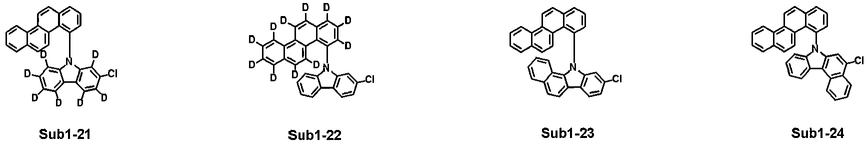

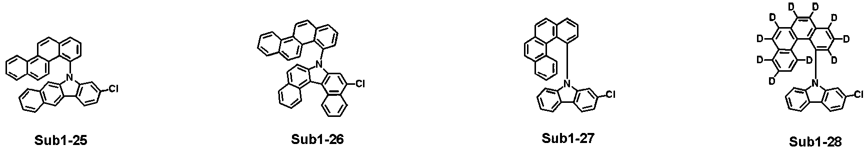

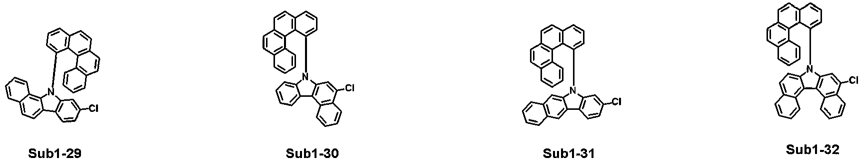

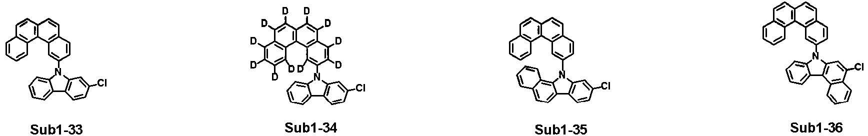

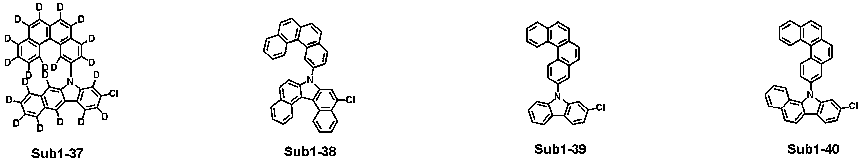

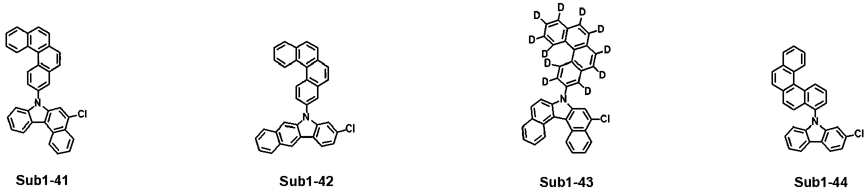

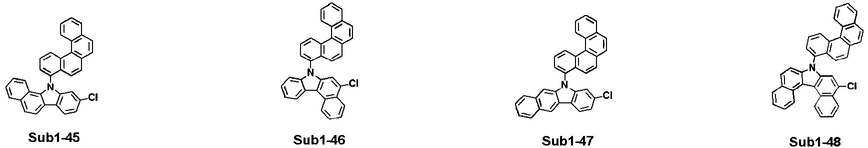

**[Table 1]**

| compound | FD-MS | compound | FD-MS |
|---|---|---|---|
| Sub1-1 | m/z=427.11(C₃₀H₁₈ClN=427.93) | Sub1-2 | m/z=438.18(C₃₀H₇D₁₁ClN=439.00) |
| Sub1-3 | m/z=434.16(C₃₀H₁₁D₇ClN=434.97) | Sub1-4 | m/z=477.13(C₃₄H₂₀ClN=477.99) |
| Sub1-5 | m/z=477.13(C₃₄H₂₀ClN=477.99) | Sub1-6 | m/z=477.13(C₃₄H₂₀ClN=477.99) |
| Sub1-7 | m/z=527.14(C₃₈H₂₂ClN=528.05) | Sub1-8 | m/z=427.11(C₃₀H₁₈ClN=427.93) |
| Sub1-9 | m/z=445.23(C₃₀D₁₈ClN=446.04) | Sub1-10 | m/z=477.13(C₃₄H₂₀ClN=477.99) |
| Sub1-11 | m/z=477.13(C₃₄H₂₀ClN=477.99) | Sub1-12 | m/z=477.13(C₃₄H₂₀ClN=477.99) |
| Sub1-13 | m/z=538.21(C₃₈H₁₁D₁₁ClN=539.12) | Sub1-14 | m/z=427.11(C₃₀H₁₈ClN=427.93) |
| Sub1-15 | m/z=438.18(C₃₀H₇D₁₁ClN=439) | Sub1-16 | m/z=477.13(C₃₄H₂₀ClN=477.99) |
| Sub1-17 | m/z=477.13(C₃₄H₂₀ClN=477.99) | Sub1-18 | m/z=477.13(C₃₄H₂₀ClN=477.99) |
| Sub1-19 | m/z=527.14(C₃₈H₂₂ClN=528.05) | Sub1-20 | m/z=427.11(C₃₀H₁₈ClN=427.93) |
| Sub1-21 | m/z=434.16(C₃₀H₁₁D₇ClN=434.97) | Sub1-22 | m/z=438.18(C₃₀H₇D₁₁ClN=439) |
| Sub1-23 | m/z=477.13(C₃₄H₂₀ClN=477.99) | Sub1-24 | m/z=477.13(C₃₄H₂₀ClN=477.99) |
| Sub1-25 | m/z=477.13(C₃₄H₂₀ClN=477.99) | Sub1-26 | m/z=527.14(C₃₈H₂₂ClN=528.05) |
| Sub1-27 | m/z=427.11(C₃₀H₁₈ClN=427.93) | Sub1-28 | m/z=437.18(C₃₀H₈D₁₀ClN=437.99) |
| Sub1-29 | m/z=477.13(C₃₄H₂₀ClN=477.99) | Sub1-30 | m/z=477.13(C₃₄H₂₀ClN=477.99) |
| Sub1-31 | m/z=477.13(C₃₄H₂₀ClN=477.99) | Sub1-32 | m/z=527.14(C₃₈H₂₂ClN=528.05) |
| Sub1-33 | m/z=427.11(C₃₀H₁₈ClN=427.93) | Sub1-34 | m/z=438.18(C₃₀H₇D₁₁ClN=439) |
| Sub1-35 | m/z=477.13(C₃₄H₂₀ClN=477.99) | Sub1-36 | m/z=477.13(C₃₄H₂₀ClN=477.99) |
| Sub1-37 | m/z=497.25(C₃₄D₂₀ClN=498.11) | Sub1-38 | m/z=527.14(C₃₈H₂₂ClN=528.05) |
| Sub1-39 | m/z=427.11(C₃₀H₁₈ClN=427.93) | Sub1-40 | m/z=477.13(C₃₄H₂₀ClN=477.99) |
| Sub1-41 | m/z=477.13(C₃₄H₂₀ClN=477.99) | Sub1-42 | m/z=477.13(C₃₄H₂₀ClN=477.99) |
| Sub1-43 | m/z=538.21(C₃₈H₁₁D₁₁ClN=539.12) | Sub 1-44 | m/z=427.11(C₃₀H₁₈ClN=427.93) |
| Sub1-45 | m/z=477.13(C₃₄H₂₀ClN=477.99) | Sub1-46 | m/z=477.13(C₃₄H₂₀ClN=477.99) |
| Sub1-47 | m/z=477.13(C₃₄H₂₀ClN=477.99) | Sub1-48 | m/z=527.14(C₃₈H₂₂ClN=528.05) |

### II. Synthesis of Sub2

Sub2 of the reaction scheme 1 is synthesized by the reaction path of the following reaction scheme 3, but is not limited thereto.

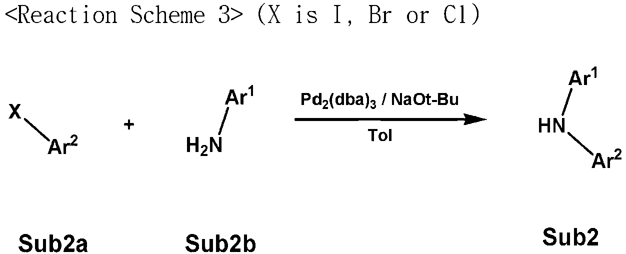

### 1. Synthesis example of Sub2-1

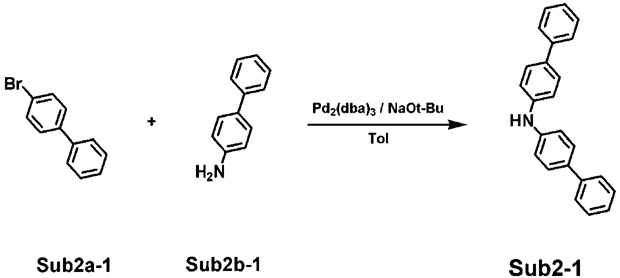

Sub2b-1 (17.4 g, 102.9 mmol) was dissolved in Toluene (340 mL) in a round-bottom flask, Sub2a-1 (16.0 g, 68.6 mmol), NaOt-Bu (13.1 g, 137.2 mmol), Pd₂(dba)₃ (1.8 g, 2.0 mmol), 50wt% P(t-Bu) ₃ (2.0 ml, 4.1 mmol) were added, and stirred at 110° C. After the reaction was completed, the organic layer was extracted with water, dried with MgSO₄, and concentrated. Afterwards, the generated compound was recrystallized after applying a silica gel column to obtain 11.6 g of the product (yield 53%).

### 2. Synthesis example of Sub2-8

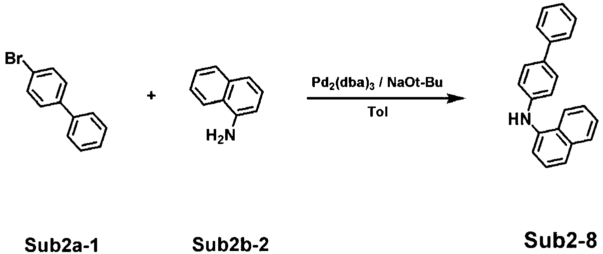

Sub2b-2 (20.27 g, 141.5 mmol) was dissolved in Toluene (470 mL) in a round-bottom flask, Sub2a-1 (22.0 g, 94.38 mmol), NaOt-Bu (18.1 g, 188.7 mmol), Pd₂(dba)₃ (2.5 g, 2.8 mmol), 50wt% P(t-Bu) ₃ (2.75 ml, 5.6 mmol) were added, and stirred at 110° C. After the reaction was completed, the organic layer was extracted with water, dried with MgSO₄, and concentrated. Afterwards, the generated compound was recrystallized after applying a silica gel column to obtain 17.0 g of the product (yield 61%).

### 3. Synthesis example of Sub2-37

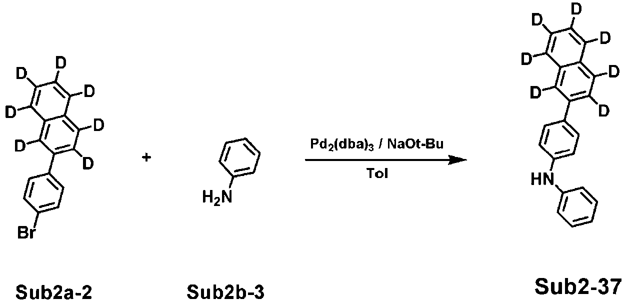

Sub2b-3 (11.55 g, 124.0 mmol) was dissolved in Toluene (413 mL) in a round-bottom flask, Sub2a-2 (24.0 g, 82.7 mmol), NaOt-Bu (15.9 g, 165.4 mmol), Pd₂(dba)₃ (2.2 g, 2.4 mmol), 50wt% P(t-Bu) ₃ (2.4 ml, 4.9 mmol) were added, and stirred at 110° C. After the reaction was completed, the organic layer was extracted with water, dried with MgSO₄, and concentrated. Afterwards, the generated compound was recrystallized after applying a silica gel column to obtain 16.7 g of the product (yield 67%).

### 4. Synthesis example of Sub2-52

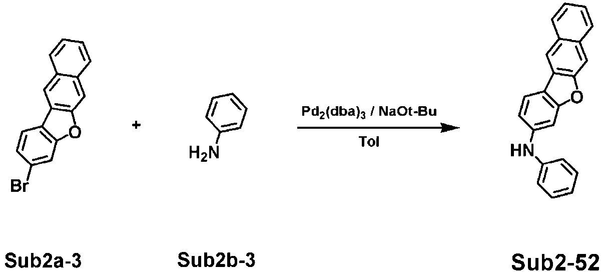

Sub2b-3 (9.4 g, 100.9 mmol) was dissolved in Toluene (336 mL) in a round-bottom flask, Sub2a-3 (20.0 g, 67.3 mmol), NaOt-Bu (12.9 g, 134.6 mmol), Pd₂(dba)₃ (1.8 g, 2.02 mmol), 50wt% P(t-Bu) ₃ (1.96 ml, 4.04 mmol) were added, and stirred at 110° C. After the reaction was completed, the organic layer was extracted with water, dried with MgSO₄, and concentrated. Afterwards, the generated compound was recrystallized after applying a silica gel column to obtain 13.32 g of the product (yield 64%).

### 5. Synthesis example of Sub2-73

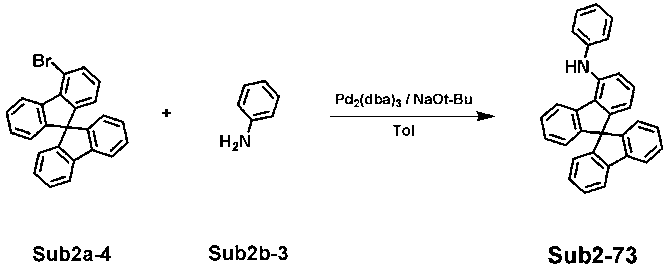

Sub2b-3 (6.7 g, 72.10 mmol) was dissolved in Toluene (240 mL) in a round-bottom flask, Sub2a-4 (19.0 g, 48.06 mmol), NaOt-Bu (9.24 g, 96.13 mmol), Pd₂(dba)₃ (1.32 g, 1.44 mmol), 50wt% P(t-Bu) ₃ (1.40 ml, 2.88 mmol) were added, and stirred at 110° C. After the reaction was completed, the organic layer was extracted with water, dried with MgSO₄, and concentrated. Afterwards, the generated compound was recrystallized after applying a silica gel column to obtain 10.18 g of the product (yield 52%).

Compounds belonging to Sub2 may include, but are not limited to, the compounds below, and Table 2 shows the FD-MS values of compounds belonging to Sub2.

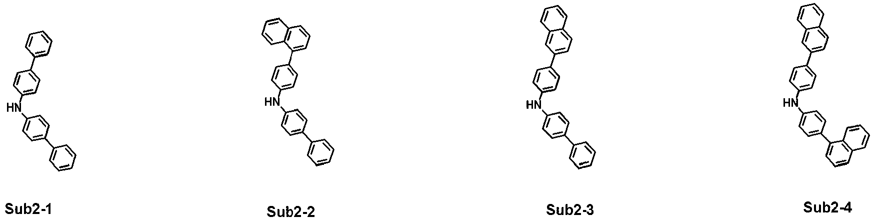

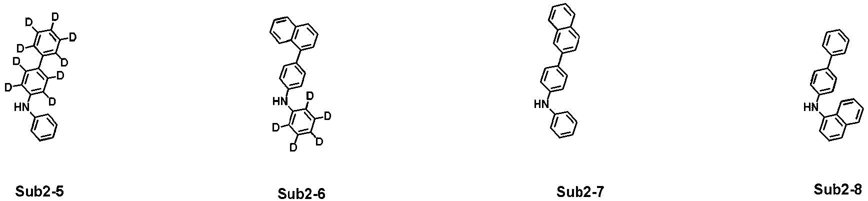

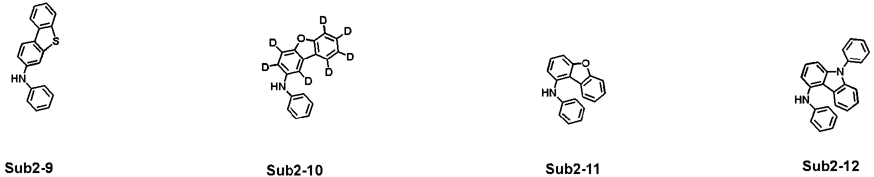

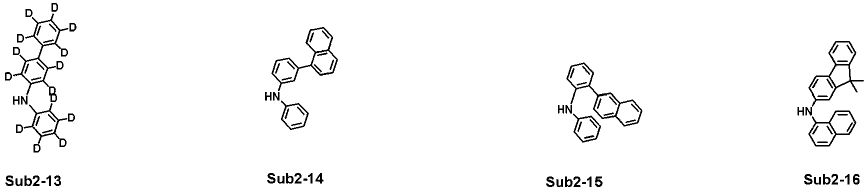

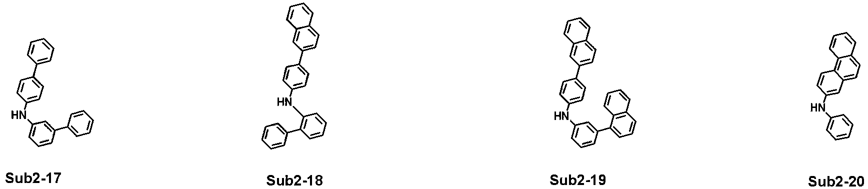

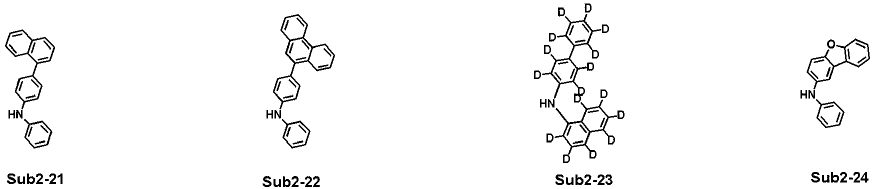

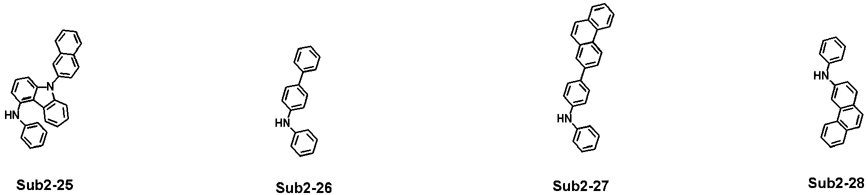

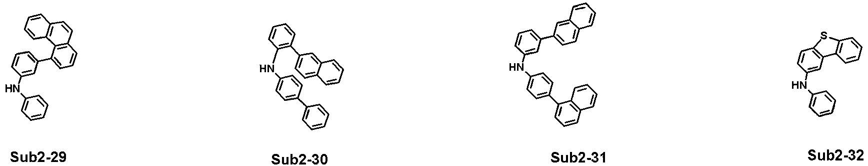

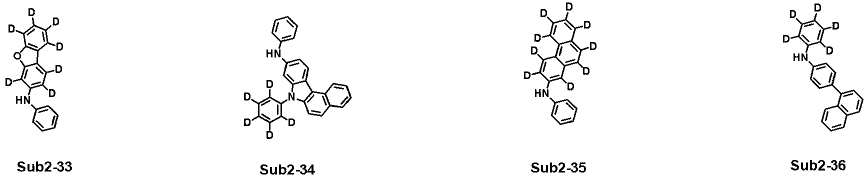

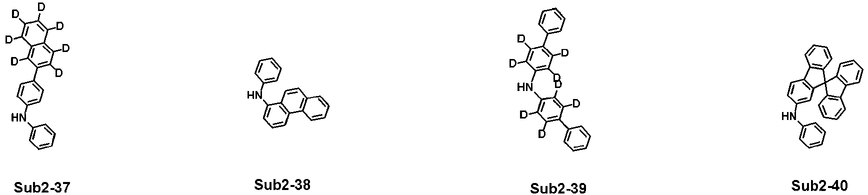

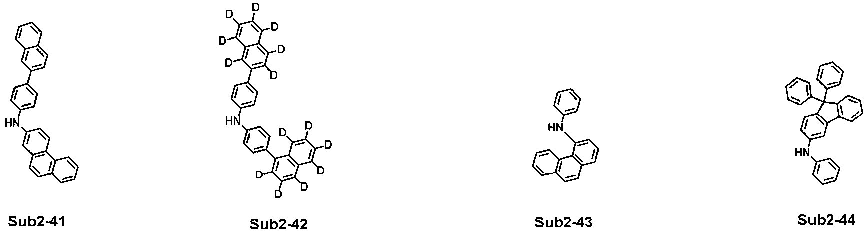

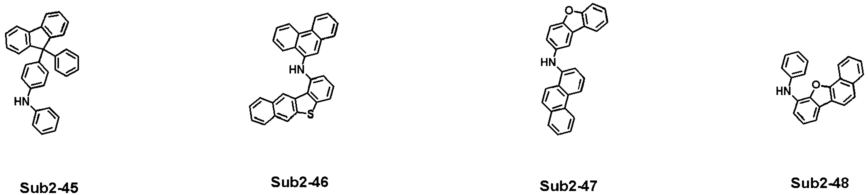

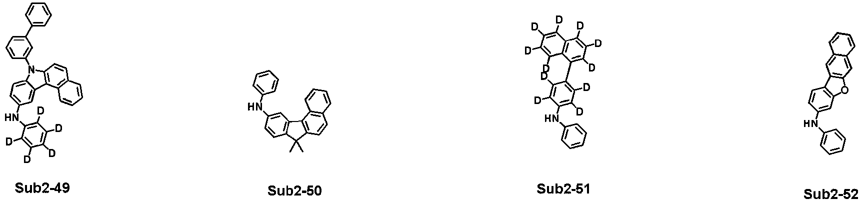

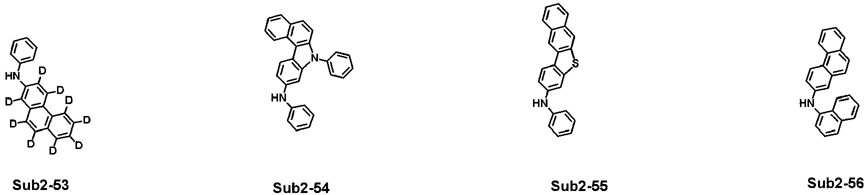

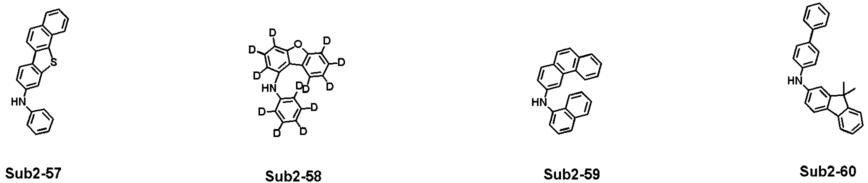

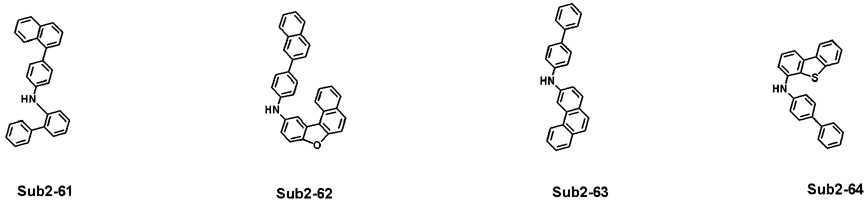

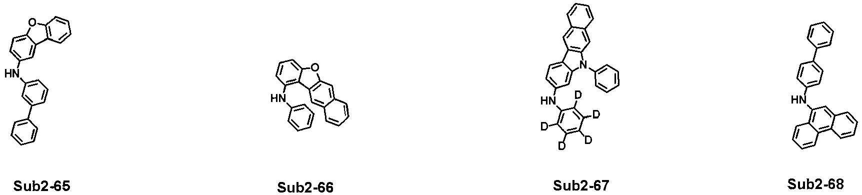

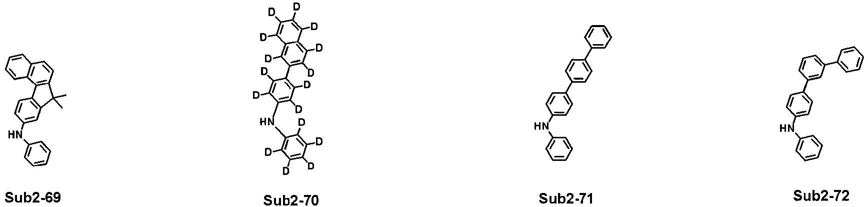

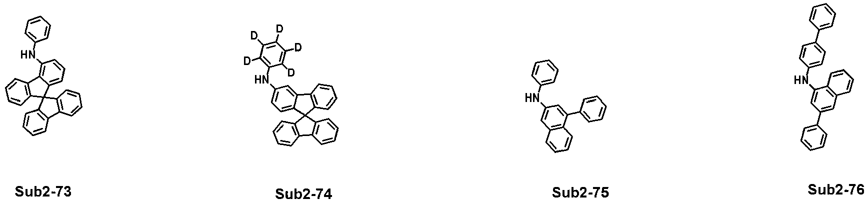

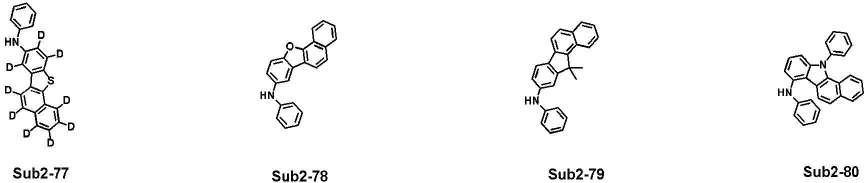

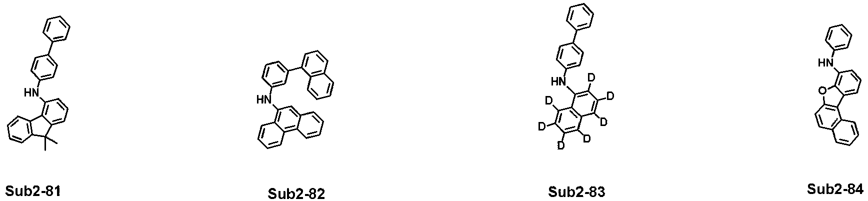

**[Table 2]**

| compound | FD-MS | compound | FD-MS |
|---|---|---|---|
| Sub2-1 | m/z=321.15(C₂₄H₁₉N=321.42) | Sub2-2 | m/z=371.17(C₂₈H₂₁N=371.48) |
| Sub2-3 | m/z=371.17(C₂₈H₂₁N=371.48) | Sub2-4 | m/z=421.18(C₃₂H₂₃N=421.54) |
| Sub2-5 | m/z=254.18(C₁₈H₆D₉N=254.38) | Sub2-6 | m/z=300.17(C₂₂H₁₂D₅N=300.42) |
| Sub2-7 | m/z=295.14(C₂₂H₁₇N=295.38) | Sub2-8 | m/z=295.14(C₂₂H₁₇N=295.38) |
| Sub2-9 | m/z=275.08(C₁₈H₁₃NS=275.37) | Sub2-10 | m/z=266.14(C₁₈H₆D₇NO=266.35) |
| Sub2-11 | m/z=259.1(C₁₈H₁₃NO=259.31) | Sub2-12 | m/z=334.15(C₂₄H₁₈N₂=334.42) |
| Sub2-13 | m/z=259.21(C₁₈HD₁₄N=259.41) | Sub2-14 | m/z=295.14(C₂₂H₁₇N=295.38) |
| Sub2-15 | m/z=295. 14(C₂₂H₁₇N=295.38) | Sub2-16 | m/z=335.17(C₂₅H₂₁N=335.45) |
| Sub2-17 | m/z=321.15(C₂₄H₁₉N=321.42) | Sub2-18 | m/z=371.17(C₂₈H₂₁N=371.48) |
| Sub2-19 | m/z=421.18(C₃₂H₂₃N=421.54) | Sub2-20 | m/z=269.12(C₂₀H₁₅N=269.35) |
| Sub2-21 | m/z=295.14(C₂₂H₁₇N=295.38) | Sub2-22 | m/z=345.15(C₂₆H₁₉N=345.44) |
| Sub2-23 | m/z=311.24(C₂₂HD₁₆N=311.48) | Sub2-24 | m/z=259.1(C₁₈H₁₃NO=259.31) |
| Sub2-25 | m/z=384.16(C₂₈H₂₀N₂=384.48) | Sub2-26 | m/z=245.12(C₁₈H₁₅N=245.32) |
| Sub2-27 | m/z=345.15(C₂₆H₁₉N=345.44) | Sub2-28 | m/z=269.12(C₂₀H₁₅N=269.35) |
| Sub2-29 | m/z=345.15(C₂₆H₁₉N=345.44) | Sub2-30 | m/z=371.17(C₂₈H₂₁N=371.48) |
| Sub2-31 | m/z=421.18(C₃₂H₂₃N=421.54) | Sub2-32 | m/z=275.08(C₁₈H₁₃NS=275.37) |
| Sub2-33 | m/z=266.14(C₁₈H₆D₇NO=266.35) | Sub2-34 | m/z=389.19(C₂₈H₁₅D₅N₂=389.51) |
| Sub2-35 | m/z=278.18(C₂₀H₆D₉N=278.4) | Sub2-36 | m/z=300.17(C₂₂H₁₂D₅N=300.42) |
| Sub2-37 | m/z=302.18(C₂₂H₁₀D₇N=302.43) | Sub2-38 | m/z=269.12(C₂₀H₁₅N=269.35) |
| Sub2-39 | m/z=329.2(C₂₄H₁₁D₈N=329.47) | Sub2-40 | m/z=407.17(C₃₁H₂₁N=407.52) |
| Sub2-41 | m/z=395.17(C₃₀H₂₁N=395.5) | Sub2-42 | m/z=435.27(C₃₂H₉D₁₄N=435.63) |
| Sub2-43 | m/z=269.12(C₂₀H₁₅N=269.35) | Sub2-44 | m/z=409.18(C₃₁H₂₃N=409.53) |
| Sub2-45 | m/z=409.18(C₃₁H₂₃N=409.53) | Sub2-46 | m/z=425.12(C₃₀H₁₉NS=425.55) |
| Sub2-47 | m/z=359.13(C₂₆H₁₇NO=359.43) | Sub2-48 | m/z=309.12(C₂₂H₁₅NO=309.37) |
| Sub2-49 | m/z=465.23(C₃₄H₁₉D₅N₂=465.61) | Sub2-50 | m/z=335.17(C₂₅H₂₁N=335.45) |
| Sub2-51 | m/z=306.21(C₂₂H₆D₁₁N=306.45) | Sub2-52 | m/z=309.12(C₂₂H₁₅NO=309.37) |
| Sub2-53 | m/z=278.4(C₂₈H₂₀N₂=278.18) | Sub2-54 | m/z=384.48(C₂₂H₁₅NS=384.16) |
| Sub2-55 | m/z=325.43(C₂₄H₁₇N=325.09) | Sub2-56 | m/z=319.41(C₂₂H₁₅NS=319.14) |
| Sub2-57 | m/z=325.43(C₁₈HD₁₂NO=325.09) | Sub2-58 | m/z=271.38(C₂₄H₁₇N=271.18) |
| Sub2-59 | m/z=319.41(C₂₇H₂₃N=319.14) | Sub2-60 | m/z=361.49(C₂₈H₂₁N=361.18) |
| Sub2-61 | m/z=371.48(C₃₂H₂₁NO=371.17) | Sub2-62 | m/z=435.53(C₂₆H₁₉N=435.16) |
| Sub2-63 | m/z=345.44(C₂₄H₁₇NS=345.15) | Sub2-64 | m/z=351.47(C₂₄H₁₇NO=351.11) |
| Sub2-65 | m/z=335.41(C₂₂H₁₅NO=335.13) | Sub2-66 | m/z=309.37(C₂₈H₁₅D₅N₂=309.12) |
| Sub2-67 | m/z=389.51(C₂₆H₁₉N=389.19) | Sub2-68 | m/z=345.44(C₂₅H₂₁N=345.15) |
| Sub2-69 | m/z=335.45(C₂₂HD₁₆N=335.17) | Sub2-70 | m/z=311.48(C₂₄H₁₉N=311.24) |
| Sub2-71 | m/z=321.42(C₂₄H₁₉N=321.15) | Sub2-72 | m/z=321.42(C₃₁H₂₁N=321.15) |
| Sub2-73 | m/z=407.52(C₃₁H₁₆D₅N=407.17) | Sub2-74 | m/z=412.2(C₃₁H₁₆D₅N=412.55) |
| Sub2-75 | m/z=295.14(C₂₂H₁₇N=295.38) | Sub2-76 | m/z=371.17(C₂₈H₂₁N=371.48) |
| Sub2-77 | m/z=334.15(C₂₂H₆D₉NS=334.48) | Sub2-78 | m/z=309.12(C₂₂H₁₅NO=309.37) |
| Sub2-79 | m/z=335.17(C₂₅H₂₁N=335.45) | Sub2-80 | m/z=384.16(C₂₈H₂₀N₂=384.48) |
| Sub2-81 | m/z=361.18(C₂₇H₂₃N=361.49) | Sub2-82 | m/z=395.17(C₃₀H₂₁N=395.5) |
| Sub2-83 | m/z=309.12(C₂₂H₁₅NO=309.37) | Sub2-84 | m/z=309.12(C₂₂H₁₅NO=309.37) |

### III. Synthesis of Final Product

### 1. Synthesis example of P-1

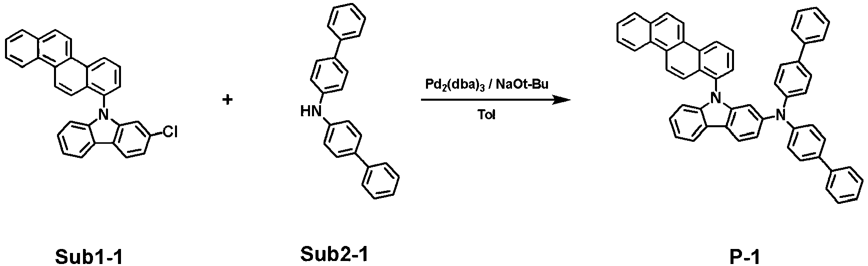

Sub2-1 (12.0 g, 37.39 mmol) was dissolved in Toluene (186 mL) in a round-bottom flask, Sub1-1 (16 g, 37.39 mmol), NaOt-Bu (7.19 g, 74.78 mmol), Pd₂(dba)₃ (1.03 g, 1.12 mmol), 50wt% P(t-Bu) ₃ (1.09 ml, 2.24 mmol) were added, and stirred at 110° C. After the reaction was completed, the organic layer was extracted with water, dried with MgSO₄, and concentrated. Afterwards, the generated compound was recrystallized after applying a silica gel column to obtain 16.25 g of the product (yield 61%).

### 2. Synthesis example of P-24

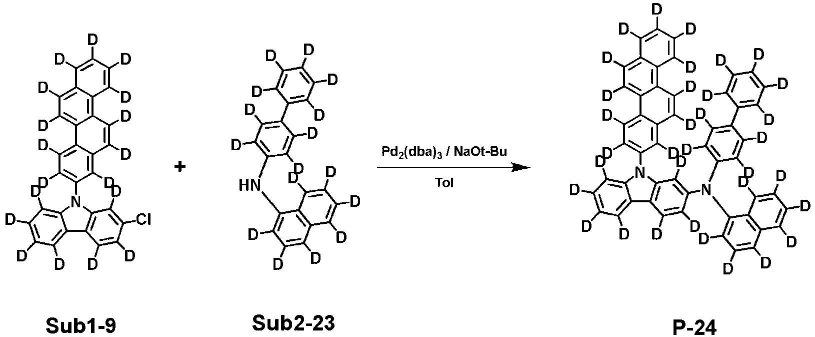

Sub2-23 (10.34 g, 33.18 mmol) was dissolved in Toluene (165 mL) in a round-bottom flask, Sub1-9 (14.8 g, 33.18 mmol), NaOt-Bu (6.38 g, 66.36 mmol), Pd₂(dba)₃ (0.91 g, 1.00 mmol), 50wt% P(t-Bu) ₃ (0.97 ml, 1.99 mmol) were added, and stirred at 110° C. After the reaction was completed, the organic layer was extracted with water, dried with MgSO₄, and concentrated. Afterwards, the generated compound was recrystallized after applying a silica gel column to obtain 13.39 g of the product (yield 56%).

### 3. Synthesis example of P-43

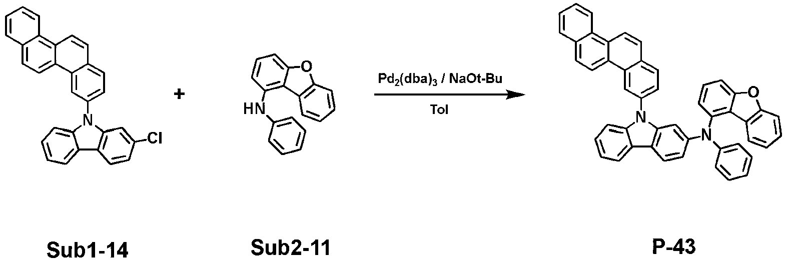

Sub2-11 (8.12 g, 31.31 mmol) was dissolved in Toluene (156 mL) in a round-bottom flask, Sub1-14 (13.4 g, 31.31 mmol), NaOt-Bu (6.02 g, 62.63 mmol), Pd₂(dba)₃ (0.86 g, 0.94 mmol), 50wt% P(t-Bu) ₃ (0.91 ml, 1.88 mmol) were added, and stirred at 110° C. After the reaction was completed, the organic layer was extracted with water, dried with MgSO₄, and concentrated. Afterwards, the generated compound was recrystallized after applying a silica gel column to obtain 13.44 g of the product (yield 66%).

### 4. Synthesis example of P-55

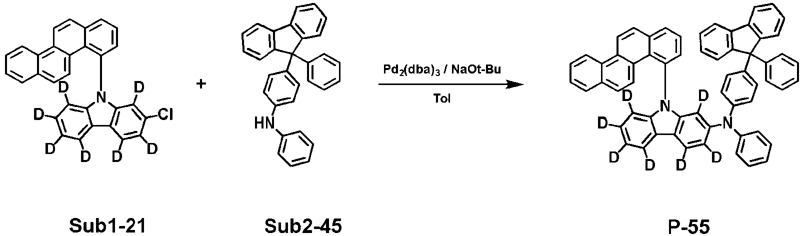

Sub2-45 (11.39 g, 27.82 mmol) was dissolved in Toluene (139 mL) in a round-bottom flask, Sub1-21 (12.1 g, 27.82 mmol), NaOt-Bu (5.35 g, 55.64 mmol), Pd₂(dba)₃ (0.76 g, 0.83 mmol), 50wt% P(t-Bu) ₃ (0.81 ml, 1.67 mmol) were added, and stirred at 110° C. After the reaction was completed, the organic layer was extracted with water, dried with MgSO₄, and concentrated. Afterwards, the generated compound was recrystallized after applying a silica gel column to obtain 11.68 g of the product (yield 52%).

### 5. Synthesis example of P-70

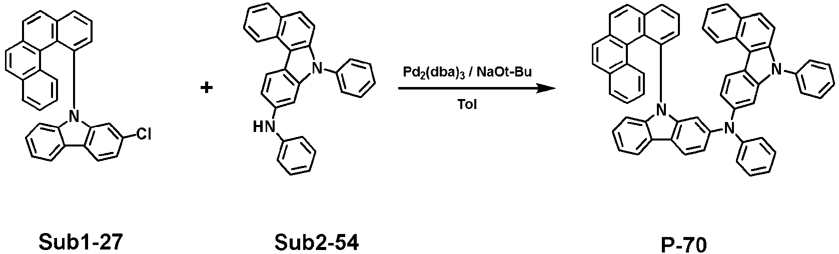

Sub2-54 (15.27 g, 39.73 mmol) was dissolved in Toluene (198 mL) in a roundbottom flask, Sub1-27 (17 g, 39.73 mmol), NaOt-Bu (7.64 g, 79.45 mmol), Pd₂(dba)₃ (1.09 g, 1.19 mmol), 50wt% P(t-Bu) ₃ (1.16 ml, 2.38 mmol) were added, and stirred at 110° C. After the reaction was completed, the organic layer was extracted with water, dried with MgSO₄, and concentrated. Afterwards, the generated compound was recrystallized after applying a silica gel column to obtain 19.72 g of the product (yield 64%).

### 6. Synthesis example of P-81

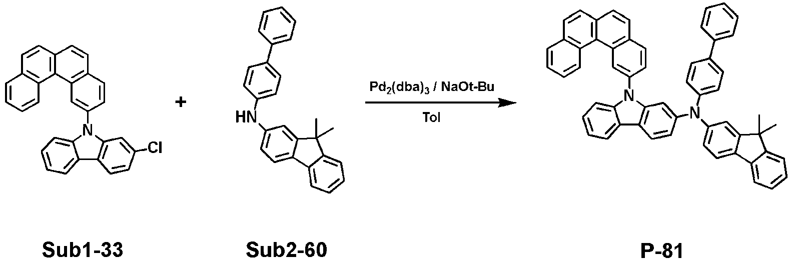

Sub2-60 (13.35 g, 36.92 mmol) was dissolved in Toluene (184 mL) in a roundbottom flask, Sub1-33 (15.8 g, 36.92 mmol), NaOt-Bu (7.10 g, 73.84 mmol), Pd₂(dba)₃ (1.01 g, 1.11 mmol), 50wt% P(t-Bu) ₃ (1.07 ml, 2.22 mmol) were added, and stirred at 110° C. After the reaction was completed, the organic layer was extracted with water, dried with MgSO₄, and concentrated. Afterwards, the generated compound was recrystallized after applying a silica gel column to obtain 16.68 g of the product (yield 60%).

### 7. Synthesis example of P-86

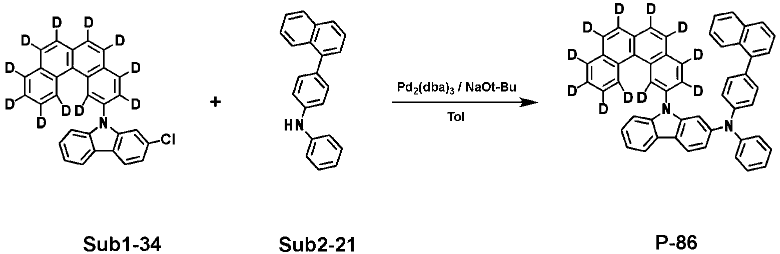

Sub2-21 (14.13 g, 47.84 mmol) was dissolved in Toluene (239 mL) in a roundbottom flask, Sub1-34 (21 g, 47.84 mmol), NaOt-Bu (9.2 g, 95.67 mmol), Pd₂(dba)₃ (1.31 g, 1.44 mmol), 50wt% P(t-Bu) ₃ (1.39 ml, 2.87 mmol) were added, and stirred at 110° C. After the reaction was completed, the organic layer was extracted with water, dried with MgSO₄, and concentrated. Afterwards, the generated compound was recrystallized after applying a silica gel column to obtain 18.69 g of the product (yield 56%).

### 8. Synthesis example of P-94

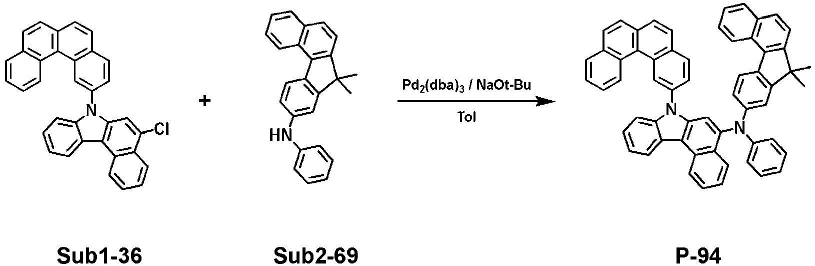

Sub2-69 (11.02 g, 32.85 mmol) was dissolved in Toluene (164 mL) in a roundbottom flask, Sub1-36 (15.7 g, 32.85 mmol), NaOt-Bu (6.31 g, 65.69 mmol), Pd₂(dba)₃ (0.90 g, 0.99 mmol), 50wt% P(t-Bu) ₃ (0.96 ml, 1.97 mmol) were added, and stirred at 110° C. After the reaction was completed, the organic layer was extracted with water, dried with MgSO₄, and concentrated. Afterwards, the generated compound was recrystallized after applying a silica gel column to obtain 16.07 g of the product (yield 63%).

### 9. Synthesis example of P-99

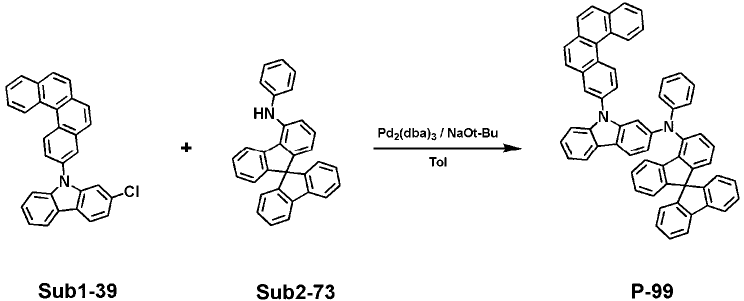

Sub2-73 (19.05 g, 46.74 mmol) was dissolved in Toluene (233 mL) in a roundbottom flask, Sub1-39 (20 g, 46.74 mmol), NaOt-Bu (8.98 g, 93.47 mmol), Pd₂(dba)₃ (1.28 g, 1.40 mmol), 50wt% P(t-Bu) ₃ (1.36 ml, 2.80 mmol) were added, and stirred at 110° C. After the reaction was completed, the organic layer was extracted with water, dried with MgSO₄, and concentrated. Afterwards, the generated compound was recrystallized after applying a silica gel column to obtain 23.52 g of the product (yield 63%).

### 10. Synthesis example of P-121

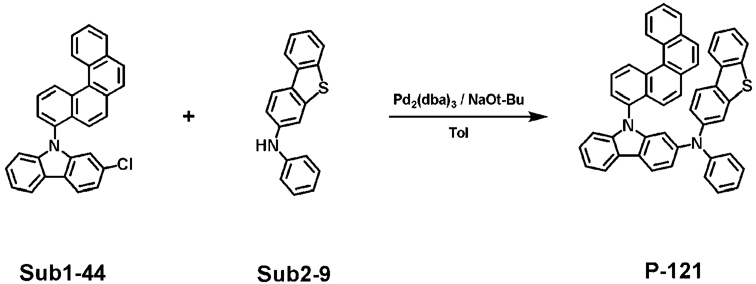

Sub2-9 (10.49 g, 38.09 mmol) was dissolved in Toluene (190 mL) in a roundbottom flask, Sub1-44 (16.3 g, 38.09 mmol), NaOt-Bu (7.32 g, 76.18 mmol), Pd₂(dba)₃ (1.05 g, 1.14 mmol), 50wt% P(t-Bu) ₃ (1.11 ml, 2.29 mmol) were added, and stirred at 110° C. After the reaction was completed, the organic layer was extracted with water, dried with MgSO₄, and concentrated. Afterwards, the generated compound was recrystallized after applying a silica gel column to obtain 12.95 g of the product (yield 51%).

Meanwhile, the FD-MS values of compounds P-1 to P-128 of the present invention manufactured according to the synthetic examples are as shown in Table 3.

**[Table 3]**

| compound | FD-MS | compound | FD-MS |
|---|---|---|---|
| P-1 | m/z=712.29(C₅₄H₃₆N₂=712.9) | P-2 | m/z=762 . 3 (C₅₈H₃₈N₂=762 . 96) |
| P-3 | m/z=762. 3(C₅₈H₃₈N₂=762 .96) | P-4 | m/z=812. 32(C₆₂H₄₀N₂=813.02 ) |
| P-5 | m/z=645.31(C₄₈H₂₃D₉N₂=645.85) | P-6 | m/z=691.3(C₅₂H₂₉D₅N₂=691.89) |
| P-7 | m/z=686.27(C₅₂H₃₄N₂=686.86) | P-8 | m/z=697.34(C₅₂H₂₃D₁₁N₂=697.93) |
| P-9 | m/z=666. 21(C₄₈H₃₀N₂S=666 . 84) | P-10 | m/z=657. 28(C₄₈H₂₃D₇N₂O=657. 82) |
| P-11 | m/z=650.24(C₄₈H₃₀N₂O=650.78) | P-12 | m/z=732 . 33(C₅₄H₂₈D₇N₃=732. 94) |
| P-13 | m/z=700.36(C₅₂H₂₀D₁₄N₂=700.94) | P-14 | m/z=736.29(C₅₆H₃₆N₂=736.92) |
| P-15 | m/z=736.29(C₅₆H₃₆N₂=736.92) | P-16 | m/z=826. 33(C₆₃H₄₂N₂=827 . 04) |
| P-17 | m/z=712.29(C₅₄H₃₆N₂=712.9) | P-18 | m/z=762.3(C₅₈H₃₈N₂=762.96) |
| P-19 | m/z=762,3(C₅₈H₃₈N₂=762.96) | P-20 | m/z=812.32(C₆₂H₄₀N₂=813.02) |
| P-21 | m/z=660 . 26(C₅₀H₃₂N₂=660 . 82) | P-22 | m/z=686. 27(C₅₂H₃₄N₂=686. 86) |
| P-23 | m/z=736.29(C₅₆H₃₆N₂=736.92) | P-24 | m/z=720.49(C₅₂D₃₄N₂=721.07) |
| P-25 | m/z=666. 21(C₄₈H₃₀N₂S=666.84) | P-26 | m/z=650.24(C₄₈H₃₀N₂O=650.78) |
| P-27 | m/z=650.24(C₄₈H₃₀N₂O=650.78) | P-28 | m/z=775.3(C₅₈H₃₇N₃=775.95) |
| P-29 | m/z=686.27(C₅₂H₃₄N₂=686.86) | P-30 | m/z=786.3(C₆₀H₃₈N₂=786.98) |
| P-31 | m/z=736.29(C₅₆H₃₆N₂=736.92) | P-32 | m/z=797.37(C₆₀H₂₇D₁₁N₂=798.05) |
| P-33 | m/z=660.26(C₅₀H₃₂N₂=660.82) | P-34 | m/z=736.29(C₅₆H₃₆N₂=736.92) |
| P-35 | m/z=762.3(C₅₈H₃₈N₂=762.96) | P-36 | m/z=812.32(C₆₂H₄₀N₂=813.02) |
| P-37 | m/z=661.41(C₄₈H₇D₂₅N₂=661.95) | P-38 | m/z=686.27(C₅₂H₃₄N₂=686. 86) |
| P-39 | m/z=686.27(C₅₂H₃₄N₂=686.86) | P-40 | m/z=636.26(C₄₈H₃₂N₂=636.80) |
| P-41 | m/z=666.21(C₄₈H₃₀N₂S=666.84) | P-42 | m/z=657.28(C₄₈H₂₃D₇N₂O=657.82) |
| P-43 | m/z=650.24(C₄₈H₃₀N₂O=650.78) | P-44 | m/z=780.33(C₅₈H₃₂D₅N₃=780.99) |
| P-45 | m/z=719.33(C₅₄H₂₅D₉N₂=719.93) | P-46 | m/z=741.32(C₅₆H₃₁D₅N₂=741.95) |
| P-47 | m/z=743.33(C₅₆H₂₉D₇N₂=743.96) | P-48 | m/z=760.29(C₅₈H₃₆N₂=760.94) |
| P-49 | m/z=720.34(C₅₄H₂₈D₈N₂=720.94) | P-50 | m/z=798.3(C₆₁H₃₈N₂=798 . 99) |
| P-51 | m/z=786.3(C₆₀H₃₈N₂=786.98) | P-52 | m/z=826.41(C₆₂H₂₆D₁₄N₂=827.1) |
| P-53 | m/z=660.26(C₅₀H₃₂N₂=660.82) | P-54 | m/z=800.32(C₆₁H₄₀N₂=801) |
| P-55 | m/z=807.36(C₆₁H₃₃D₇N₂=808.05) | P-56 | m/z=686.27(C₅₂H₃₄N₂=686. 86) |
| P-57 | m/z=816.26(C₆₀H₃₆N₂S=817.02) | P-58 | m/z=761.34(C₅₆H₂₃D₁₁N₂O=761.97) |
| P-59 | m/z=700.25(C₅₂H₃₂N₂O=700.84) | P-60 | m/z=856.36(C₆₄H₃₆D₅N₃=857.08) |
| P-61 | m/z=776.32(C₅₉H₄₀N₂=776.98) | P-62 | m/z=747.36(C₅₆H₂₅D₁₁N₂=747.99) |
| P-63 | m/z=736.29(C₅₆H₃₆N₂=736.92) | P-64 | m/z=786.3(C₆₀H₃₈N₂=786.98) |
| P-65 | m/z=700.25(C₅₂H₃₂N₂O=700.84) | P-66 | m/z=762.3(C₅₈H₃₈N₂=762.96) |
| P-67 | m/z=762.3(C₅₈H₃₈N₂=762.96) | P-68 | m/z=812.32(C₆₂H₄₀N₂=813.02) |
| P-69 | m/z=669.31(C₅₀H₂₃D₉N₂=669.87) | P-70 | m/z=775.3(C₅₈H₃₇N₃=775.95) |
| P-71 | m/z=716.23(C₅₂H₃₂N₂S=716.9) | P-72 | m/z=720.33(C₅₄H₂₄D₁₀N₂=720.94) |
| P-73 | m/z=716.23(C₅₂H₃₂N₂S=716.9) | P-74 | m/z=650.24(C₄₈H₃₀N₂O=650.78) |
| P-75 | m/z=662.31(C₄₈H₁₈D₁₂N₂O=662.85) | P-76 | m/z=725.28(C₅₄H₃₅N₃=725.89) |
| P-77 | m/z=686.27(C₅₂H₃₄N₂=686.86) | P-78 | m/z=736.29(C₅₆H₃₆N₂=736.92) |
| P-79 | m/z=736.29(C₅₆H₃₆N₂=736.92) | P-80 | m/z=810.3(C₆₂H₃₈N₂=811) |
| P-81 | m/z=752.32(C₅₇H₄₀N₂=752 . 96) | P-82 | m/z=762.3(C₅₈H₃₈N₂=762.96) |
| P-83 | m/z=762.3(C₅₈H₃₈N₂=762.96) | P-84 | m/z=826.3(C₆₂H₃₈N₂O=827) |
| P-85 | m/z=736.29(C₅₆H₃₆N₂=736.92) | P-86 | m/z=697.34(C₅₂H₂₃D₁₁N₂=697.93) |
| P-87 | m/z=686.27(C₅₂H₃₄N₂=686.86) | P-88 | m/z=686.27(C₅₂H₃₄N₂=686. 86) |
| P-89 | m/z=742.24(C₅₄H₃₄N₂S=742.94) | P-90 | m/z=726.27(C₅₄H₃₄N₂O=726.88) |
| P-91 | m/z=700.25(C₅₂H₃₂N₂O=700.84) | P-92 | m/z=780.33(C₅₈H₃₂D₅N₃=780.99) |
| P-93 | m/z=786.3(C₆₀H₃₈N₂=786.98) | P-94 | m/z=776.32(C₅₉H₄₀N₂=776.98) |
| P-95 | m/z=772.51(C₅₆D₃₆N₂=773.14) | P-96 | m/z=812.32(C₆₂H₄₀N₂=813.02) |
| P-97 | m/z=712.29(C₅₄H₃₆N₂=712.9) | P-98 | m/z=762.3(C₅₈H₃₈N₂=762.96) |
| P-99 | m/z=798.3(C₆₁H₃₈N₂=798,99) | P-100 | m/z=812.32(C₆₂H₄₀N₂=813.02) |
| P-101 | m/z=636.26(C₄₈H₃₂N₂=636.8) | P-102 | m/z=803.33( C₆₁H₃₃D₅N₂=804.02) |
| P-103 | m/z=686.27(C₅₂H₃₄N₂=686,86) | P-104 | m/z=762.3(C₅₈H₃₈N₂=762.96) |
| P-105 | m/z=725.29(C₅₂H₂₃D₉N₂S=725.96) | P-106 | m/z=700.25(C₅₂H₃₂N₂O=700.84) |
| P-107 | m/z=650. 24(C₄₈H₃₀N₂O=650 . 78) | P-108 | m/z=775 . 3 (C₅₈H₃₇N₃=775 . 95) |
| P-109 | m/z=686. 27(C₅₂H₃₄N₂=686. 86) | P-110 | m/z=736.29(C₅₆H₃₆N₂=736.92) |
| P-111 | m/z=776 . 32 (C₅₉H₄₀N₂=776 . 98) | P-112 | m/z=797. 37(C₆₀H₂₇D₁₁N₂=798 . 05) |
| P-113 | m/z=752 . 32 (C₅₇H₄₀N₂=752 . 96) | P-114 | m/z=762 . 3 (C₅₈H₃₈N₂=762 . 96) |
| P-115 | m/z=762. 3(C₅₈H₃₈N₂=762.96) | P-116 | m/z=812. 32(C₆₂H₄₀N₂=813.02) |
| P-117 | m/z=636. 26(C₄₈H₃₂N₂=636. 8) | P-118 | m/z=786 . 3 (C₆₀H₃₈N₂=786. 98) |
| P-119 | m/z=686. 27(C₅₂H₃₄N₂=686.86) | P-120 | m/z=693.32(C₅₂H₂₇D₇N₂=693.9) |
| P-121 | m/z=666.21(C₄₈H₃₀N₂S=666.84) | P-122 | m/z=750.27(C₅₆H₃₄N₂O=750.9) |
| P-123 | m/z=700.25(C₅₂H₃₂N₂O=700.84) | P-124 | m/z=775 . 3 (C₅₈H₃₇N₃=775 . 95) |
| P-125 | m/z=686.27(C₅₂H₃₄N₂=686.86) | P-126 | m/z=736.29(C₅₆H₃₆N₂=736.92) |
| P-127 | m/z=736.29(C₅₆H₃₆N₂=736.92) | P-128 | m/z=786.3(C₆₀H₃₈N₂=786.98) |

### [Synthesis example 2]

The compound represented by Formula 2 can be prepared by a known synthetic method (named reaction) or by referring to published patent publications, for example, Korean Patent Publication Nos. 2020-0129334, 2022-0055392, 2023-000502, etc., but is not limited thereto.

Meanwhile, the FD-MS values of compounds N-1 to N-276 of the present invention are as shown in Table 4.

**[Table 4]**

| compound | FD-MS | compound | FD-MS |
|---|---|---|---|
| N-1 | m/z=399.14(C₂₇H₁₇N₃O=399.45) | N-2 | m/z=415.11(C₂₇H₁₇N₃S=415.51) |
| N-3 | m/z=474.18(C₃₃H₂₂N₄=474.57) | N-4 | m/z=449.15(C₃₁H₁₉N₃O=449.51) |
| N-5 | m/z=449.15(C₃₁H₁₉N₃O=449.51) | N-6 | m/z=515.15(C₃₅H₂₁N₃S=515.63) |
| N-7 | m/z=600.23(C₄₃H₂₈N₄=600.73) | N-8 | m/z=499.17(C₃₅H₂₁N₃O=499.57) |
| N-9 | m/z=551.20(C₃₉H₂₅N₃O=551.65) | N-10 | m/z=567.18(C₃₉H₂₅N₃S=567.71) |
| N-11 | m/z=702.28(C₅₁H₃₄N₄=702.86) | N-12 | m/z=657.22(C₄₆H₃₁N₃S=657.84) |
| N-13 | m/z=551.20(C₃₉H₂₅N₃O=551.65) | N-14 | m/z=541.16(C₃₇H₂₃N₃S=541.67) |
| N-15 | m/z=700.26(C₅₁H₃₂N₄=700.85) | N-16 | m/z=703.21(C₅₀H₂₉N₃S=703.86) |
| N-17 | m/z=525.18(C₃₇H₂₃N₃O=525.61) | N-18 | m/z=591.18(C₄₁H₂₅N₃S=591.73) |
| N-19 | m/z=627.24(C₄₄H₂₉N₅=627.75) | N-20 | m/z=524.20(C₃₇H₂₄N₄=524.63) |
| N-21 | m/z=551.20(C₃₉H₂₅N₃O=551.65) | N-22 | m/z=567.18(C₃₉H₂₅N₃S=567.71) |
| N-23 | m/z=702.28(C₅₁H₃₄N₄=702.86) | N-24 | m/z=474.18(C₃₃H₂₂N₄=474.57) |
| N-25 | m/z=779.29(C₅₇H₃₇N₃O=779.94) | N-26 | m/z=731. 24(C₅₂H₃₃N₃S=731.92) |
| N-27 | m/z=601.23(C₄₂H₂₇N₅=601.71) | N-28 | m/z=475.17(C₃₃H₂₁N₃O=475.55) |
| N-29 | m/z=641.21(C₄₅H₂₇N₃0₂=641.73) | N-30 | m/z=746.21(C₅₁H₃₀N₄OS=746.89) |
| N-31 | m/z=716.26(C₅₁H₃₂N₄O=716.84) | N-32 | m/z=681.19(C₄₇H₂₇N₃OS=681.81) |
| N-33 | m/z=475.17(C₃₃H₂₁N₃O=475.55) | N-34 | m/z=491.15(C₃₃H₂₁N₃S=491.61) |
| N-35 | m/z=550.22(C₃₉H₂₆N₄=550.67) | N-36 | m/z=525.18(C₃₇H₂₃N₃O=525.61) |
| N-37 | m/z=475.17(C₃₃H₂₁N₃O=475.55) | N-38 | m/z=491.15(C₃₃H₂₁N₃S=491.61) |
| N-39 | m/z=704.27(C₄₉H₃₂N₆=704.84) | N-40 | m/z=541.16(C₃₇H₂₃N₃S=541.67) |
| N-41 | m/z=551.20(C₃₉H₂₅N₃O=551.65) | N-42 | m/z=541.16(C₃₇H₂₃N₃S=541.67) |
| N-43 | m/z=626.25(C₄₅H₃₀N₄=626.76) | N-44 | m/z=676.26(C₄₉H₃₂N₄=676.82) |
| N-45 | m/z=551.2(C₃₉H₂₅N₃O=551.65) | N-46 | m/z=567.18(C₃₉H₂₅N₃S=567.71) |
| N-47 | m/z=614.25(C₄₄H₃₀N₄=614.75) | N-48 | m/z=575.17(C₃₉H₂₁N₅O=575.63) |
| N-49 | m/z=525.18(C₃₇H₂₃N₃O=525.61) | N-50 | m/z=541.16(C₃₇H₂₃N₃S=541.67) |
| N-51 | m/z=600.23(C₄₃H₂₈N₄=600.73) | N-52 | m/z=625.22(C₄₅H₂₇N₃O=625.73) |
| N-53 | m/z=525.18(C₃₇H₂₃N₃O=525.61) | N-54 | m/z=591.18(C₄₁H₂₅N₃S=591.73) |
| N-55 | m/z=600.23(C₄₃H₂₈N₄=600.73) | N-56 | m/z=693.22(C₄₉H₃₁N₃S=693.87) |
| N-57 | m/z=505.12(C₃₃H₁₉N₃OS=505.60) | N-58 | m/z=641.21(C₄₅H₂₇N₃O₂=641.73) |
| N-59 | m/z=571.12(C₃₇H₂₁N₃3₂=571.72) | N-60 | m/z=564.20(C₃₉H₂₄N₄O=564.65) |
| N-61 | m/z=581.16(C₃₉H₂₃N₃OS=581.69) | N-62 | m/z=521.10(C₃₃H₁₉N₃S₂=521.66) |
| N-63 | m/z=489.15(C₃₃H₁₉N₃O₂=489,53) | N-64 | m/z=640.23(C₄₅H₂₈N₄O=640.75) |
| N-65 | m/z=489.15(C₃₃H₁₉N₃O₂=489.53) | N-66 | m/z=505.12(C_{33 19}N₃OS=505.60) |
| N-67 | m/z=580.17(C₃₉H₂₄N₄S=580.71) | N-68 | m/z=564.20(C₃₉H₂₄N₄O=564.65) |
| N-69 | m/z=489.15(C₃₃H₁₉N₃O₂=489.53) | N-70 | m/z=505.12(C₃₃H₁₉N₃OS=505.60) |
| N-71 | m/z=505.12(C₃₃H₁₉N₃OS=505.60) | N-72 | m/z=639.24(C₄₅H₂₉N₅=639.76) |
| N-73 | m/z=607.21(C₄₂H₂₉N₃S=607.78) | N-74 | m/z=715.26(C₅₂H₃₃N₃O=715.86) |
| N-75 | m/z=640.23(C₄₅H₂₈N₄O=640.75) | N-76 | m/z=707.20(C₄₉H₂₉N₃OS=707.85) |
| N-77 | m/z=591.23(C₄₂H₂₉N₃O=591.71) | N-78 | m/z=617.28(C₄₅H₃₅N₃=617.80) |
| N-79 | m/z=653.25(C₄₇H₃₁N₃O=653.79) | N-80 | m/z=733.22(C₅₁H₃₁N₃OS=733.89) |
| N-81 | m/z=615.19(C₄₃H₂₅N₃O₂=615.69) | N-82 | m/z=681.19(C₄₇H₂₇N₃OS=681.81) |
| N-83 | m/z=716.29(C₅₂H₃₆N₄=716.89) | N-84 | m/z=690.24(C₄₉H₃₀N₄O=690.81) |
| N-85 | m/z=641.25(C₄₆H₃₁N₃O=641.77) | N-86 | m/z=693.22(C₄₉H₃₁N₃S=693.87) |
| N-87 | m/z=690.24(C₄₉H₃₀N₄O=690.81) | N-88 | m/z=631.17(C₄₃H₂₅N₃OS=631.75) |
| N-89 | m/z=595.14(C₃₉H₂₁N₃O₂S=595.68) | N-90 | m/z=659.24(C₄₅H₂₁D₅N₄O₂=659.76) |
| N-91 | m/z=637.16(C₄₂H₂₇N₃S₂=637.82) | N-92 | m/z=729.25(C₅₁H₃₁N₅O=729.84) |
| N-93 | m/z=578.17(C₃₉H₂₂N₄O₂=578.63) | N-94 | m/z=746.21(C₅₁H₃₀N₄OS=746.89) |
| N-95 | m/z=681.24(C₄₈H₃₁N₃O₂=681.80) | N-96 | m/z=762.19(C₅₁H₃₀N₄S₂=762.95) |
| N-97 | m/z=436.17(C₃₀H₂₀N₄=436.52) | N-98 | m/z=437.16(C₂₉H₁₉N₅=437.51) |
| N-99 | m/z=513.20(C₃₅H₂₃N₅=513.60) | N-100 | m/z=589.23(C₄₁H₂₇N₅=589.70) |
| N-101 | m/z=486.18(C₃₄H₂₂N₄=486.58) | N-102 | m/z=527.17(C₃₅H₂₁N₅O=527.59) |
| N-103 | m/z=589.23(C₄₁H₂₇N₅=589.70) | N-104 | m/z=502.18(C₃₄H₂₂N₄O=502.58) |
| N-105 | m/z=511.20(C₃₇H₂₅N₃=511.63) | N-106 | m/z=563.21(C₃₉H₂₅N₅=563.66) |
| N-107 | m/z=511.20(C₃₇H₂₅N₃=511.63) | N-108 | m/z=589.23(C₄₁H₂₇N₅=589.70) |
| N-109 | m/z=513.20(C₃₅H₂₃N₅=513.60) | N-110 | m/z=462.16(C₃₀H₁₈N₆=462.52) |
| N-111 | m/z=612.21(C₄₂H₂₄N₆=612.70) | N-112 | m/z=499.20(C₃₆H₂₅N₃=499.62) |
| N-113 | m/z=569.17(C₃₇H₂₃N₅S=569.69) | N-114 | m/z=629.22(C₄₃H₂₇N₅O=629.72) |
| N-115 | m/z=629.22(C₄₃H₂₇N₅O=629.72) | N-116 | m/z=563.21(C₃₉H₂₅N₅=563.66) |
| N-117 | m/z=565.2(C₃₇H₂₃N₇=565.64) | N-118 | m/z=630.22(C₄₂H₂₆N₆O=630.71) |
| N-119 | m/z=611.24(C₄₅H₂₉N₃=611.75) | N-120 | m/z=803.29(C₅₉H₃₇N₃O=803.97) |
| N-121 | m/z=563.20(C₄₀H₂₅N₃O=563.66) | N-122 | m/z=549.22(C₄₀H₂₇N₃=549.68) |
| N-123 | m/z=449.15(C₃₁H₁₉N₃O=449.51) | N-124 | m/z=579.18(C₄₀H₂₅N_{3S}=579.72) |
| N-125 | m/z=435.17(C₃₁H₂₁N₃=435.53) | N-126 | m/z=435.17(C₃₁H₂₁N₃=435.53) |
| N-127 | m/z=435.17(C₃₁H₂₁N₃=435.53) | N-128 | m/z=435.17(C₃₁H₂₁N₃=435.53) |
| N-129 | m/z=435.17(C₃₁H₂₁N₃=435.53) | N-130 | m/z=435.17(C₃₁H₂₁N₃=435.53) |
| N-131 | m/z=435.17(C₃₁H₂₁N₃=435.53) | N-132 | m/z=434.18(C₃₂H₂₂N₂=434.54) |
| N-133 | m/z=511.20(C₃₇H₂₅N₃=511.63) | N-134 | m/z=611.24(C₄₅H₂₉N₃=611.75) |
| N-135 | m/z=485.19(C₃₅H₂₃N₃=485.59) | N-136 | m/z=511.2(C₃₇H₂₅N₃=511.63) |
| N-137 | m/z=511.20(C₃₇H₂₅N₃=511.63) | N-138 | m/z=485.19(C₃₅H₂₃N₃=485. 59) |
| N-139 | m/z=434.18(C₃₂H₂₂N₂=434.54) | N-140 | m/z=434.18(C₃₂H₂₂N₂=434.54) |
| N-141 | m/z=511.20(C₃₇H₂₅N₃=511.63) | N-142 | m/z=561.22(C₄₁H₂₇N₃=561.69) |
| N-143 | m/z=587.24(C₄₃H₂₉N₃=587.73) | N-144 | m/z=511.20(C₃₇H₂₅N₃=511.63) |
| N-145 | m/z=511.20(C₃₇H₂₅N₃=511.63) | N-146 | m/z=511.20(C₃₇H₂₅N₃=511.63) |
| N-147 | m/z=511.20(C₃₇H₂₅N₃=511.63) | N-148 | m/z=587.24(C₄₃H₂₉N₃=587.73) |
| N-149 | m/z=435.17(C₃₁H₂₁N₃=435.53) | N-150 | m/z=435.17(C₃₁H₂₁N₃=435.53) |
| N-151 | m/z=435.17(C₃₁H₂₁N₃=435.53) | N-152 | m/z=435.17(C₃₁H₂₁N₃=435.53) |
| N-153 | m/z=435.17(C₃₁H₂₁N₃=435.53) | N-154 | m/z=435.17(C₃₁H₂₁N₃=435.53) |
| N-155 | m/z=435.17(C₃₁H₂₁N₃=435.53) | N-156 | m/z=434.18(C₃₂H₂₂N₂=434.54) |
| N-157 | m/z=485.19(C₃₅H₂₃N₃=485.59) | N-158 | m/z=511.2(C₃₇H₂₅N₃=511.63) |
| N-159 | m/z=511.20(C₃₇H₂₅N₃=511.63) | N-160 | m/z=511.20(C₃₇H₂₅N₃=511.63) |
| N-161 | m/z=485.19(C₃₅H₂₃N₃=485.59) | N-162 | m/z=511.20(C₃₇H₂₅N₃=511.63) |
| N-163 | m/z=485.19(C₃₅H₂₃N₃=485.59) | N-164 | m/z=611.24(C₄₅H₂₉N₃=611.75) |
| N-165 | m/z=511.20(C₃₇H₂₅N₃=511.63) | N-166 | m/z=511.20(C₃₇H₂₅N₃=511.63) |
| N-167 | m/z=587.24(C₄₃H₂₉N₃=587.73) | N-168 | m/z=587.24(C₄₃H₂₉N₃=587.73) |
| N-169 | m/z=587.24(C₄₃H₂₉N₃=587.73) | N-170 | m/z=561.22(C₄₁H₂₇N₃=561.69) |
| N-171 | m/z=511.20(C₃₇H₂₅N₃=511.63) | N-172 | m/z=587.24(C₄₃H₂₉N₃=587.73) |
| N-173 | m/z=485.19(C₃₅H₂₃N₃=485.59) | N-174 | m/z=485.19(C₃₅H₂₃N₃=485.59) |
| N-175 | m/z=485.19(C₃₅H₂₃N₃=485.59) | N-176 | m/z=485.19(C₃₅H₂₃N₃=485.59) |
| N-177 | m/z=485.19(C₃₅H₂₃N₃=485.59) | N-178 | m/z=535.20(C₃₉H₂₅N₃=535.65) |
| N-179 | m/z=485.19(C₃₅H₂₃N₃=485.59) | N-180 | m/z=485.19(C₃₅H₂₃N₃=485.59) |
| N-181 | m/z=561.22(C₄₁H₂₇N₃=561.69) | N-182 | m/z=561.22(C₄₁H₂₇N₃=561.69) |
| N-183 | m/z=561.22(C₄₁H₂₇N₃=561.69) | N-184 | m/z=637.25(C₄₇H₃₁N₃=637.79) |
| N-185 | m/z=561.22(C₄₁H₂₇N₃=561.69) | N-186 | m/z=561.22(C₄₁H₂₇N₃=561.69) |
| N-187 | m/z=637.25(C₄₇H₃₁N₃=637.79) | N-188 | m/z=637.25(C₄₇H₃₁N₃=637.79) |
| N-189 | m/z=637.25(C₄₇H₃₁N₃=637.79) | N-190 | m/z=485.19(C₃₅H₂₃N₃=485.59) |
| N-191 | m/z=485.19(C₃₅H₂₃N₃=485.59) | N-192 | m/z=611.24(C₄₅H₂₉N₃=611.75) |
| N-193 | m/z=485.19(C₃₅H₂₃N₃=485.59) | N-194 | m/z=485.19(C₃₅H₂₃N₃=485.59) |
| N-195 | m/z=611.24(C₄₅H₂₉N₃=611.75) | N-196 | m/z=485.19(C₃₅H₂₃N₃=485.59) |
| N-197 | m/z=485.19(C₃₅H₂₃N₃=485.59) | N-198 | m/z=561.22(C₄₁H₂₇N₃=561.69) |
| N-199 | m/z=485.19(C₃₅H₂₃N₃=485.59) | N-200 | m/z=485.19(C₃₅H₂₃N₃=485.59) |
| N-201 | m/z=611.24(C₄₅H₂₉N₃=611.75) | N-202 | m/z=611.24(C₄₅H₂₉N₃=611.75) |
| N-203 | m/z=485.19(C₃₅H₂₃N₃=485.59) | N-204 | m/z=485.19(C₃₅H₂₃N₃=485.59) |
| N-205 | m/z=485.19(C₃₅H₂₃N₃=485.59) | N-206 | m/z=485.19(C₃₅H₂₃N₃=485.59) |
| N-207 | m/z=535.20(C₃₉H₂₅N₃=535.65) | N-208 | m/z=535.2(C₃₉H₂₅N₃=535.65) |
| N-209 | m/z=585.22(C₄₃H₂₇N₃=585.71) | N-210 | m/z=535.2(C₃₉H₂₅N₃=535.65) |
| N-211 | m/z=585.22(C₄₃H₂₇N₃=585.71) | N-212 | m/z=585.22(C₄₃H₂₇N₃=585.71) |
| N-213 | m/z=611.24(C₄₅H₂₉N₃=611.75) | N-214 | m/z=611.24(C₄₅H₂₉N₃=611.75) |
| N-215 | m/z=585.22(C₄₃H₂₇N₃=585.71) | N-216 | m/z=611.24(C₄₅H₂₉N₃=611.75) |
| N-217 | m/z=687.27(C₅₁H₃₃N₃=687.85) | N-218 | m/z=611.24(C₄₅H₂₉N₃=611.75) |
| N-219 | m/z=511.20(C₃₇H₂₅N₃=511.63) | N-220 | m/z=611.24(C₄₅H₂₉N₃=611.75) |
| N-221 | m/z=561.22(C₄₁H₂₇N₃=561.69) | N-222 | m/z=587.24(C₄₃H₂₉N₃=587.73) |
| N-223 | m/z=663.27(C₄₉H₃₃N₃=663.82) | N-224 | m/z=713.28(C₅₃H₃₅N₃=713.88) |
| N-225 | m/z=575.20(C₄₁H₂₅N₃O=575.67) | N-226 | m/z=601.22(C₄₃H₂₇N₃O=601.71) |
| N-227 | m/z=700.26(C₅₁H₃₂N₄=700.85) | N-228 | m/z=701.25(C₅₁H₃₁N₃O=701.83) |
| N-229 | m/z=667.21(C₄₇H₂₉N₃S=667.83) | N-230 | m/z=541.16(C₃₇H₂₃N₃S=541.67) |
| N-231 | m/z=612.23(C₄₄H₂₈N₄=612.74) | N-232 | m/z=562.22(C₄₀H₂₆N₄=562.68) |
| N-233 | m/z=689.26(C₄₉H₃₁N₅=689.82) | N-234 | m/z=639.24(C₄₅H₂₉N₅=639.76) |
| N-235 | m/z=701.25(C₅₁H₃₁N₃O=701.83) | N-236 | m/z=631.17(C₄₃H₂₅N₃OS=631.75) |
| N-237 | m/z=625.22(C₄₅H₂₇N₃O=625.73) | N-238 | m/z=591.18(C₄₁H₂₅N₃S=591.73) |
| N-239 | m/z=687.27(C₅₁H₃₃N₃=687.85) | N-240 | m/z=701.25(C₅₁H₃₁N₃O=701.83) |
| N-241 | m/z=619.30(C₄₅H₃₇N₃=619.81) | N-242 | m/z=601.25(C₄₄H₃₁N₃=601.75) |
| N-243 | m/z=667.23(C₄₇H₂₉N₃O₂=667.77) | N-244 | m/z=540.24(C₃₉H₂₀D₅N₃=540.68) |
| N-245 | m/z=521.17(C₃₅H₂₁F₂N₃=521.57) | N-246 | m/z=510.18(C₃₆H₂₂N₄=510.60) |
| N-247 | m/z=652.23(C₄₆H₂₈N₄O=652.76) | N-248 | m/z=527.24(C₃₈H₂₉N₃=527.67) |
| N-249 | m/z=535.20(C₃₉H₂₅N₃=535.65) | N-250 | m/z=535.20(C₃₉H₂₅N₃=535.65) |
| N-251 | m/z=535.20(C₃₉H₂₅N₃=535.65) | N-252 | m/z=535.20(C₃₉H₂₅N₃=535.65) |
| N-253 | m/z=587. 24(C₄₃H₂₉N₃=587.73) | N-254 | m/z=612.23(C₄₄H₂₈N₄=612.74) |
| N-255 | m/z=561.22(C₄₁H₂₇N₃=561.69) | N-256 | m/z=687.27(C₅₁H₃₃N₃=687.85) |
| N-257 | m/z=663.27(C₄₉H₃₃N₃=663.82) | N-258 | m/z=601.22(C₄₃H₂₇N₃O=601.71) |
| N-259 | m/z=617.19(C₄₃H₂₇N₃S=617.77) | N-260 | m/z=752.29(C₅₅H₃₆N₄=752.92) |
| N-261 | m/z=651.23(C₄₇H₂₉N₃O=651.77) | N-262 | m/z=677.25(C₄₉H₃₁N₃O=677.81) |
| N-263 | m/z=541.16(C₃₇H₂₃N₃S=541.67) | N-264 | m/z=750.28(C₅₅H₃₄N₄=750.91) |
| N-265 | m/z=707.24(C₅₀H₃₃N₃S=707.90) | N-266 | m/z=651.23(C₄₇H₂₉N₃O=651.77) |
| N-267 | m/z=617.19(C₄₃H₂₇N₃S=617.77) | N-268 | m/z=667.21(C₄₇H₂₉N₃S=667.83) |
| N-269 | m/z=631.17(C₄₃H₂₅N₃OS=631.75) | N-270 | m/z=767.26(C₅₅H₃₃N₃O₂=767.89) |
| N-271 | m/z=647.15(C₄₃H₂₅N₃S₂=647.81) | N-272 | m/z=690.24(C₄₉H₃₀N₄O=690.81) |
| N-273 | m/z=575.2(C₄₁H₂₅N₃O=575.67) | N-274 | m/z=614.21(C₄₃H₂₆N₄O=614.71) |
| N-275 | m/z=575.2(C₄₁H₂₅N₃O=575.67) | N-276 | m/z=549.18(C₃₉H₂₃N₃O=549.63) |

Meanwhile, exemplary synthetic examples or references of the present invention represented by Formula 1 and Formula 2 have been described, but these are all based on the Buchwald-Hartwig cross coupling reaction, Miyaura boration reaction, Suzuki cross-coupling reaction, Intramolecular acid-induced cyclization reaction (J. mater. Chem.1999, 9, 2095.), Pd(II)-catalyzed oxidative cyclization reaction (Org. Lett.2011, 13, 5504), and PPh₃-mediated reductive cyclization reaction (J. Org. Chem. 2005, 70, 5014.), and it will be easily understood by those skilled in the art that the reaction proceeds even when other substituents defined in Formula 1 or Formula 2 are bonded in addition to the substituents specified in the specific synthesis examples.

### Organic Electroluminescent Devices

### [Example 1] Red Organic Electroluminescent Device (Phosphorescent host)

An organic electroluminescent device was manufactured using a compound obtained through synthesis as a light-emitting host material of an emitting layer according to a conventional method. First, N¹-(naphthalen-2-yl)-N⁴,N⁴-bis(4-(naphthalen-2-yl(phenyl)amino)phenyl)-N¹-phenylbenzene-1,4-diamine (hereinafter, 2-TNATA) film was vacuum-deposited on an ITO layer (anode) formed on a glass substrate to form a hole injection layer with a thickness of 50 nm, and then 4,4-bis[N-(1-naphthyl)-N-phenylamino]biphenyl (hereinafter, NPB) was vacuum-deposited on the hole injection layer to a thickness of 80 nm as a hole transport compound to form a hole transport layer. After forming the hole transport layer, the compound P-2 of the present invention represented by Formula 1 and the compound N-128 of the present invention represented by Formula 2 were used as hosts at a weight ratio of (5:5) on the upper portion of the hole transport layer, and (piq)₂Ir(acac) was doped as a dopant material at a weight ratio of 95:5 to deposit an emitting layer with a thickness of 40 nm. Next, (1,1'-bisphenyl)-4-oleato)bis(2-methyl-8-quinolineoleato)aluminum (hereinafter, BAlq) was vacuum-deposited to a thickness of 10 nm as a hole-blocking layer, and bis(10-hydroxybenzo[h]quinolinato)beryllium (hereinafter, BeBq₂) was deposited to a thickness of 20 nm as an electron transport layer. Afterwards, LiF, a halogenated alkali metal, was deposited as an electron injection layer with a thickness of 0.2 nm, and Al was then deposited with a thickness of 150 nm and used as a cathode, thereby manufacturing an organic light emitting device.

### [Example 2] to [Example 34]

An organic electroluminescent device was manufactured in the same manner as in Example 1, except that the compounds of the present invention described in Table 5 were used instead of the compound P-2 and N-128 of the present invention as a phosphorescent host material.

### [Comparative Example 1] to [Comparative Example 5]

An organic electroluminescent device was manufactured in the same manner as Example 1, except that Comparative Compounds A to E were used instead of Compound P-2 of the present invention as a phosphorescent host material.

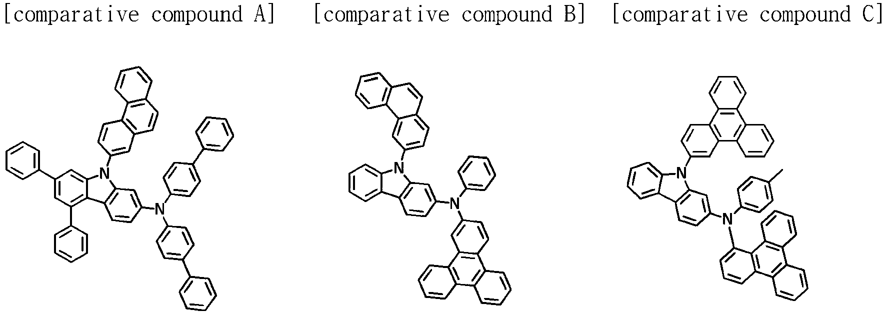

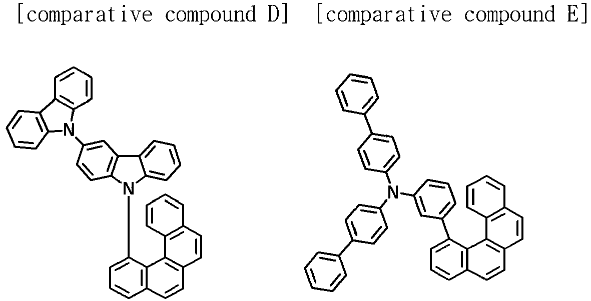

The electroluminescence (EL) characteristics were measured using a PR-650 from Photoresearch by applying a forward bias DC voltage to the organic electroluminescent devices manufactured in this manner, and the T95 lifespan was measured using a lifespan measuring device manufactured by Maxscience at a standard luminance of 2,500 cd/m². Table 5 shows the results of the device fabrication and evaluation.

The measuring apparatus can evaluate the performance of new materials compared to comparative compounds under identical conditions, without being affected by possible daily fluctuations in deposition rate, vacuum quality or other parameters.

During the evaluation, one batch contains 4 identically prepared OLEDs including a comparative compound, and the performance of a total of 12 OLEDs is evaluated in 3 batches, so the value of the experimental results obtained in this way indicates statistical significance.

**[Table 5]**

| | First compound | Second compound | Driving Voltage (V) | Current Density (mA/cm²) | Efficiency (cd/A) | T(95) |
|---|---|---|---|---|---|---|
| comparative example 1 | comparative compound A | compound(N-128) | 5.2 | 11.6 | 21.6 | 93.1 |
| comparative example 2 | comparative compound B | compound(N-128) | 5.3 | 11.7 | 21.3 | 91.6 |
| comparative example 3 | comparative compound C | compound(N-128) | 5.3 | 12.0 | 20.8 | 90.3 |
| comparative example 4 | comparative compound D | compound(N-128) | 5.4 | 12.8 | 19.6 | 95.7 |
| comparative example 5 | comparative compound E | compound(N-128) | 5.1 | 11.2 | 22.4 | 95.3 |
| Example 1 | compound(P-2) | compound(N-128) | 4.7 | 8.7 | 28.7 | 105.6 |
| Example 2 | compound(P-11) | compound(N-128) | 4.8 | 8.8 | 28.3 | 108.6 |
| Example 3 | compound (P-27) | compound(N-128) | 4.7 | 8.8 | 28.5 | 111.3 |
| Example 4 | compound(P-37) | compound(N-128) | 4.7 | 8.5 | 29.5 | 112.1 |
| Example 5 | compound(P-40) | compound(N-128) | 4.6 | 8.6 | 29.2 | 109.6 |
| Example 6 | compound(P-41) | compound(N-128) | 4.6 | 8.7 | 28.7 | 111.6 |
| Example 7 | compound(P-43) | compound(N-128) | 4.7 | 8.7 | 28.8 | 112.7 |
| example8 | compound(P-49) | compound(N-128) | 4.6 | 8.8 | 28.3 | 110.3 |
| Example 9 | compound(P-50) | compound(N-128) | 4.7 | 9.4 | 26.7 | 105.4 |
| Example 10 | compound(P-66) | compound(N-128) | 4.7 | 9.2 | 27.1 | 104.4 |
| Example 11 | compound (P-75) | compound(N-128) | 4.8 | 9.2 | 27.0 | 109.9 |
| Example 12 | compound(P-90) | compound(N-128) | 4.7 | 8.8 | 28.3 | 106.2 |
| Example 13 | compound(P-101) | compound(N-128) | 4.8 | 9.0 | 27.9 | 104.9 |
| Example 14 | compound(P-102) | compound(N-128) | 5.0 | 9.3 | 26.9 | 104.9 |
| Example 15 | compound(P-107) | compound(N-128) | 4.8 | 9.1 | 27.6 | 107.8 |
| Example 16 | compound(P-109) | compound(N-128) | 4.8 | 8.8 | 28.5 | 103.9 |
| Example 17 | compound(P-117) | compound(N-128) | 4.8 | 9.0 | 27.7 | 102.3 |
| Example 18 | compound(P-2) | compound (N-274) | 4.8 | 9.0 | 27.7 | 108.7 |
| Example 19 | compound(P-11) | compound (N-274) | 4.8 | 9.1 | 27.4 | 111.8 |
| Example 20 | compound (P-27) | compound (N-274) | 4.8 | 9.1 | 27.6 | 114.6 |
| Example 21 | compound(P-37) | compound(N-274) | 4.7 | 8.7 | 28.6 | 115.5 |
| Example 22 | compound(P-40) | compound (N-274) | 4.7 | 8.8 | 28.3 | 112.9 |
| Example 23 | compound(P-41) | compound (N-274) | 4.6 | 9.0 | 27.8 | 114.9 |
| Example 24 | compound(P-43) | compound (N-274) | 4.7 | 9.0 | 27.9 | 116.1 |
| Example 25 | compound(P-49) | compound (N-274) | 4.7 | 9.1 | 27.4 | 113.6 |
| Example 26 | compound(P-50) | compound (N-274) | 4.8 | 9.7 | 25.8 | 108.5 |
| Example 27 | compound(P-66) | compound (N-274) | 4.7 | 9.5 | 26.2 | 107.6 |
| Example 28 | compound(P-75) | compound (N-274) | 4.8 | 9.5 | 26.2 | 113.2 |
| Example 29 | compound(P-90) | compound (N-274) | 4.8 | 9.1 | 27.4 | 109.4 |
| Example 30 | compound(P-101) | compound (N-274) | 4.8 | 9.3 | 27.0 | 108.0 |
| Example 31 | compound(P-102) | compound (N-274) | 5.0 | 9.6 | 26.1 | 108.0 |
| Example 32 | compound(P-107) | compound (N-274) | 4.9 | 9.4 | 26.7 | 111.1 |
| Example 33 | compound(P-109) | compound (N-274) | 4.9 | 9.1 | 27.6 | 107.0 |
| Example 34 | compound(P-117) | compound (N-274) | 4.9 | 9.3 | 26.9 | 105.4 |

As can be seen in Table 5, when a red organic electroluminescent device is manufactured using the material for an organic electroluminescent device of the present invention as a phosphorescent host material, the compound of the present invention exhibits remarkable characteristics in device performance compared to the case where comparative compounds A to E were used, and particularly, exhibits excellent characteristics in terms of efficiency.

As can be seen above, when forming a host for an emitting layer by mixing multiple compounds, it can be confirmed that there is a significant difference in characteristics depending on the type of the first and second compounds. Similarly, there are differences in driving voltage, efficiency, and lifespan depending on the type of the second compound.

Comparing the compounds of the present invention with comparative compounds A to C, the present invention differs in that chrysene is substituted as a substituent of carbazole, whereas comparative compounds A to C are substituted with phenanthrene or triphenylene. This can be confirmed through Table 6.

Table 6 shows the energy levels of comparative compounds A to C and compound P-40 of the present invention measured using the DFT Method (B3LYP/6-31g(D)) of the Gaussian program.

**[Table 6]**

| | **comparative compound A** | **comparative compound B** | **comparative compound C** | **P-40** |
|---|---|---|---|---|
| **T1** | 2.58 | 2.65 | 2.65 | 2.46 |

As can be seen from the results in Table 6, it can be confirmed that the T1 of compound P-40 of the present invention is lower than that of comparative compounds A to C.

This means that the red dopant has the lowest T1 among phosphorescent R/G/B, and the lowest T1 energy gap with the red dopant. That is, it simultaneously plays a role in transferring excitons from the host P-40 to the dopant and blocking excitons moving from the dopant to the hole transport layer, resulting in a very significant difference in efficiency.

Additionally, when the comparative compound D and the compound of the present invention are compared, the comparative compound D has a structure in which 2 carbazoles are bonded, whereas the compound of the present invention has an amino group bonded to the 2nd position of the carbazole, which results in a large difference in the physical properties or morphology of the compounds (especially the HOMO level), and this results in a difference in the overall performance of the compounds.

Finally, when comparing the comparative compound E with the compound of the present invention, there is a difference in that the comparative compound E has a composition in which carbazole is not substituted, but the compound of the present invention includes carbazole.

Table 7 describes the calculated Reorganization Energy values for Comparative Compounds E and P-40.

The RE ratio values listed in Table 7 represent the RE_{hole/}RE_{elec} values calculated after calculating REₕₒₗₑ and RE_{elec}.

**[Table 7]**

| | **comparative compound E** | **P-40** |
|---|---|---|
| **RE ratio** | 0.545 | 1.079 |

When Table 7 is explained in detail, it can be seen that the RE ratio value of the compound P-40 of the present invention is greater than that of the comparative compound E. This means a correlation between hole mobility and electron mobility, and that the higher the RE_{elec} value, the lower the RE ratio.

That is, as RE_{elec} increases, electron mobility decreases, which results in an increase in the light emmitting region. Therefore, the efficiency decreases due to the wide luminescent region. Whereas, as RE ratio increases, electron mobility increases, which results in a narrow luminescent region, which results in an increase in efficiency. Therefore, it appears that the compound of the present invention has higher efficiency than the comparative compound E.

Therefore, it appears that the efficiency is dramatically increased due to the synergistic effect that occurs by substituting a specific substituent as in the present invention.

Although exemplary embodiments of the present invention have been described for illustrative purposes, those skilled in the art will appreciate that various modifications, additions and substitutions are possible, without departing from the scope and spirit of the invention as disclosed in the accompanying claims. Therefore, the embodiment disclosed in the present invention is intended to illustrate the scope of the technical idea of the present invention, and the scope of the present invention is not limited by the embodiment. The scope of the present invention shall be construed on the basis of the accompanying claims, and it shall be construed that all of the technical ideas included within the scope equivalent to the claims belong to the present invention.

### [Industrial Applicability]

According to the present invention, organic devices with excellent device characteristics such as high brightness, high luminescence, and long lifespan can be manufactured, thus demonstrating industrial applicability.

## Claims

1. A compound represented by Formula 1: 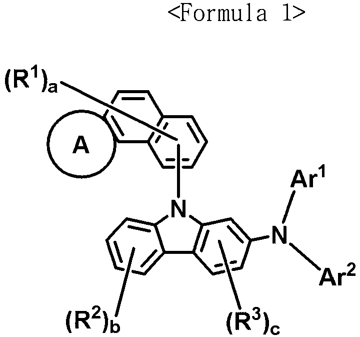 wherein:
Ring A is a C₁₀ aryl group,
R¹, R² and R³ are independently the same or different from each other, and are independently selected from the group consisting of hydrogen; deuterium; a C₆-C₆₀ aryl group; a fluorenyl group; a C₂-C₆₀ heterocyclic group including at least one heteroatom of O, N, S, Si or P; a C₁-C₅₀ alkyl group; a C₂-C₂₀ alkenyl group; a C₂-C₂₀ alkynyl group; a C₁-C₃₀ alkoxyl group; and a C₆-C₃₀ aryloxy group; or a plurality of adjacent R² and R³ are may be bonded to each other to form an aromatic ring,
Ar¹ and Ar² are independently selected from the group consisting of a C₆-C₆₀ aryl group; a fluorenyl group; a C₂-C₆₀ heterocyclic group including at least one heteroatom of O, N, S, Si or P; and a fused ring group of a C₃-C₆₀ aliphatic ring and a C₆-C₆₀ aromatic ring;
a is an integer of 0 to 11, b is an integer of 0 to 4, c is an integer of 0 to 3,
wherein, the aryl group and heterocyclic group may be further substituted with one or more substituents selected from the group consisting of deuterium, a C₁-C₂₀ alkyl group; a C₆-C₂₀ aryl group; a C₆-C₂₀ aryl group substituted with deuterium; and a C₂-C₂₀ heterocyclic group.

2. The compound according to claim 1, wherein Formula 1 is represented by any one of the following Formulas 3 to 10: 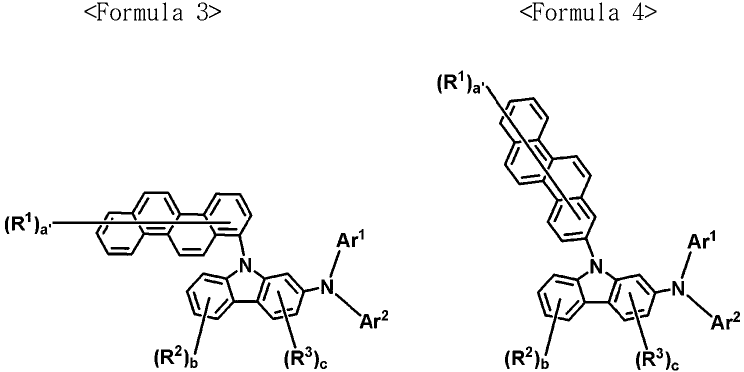 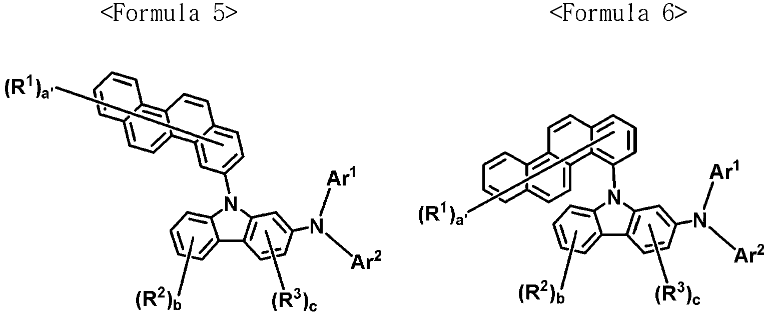 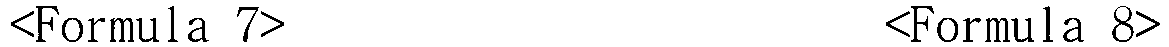 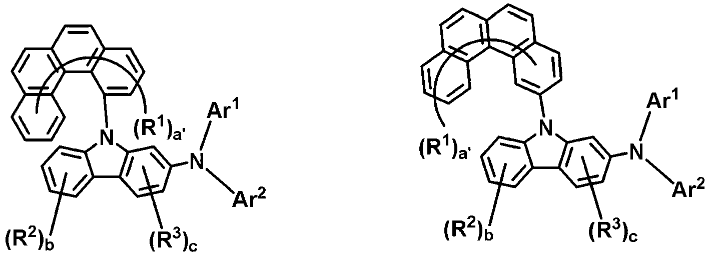 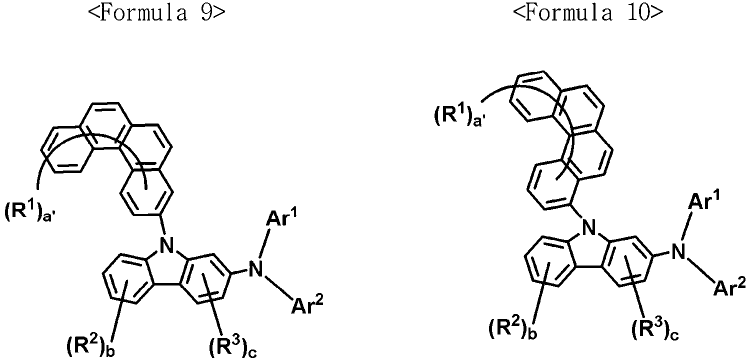 wherein:
R¹, R², R³, Ar¹, Ar², b and c are the same as defined in claim 1,
a' is an integer of 0 to 11.

3. The compound according to claim 1, wherein Ar¹ and Ar² are represented by any one of the following Formulas a-1 to a-15: 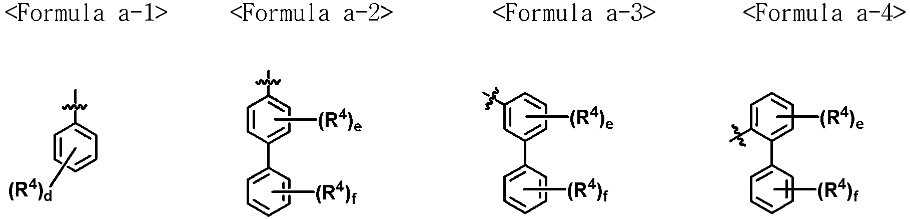 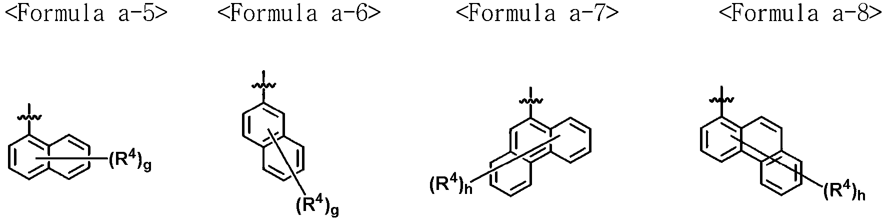 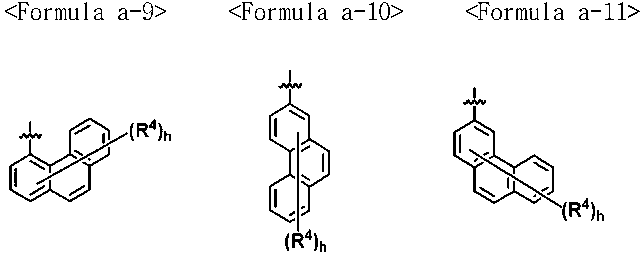 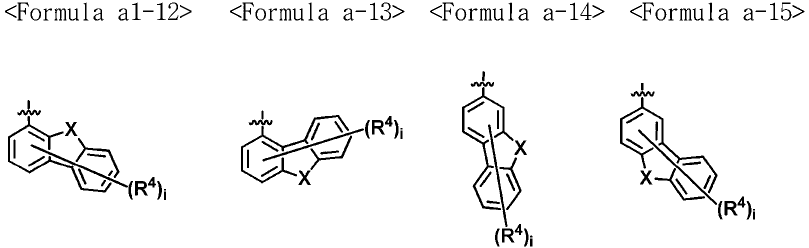 wherein:
R⁴ is independently the same or different from each other and is independently selected from the group consisting of deuterium; a C₆-C₆₀ aryl group; and a C₆-C₂₀ aryl group substituted with deuterium; or a plurality of adjacent groups thereof may be bonded to each other to form a ring,
d and f are each independently an integer of 0 to 5, e is an integer of 0 to 4, g and i are each independently an integer of 0 to 7, h is an integer of 0 to 9,
X is O, S, NR' or CR'R",
R' and R" are independently selected from the group consisting of hydrogen; deuterium; a C₁-C₂₀ alkyl group; a C₆-C₆₀ aryl group; and a C₆-C₂₀ aryl group substituted with deuterium; a C₂-C₆₀ heterocyclic group including at least one heteroatom of O, N, S, Si or P; or R' and R" may be bonded to each other to form a ring, 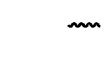 indicates the position to be bonded.

4. The compound according to claim 1, wherein the compound of Formula 1 may be any one of the following compounds P-1 to P-128:

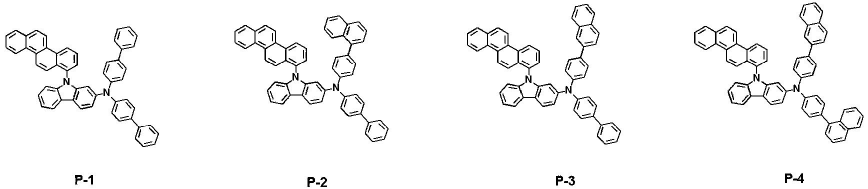

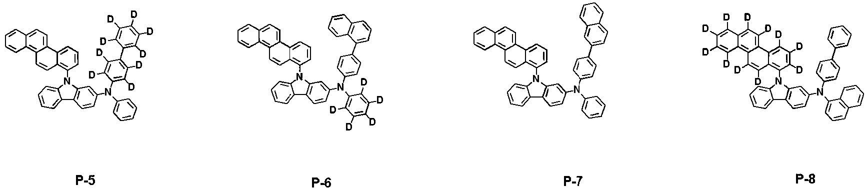

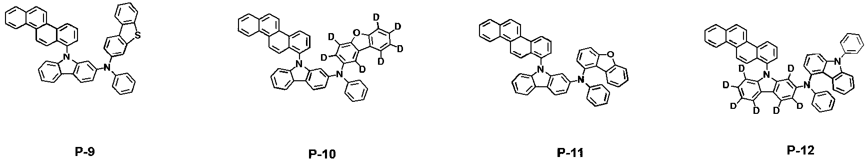

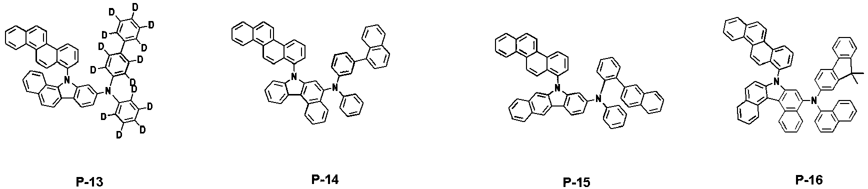

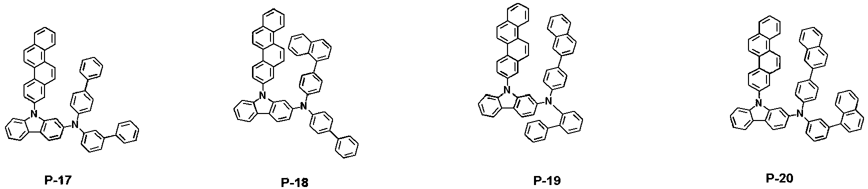

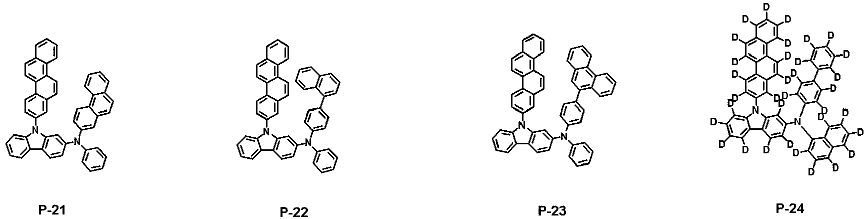

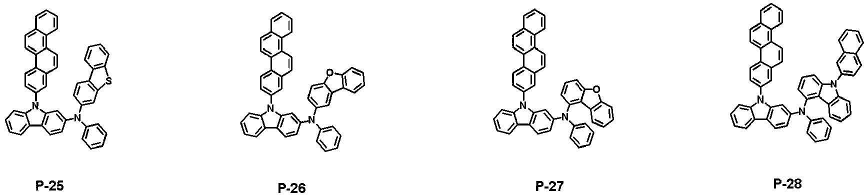

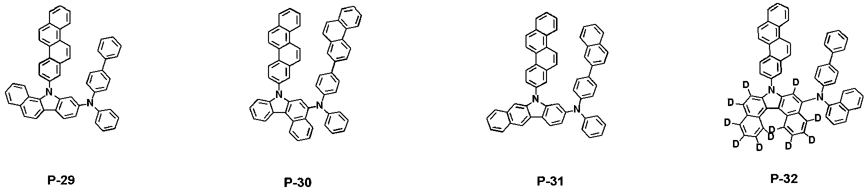

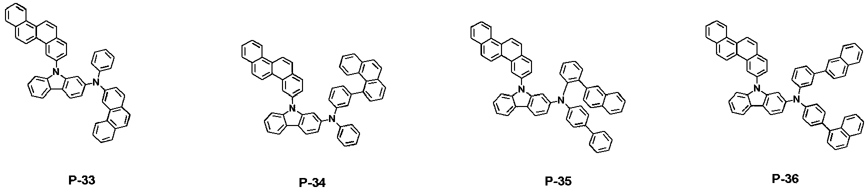

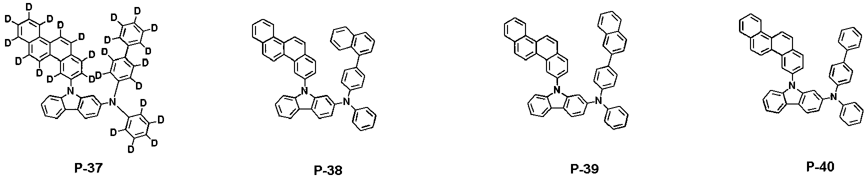

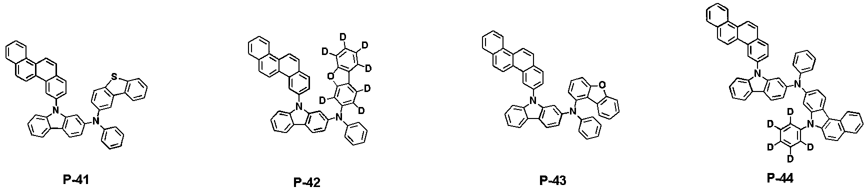

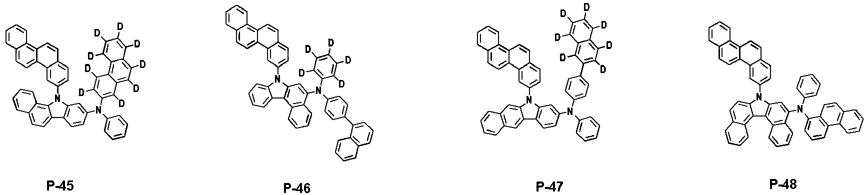

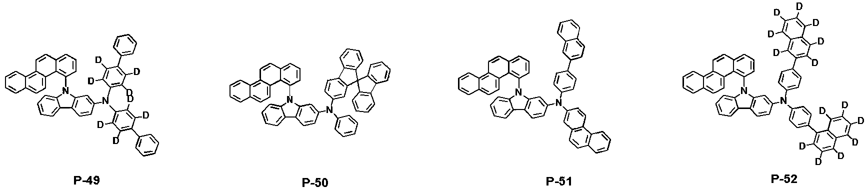

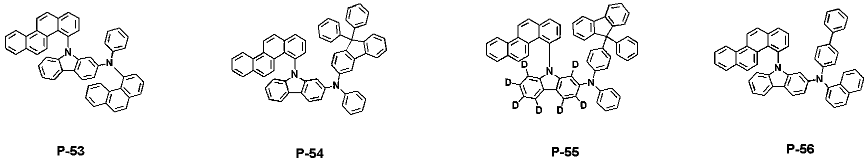

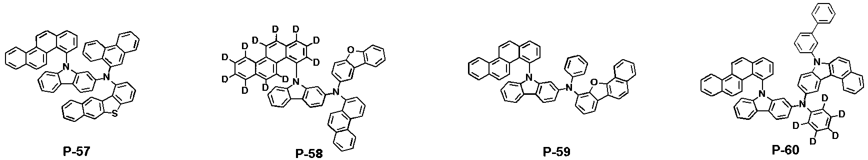

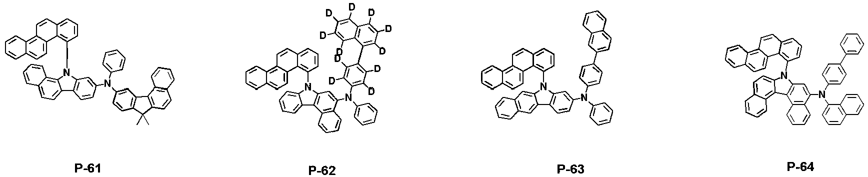

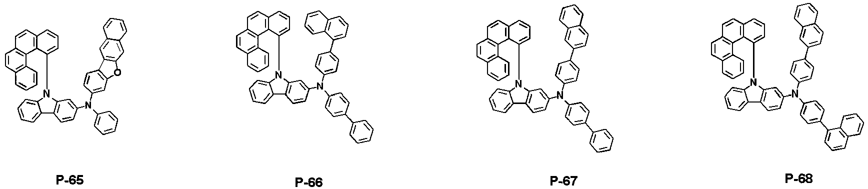

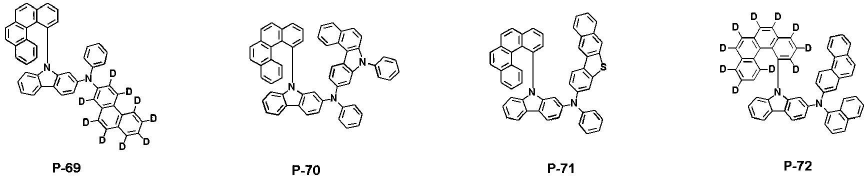

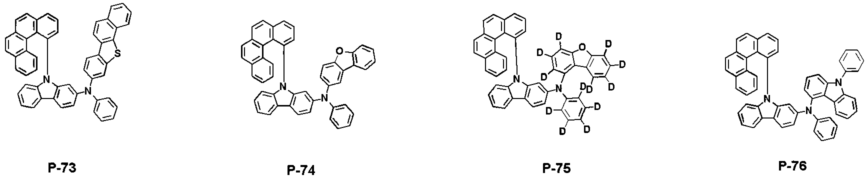

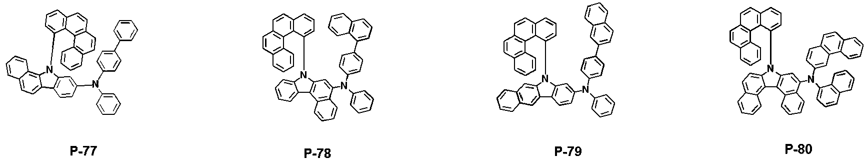

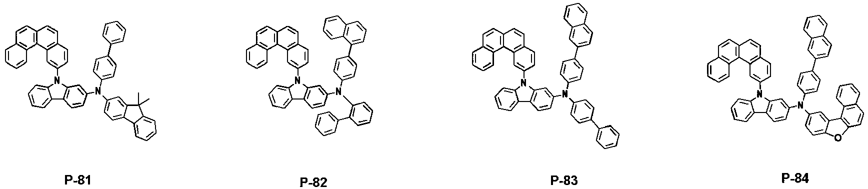

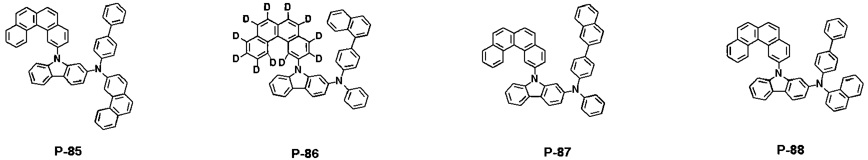

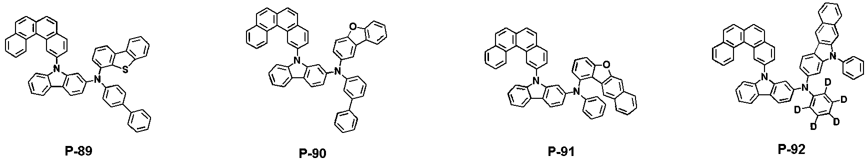

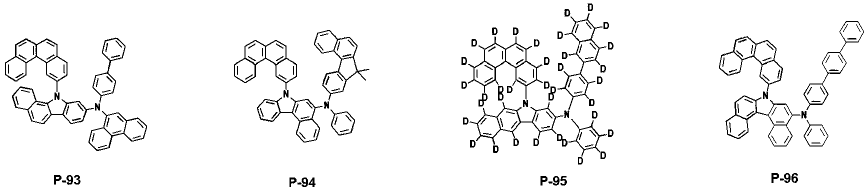

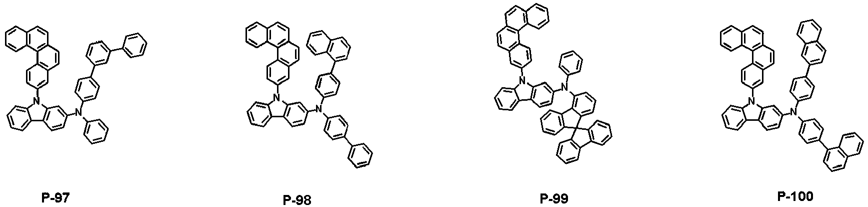

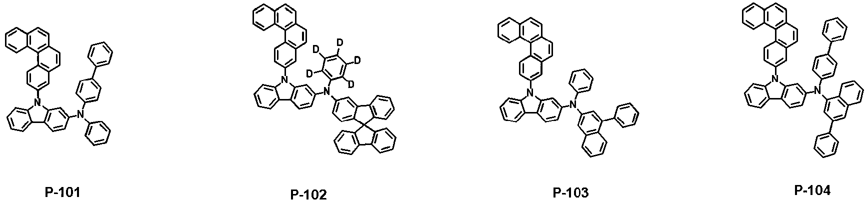

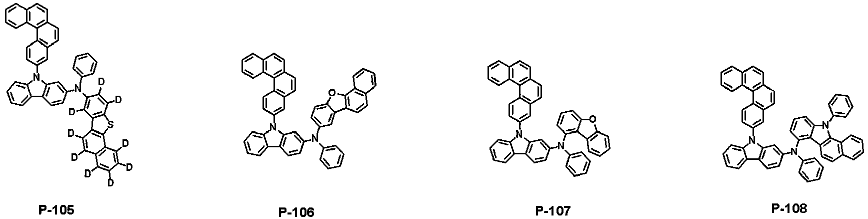

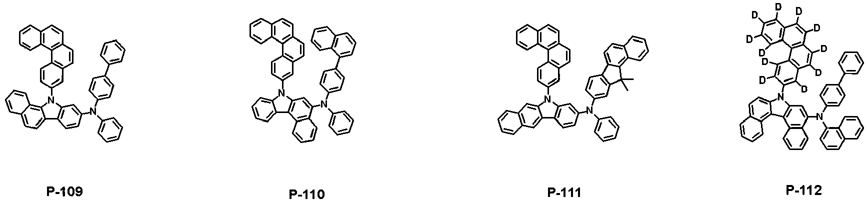

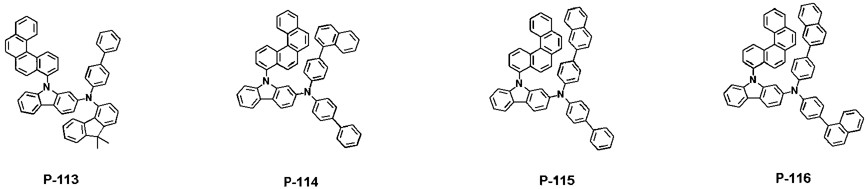

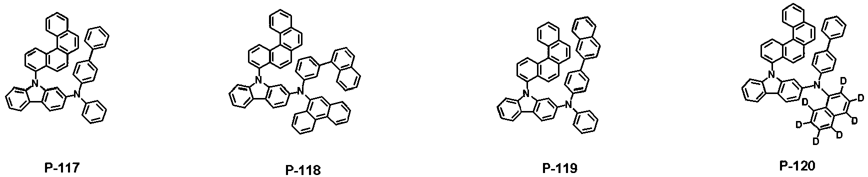

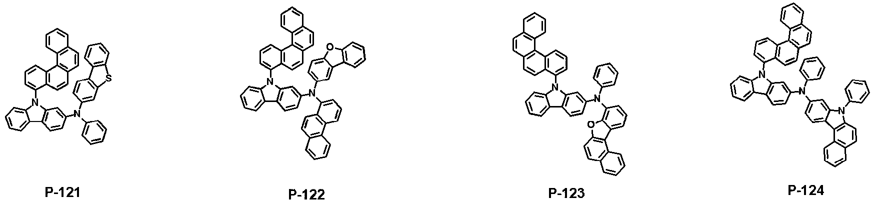

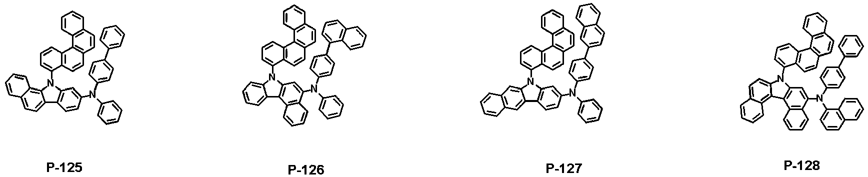

5. A composition for a phosphorescent light-emitting layer of an organic electronic element comprising a mixture of a compound represented by Formula 1 according to claim 1 and a compound represented by Formula 2: 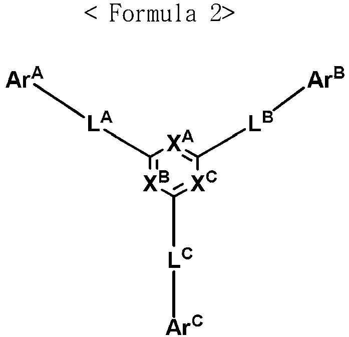 wherein:
X^{A}, X^{B} and X^{C} are each independently CR^{a} or N,
However, at least one of X^{A}, X^{B} and X^{C} is N,
Ar^{A}, Ar^{B} and Ar^{C} are independently selected from the group consisting of a C₆-C₆₀ aryl group; a fluorenyl group; a C₂-C₆₀ heterocyclic group including at least one heteroatom of O, N, S, Si or P; a C₃-C₆₀ cycloalkyl group; a fused ring group of a C₃-C₆₀ aliphatic ring and a C₆-C₆₀ aromatic ring;
L^{A}, L^{B} and L^{C} are independently selected from the group consisting of a single bond; a C₆-C₆₀ arylene group; a fluorenylene group; a C₂-C₆₀ heterocyclic group including at least one heteroatom of O, N, S, Si or P; and a fused ring group of a C₃-C₆₀ aliphatic ring and a C₆-C₆₀ aromatic ring;
The R^{a} is hydrogen; or deuterium;
wherein the aryl group, arylene group, heterocyclic group, fluorenyl group, fluorenylene group, cycloalkyl group and fused ring group may be substituted with one or more substituents selected from the group consisting of deuterium; halogen; silane group; siloxane group; boron group; germanium group; a cyano group; a nitro group; a C₁-C₂₀ alkylthio group; a C₁-C₂₀ alkoxyl group; ; a C₆-C₂₀ aryloxy group a C₁-C₂₀ alkyl group; a C₂-C₂₀ alkenyl group; a C₂-C₂₀ alkynyl group; a C₆-C₂₀ aryl group; a C₆-C₂₀ aryl group substituted with deuterium; a fluorenyl group; a C₂-C₂₀ heterocyclic group; a C₃-C₂₀ aliphatic ring; a C₇-C₂₀ arylalkyl group; a C₈-C₂₀ arylalkenyl group; and a C₇-C₂₀ alkylaryl group; and
the hydrogen of these substituents may be further substituted with one or more deuterium or a C₆-C₂₀ aryl group; and the substituents may be bonded to each other to form a saturated or unsaturated ring, wherein the term 'ring' means a C₃-C₆₀ aliphatic ring or a C₆-C₆₀ aromatic ring or a C₂-C₆₀ heterocyclic group or a fused ring formed by the combination thereof.

6. The composition for a phosphorescent light-emitting layer of an organic electronic element according to claim 5, wherein at least one of the Ar^{A}, Ar^{B} and Ar^{C} is represented by any one of Formulae Ar-a to Ar-d: 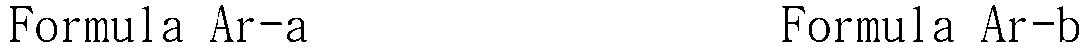 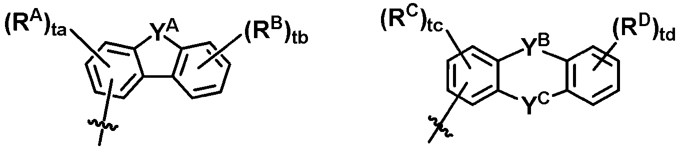 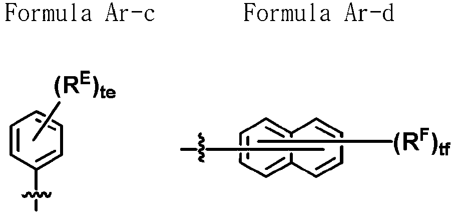 wherein:
Y^{A}, Y^{B} and Y^{C} are each independently 0, S, NR^{1A} or CR^{1B}R^{1C},
R^{A}, R^{B}, R^{C,} R^{D}, R^{E}, R^{F}, R^{1A}, R^{1B} and R^{1C} are independently the same or different from each other and are independently selected from the group consisting of hydrogen; deuterium; halogen; a cyano group; a C₆-C₂₀ aryl group; a fluorenyl group; a C₂-C₂₀ heterocyclic group including at least one heteroatom of O, N, S, Si or P; a fused ring group of a C₃-C₂₀ aliphatic ring and a C₆-C₂₀ aromatic ring; a C₁-C₂₀ alkyl group; a C₂-C₂₀ alkenyl group; a C₂-C₂₀ alkynyl group; a C₁-C₂₀ alkoxyl group; and a C₆-C₂₀ aryloxy group; or a plurality of adjacent groups thereof may be bonded to each other to form a ring,
ta and tc are each independently an integer of 0 to 3, tb and td are each independently an integer of 0 to 4, te is an integer of 0 to 5, tf is an integer of 0 to 7, 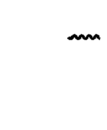 indicates the position to be bonded.

7. The composition for a phosphorescent light-emitting layer of an organic electronic element according to claim 5, wherein the compound of Formula 2 may be any one of the following compounds N-1 to N-276:

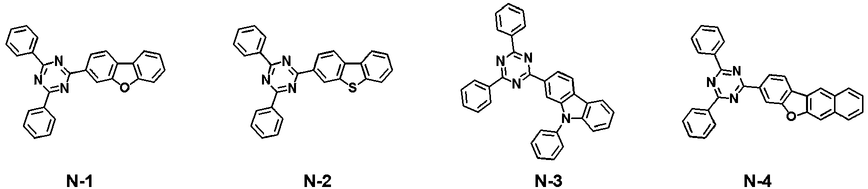

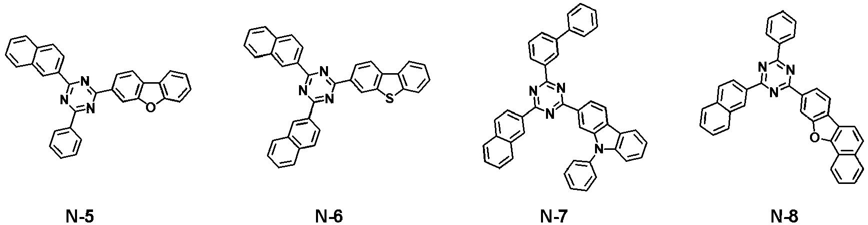

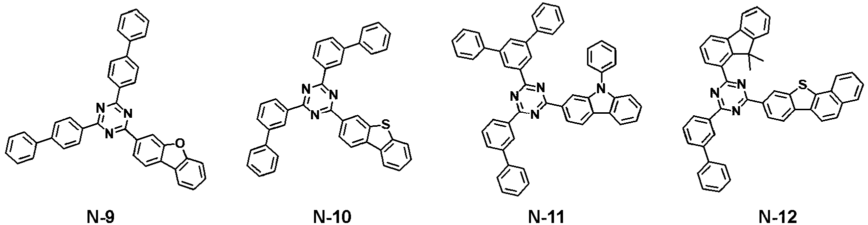

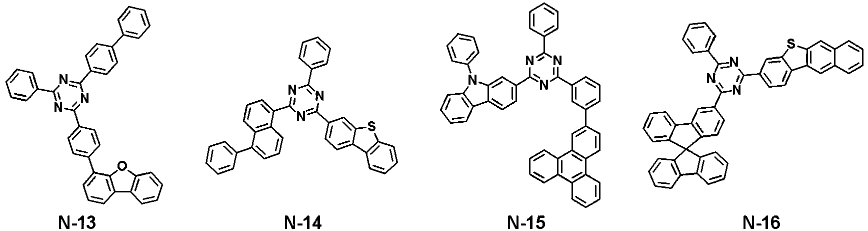

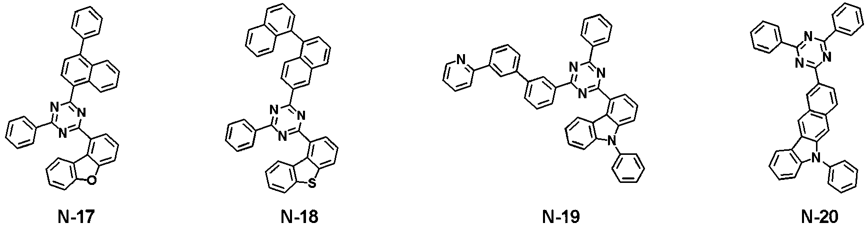

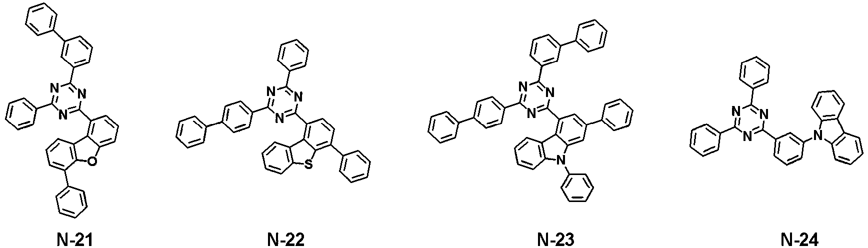

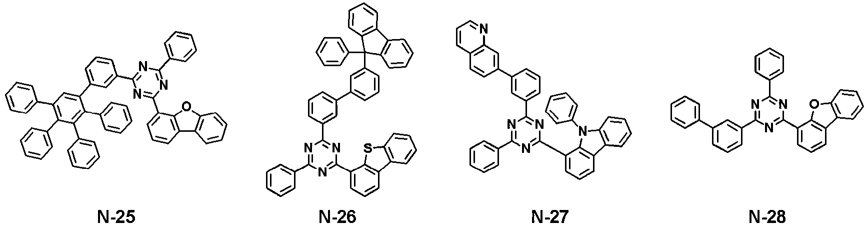

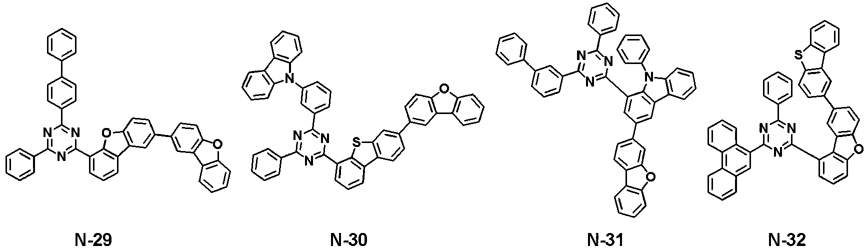

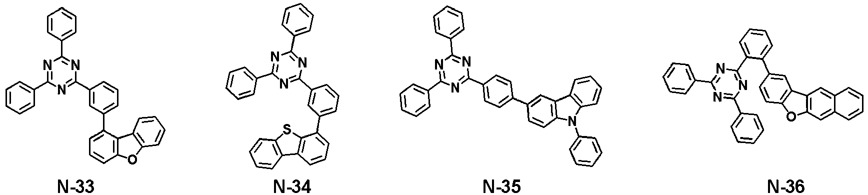

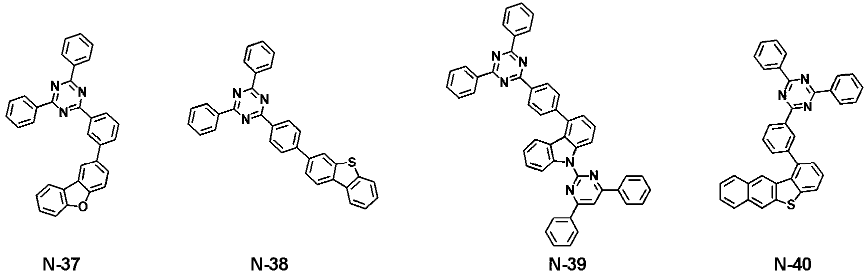

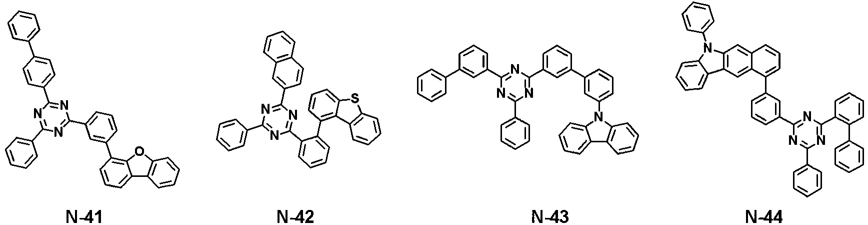

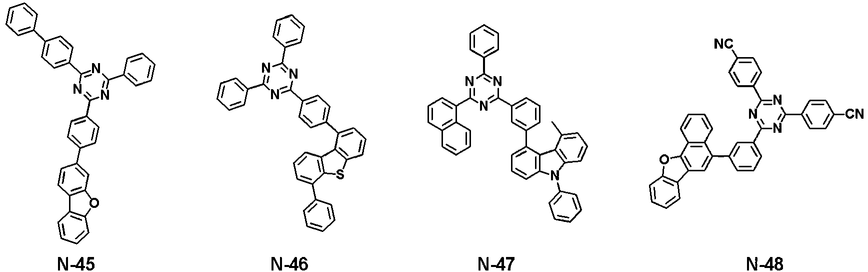

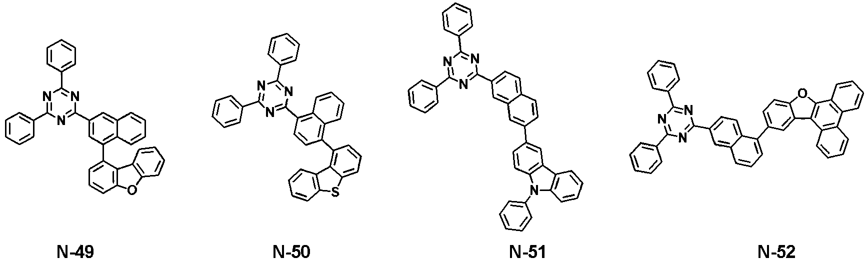

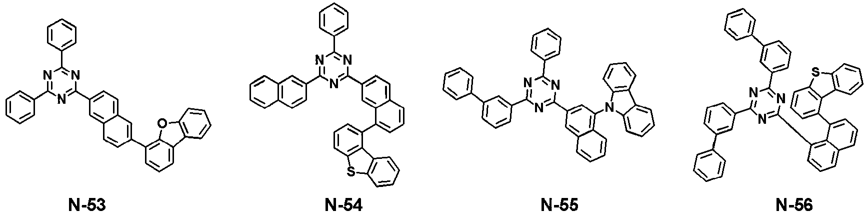

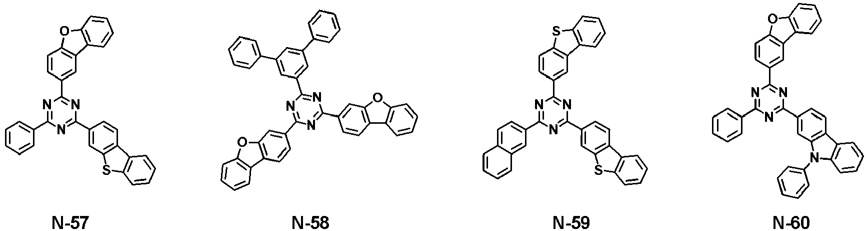

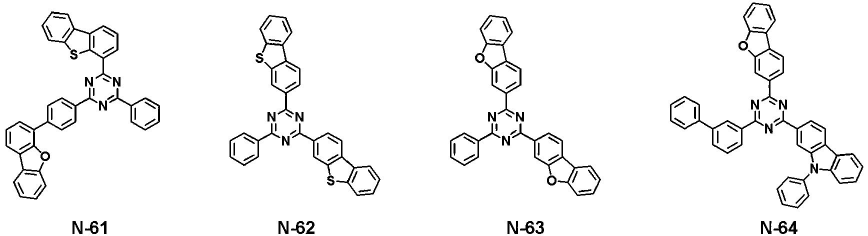

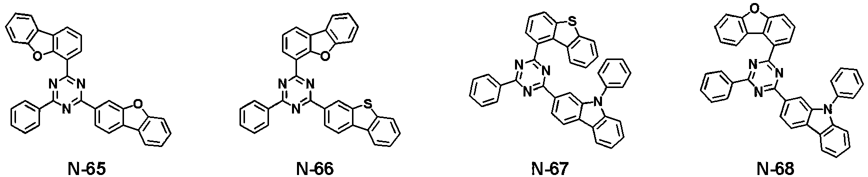

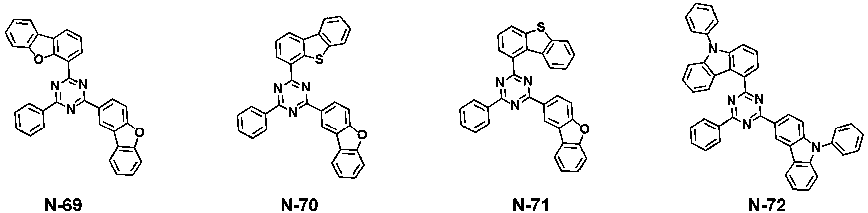

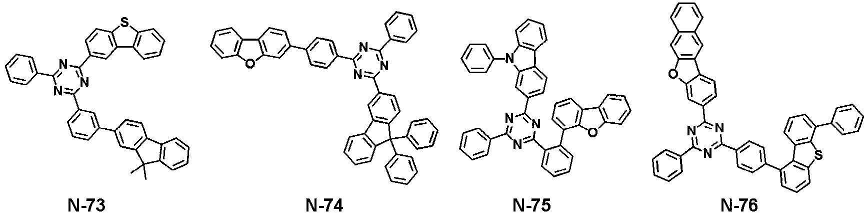

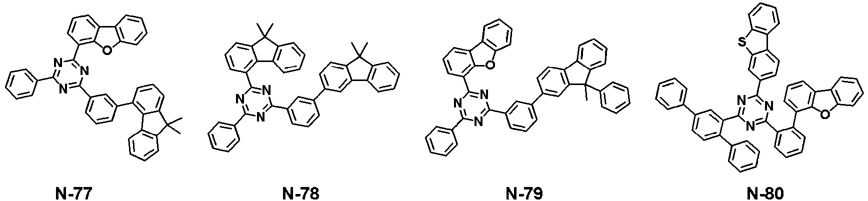

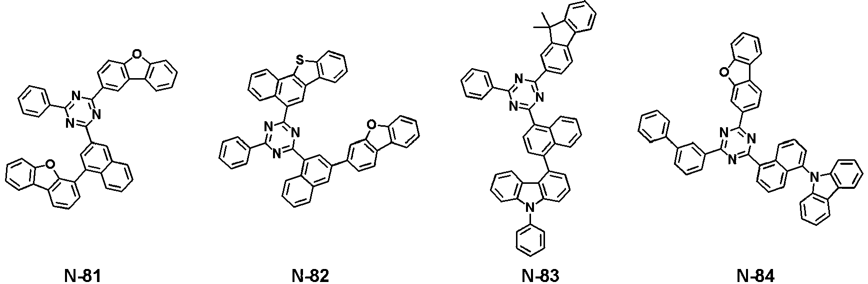

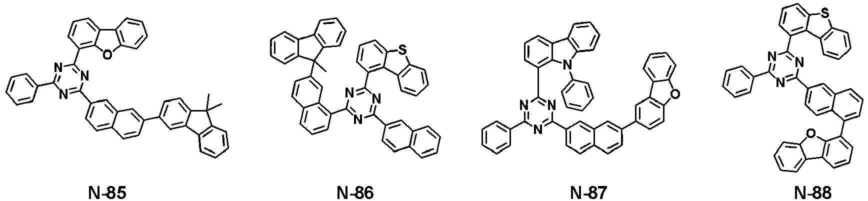

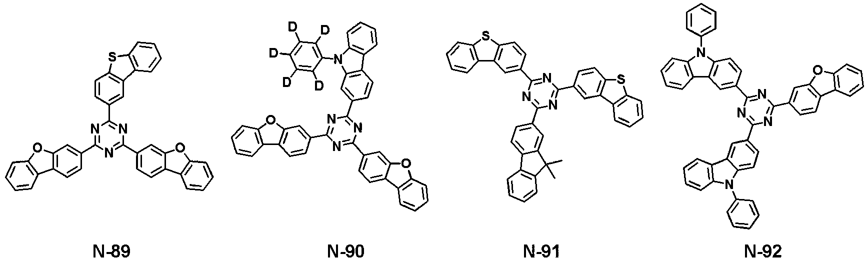

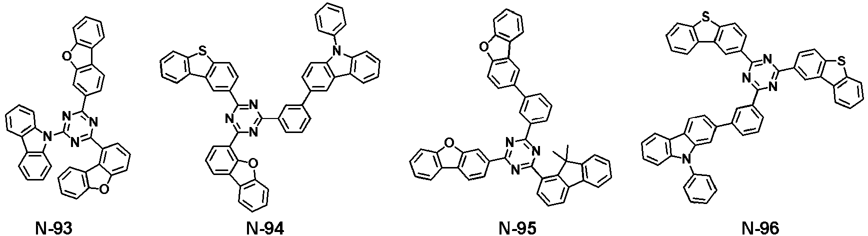

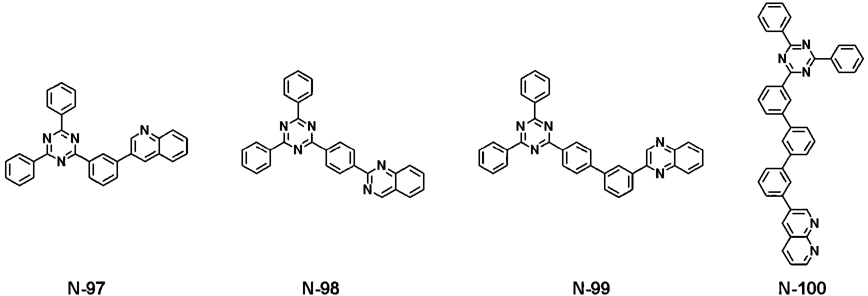

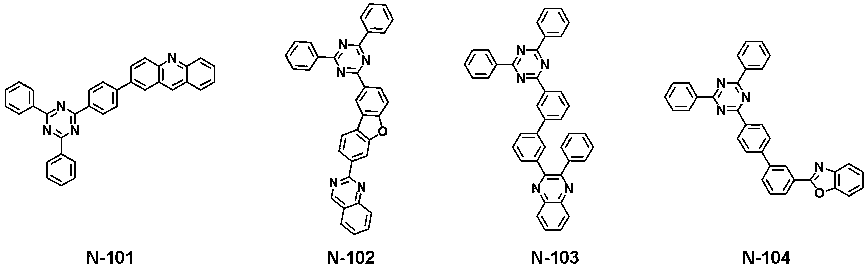

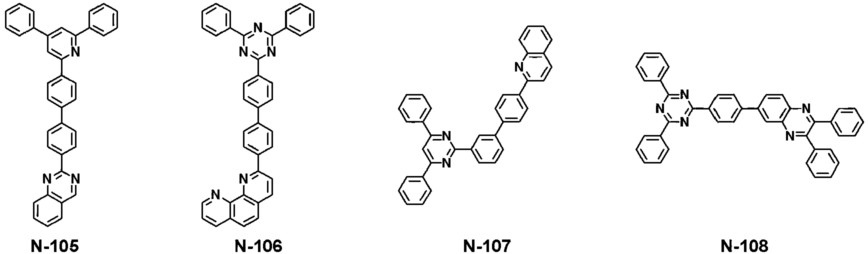

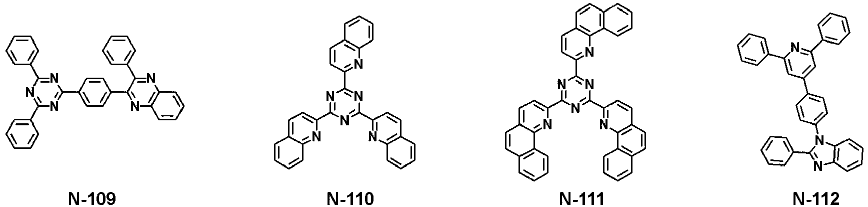

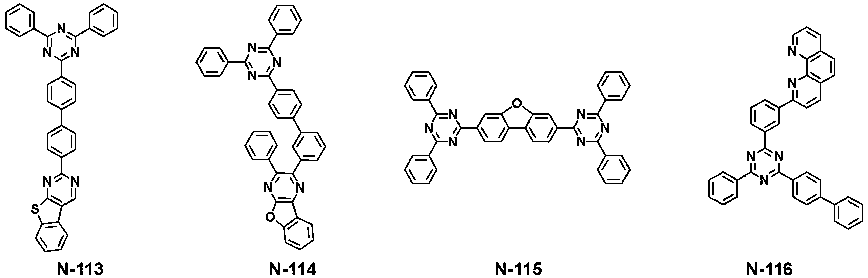

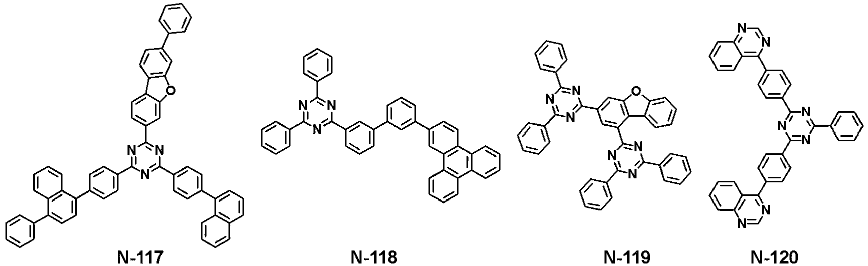

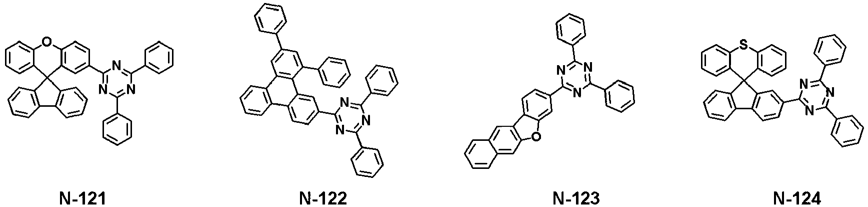

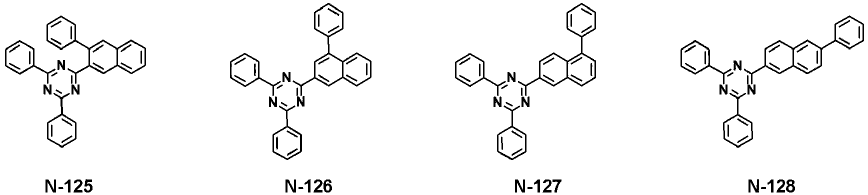

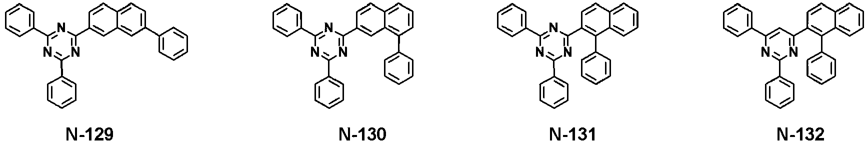

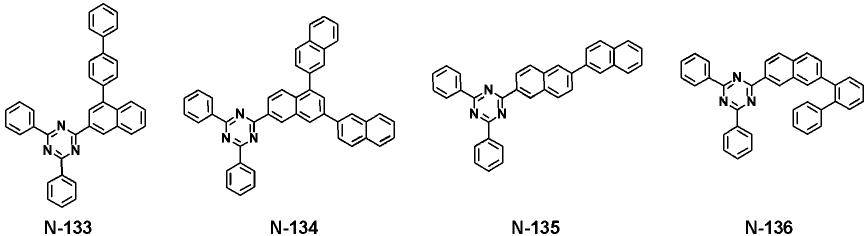

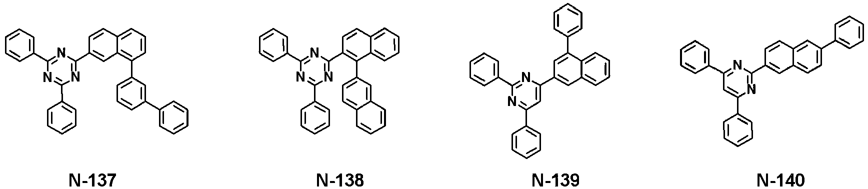

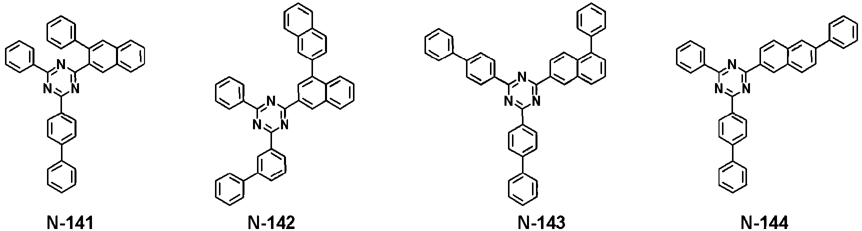

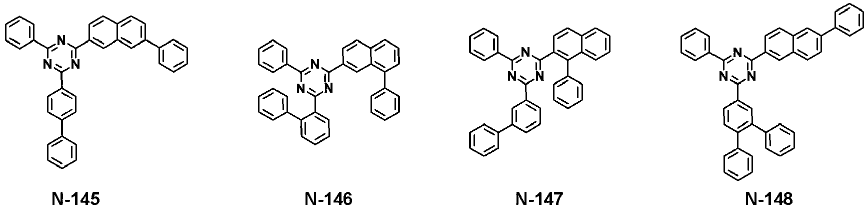

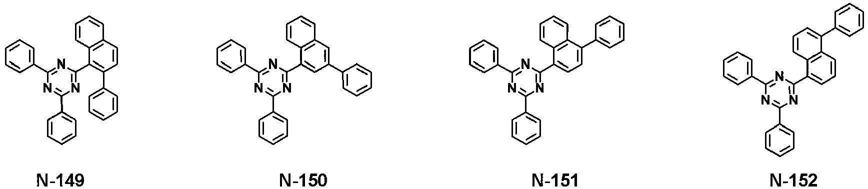

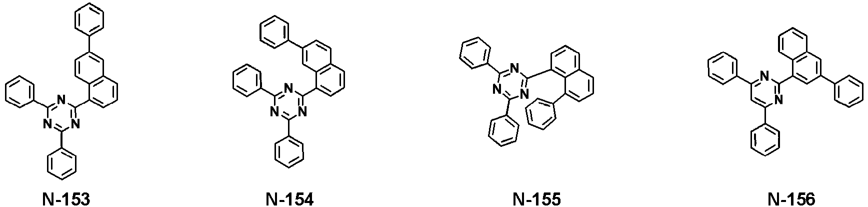

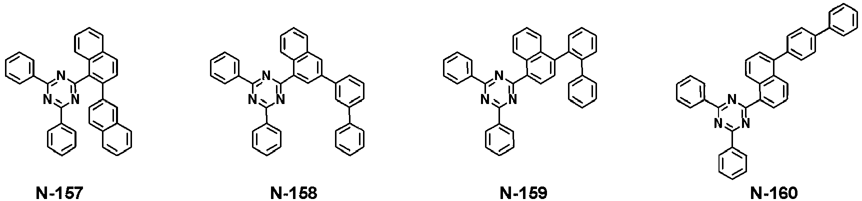

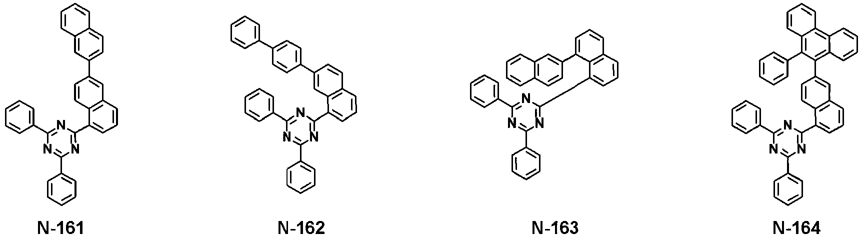

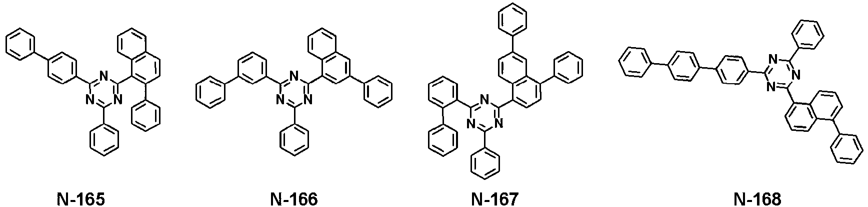

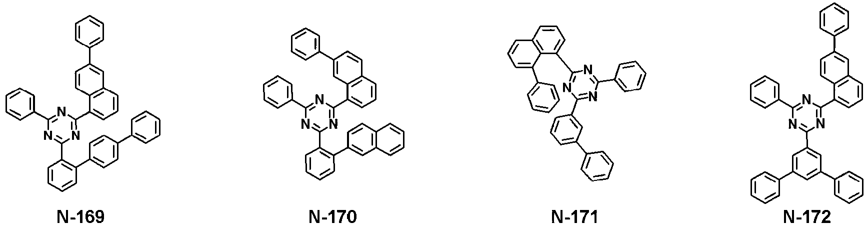

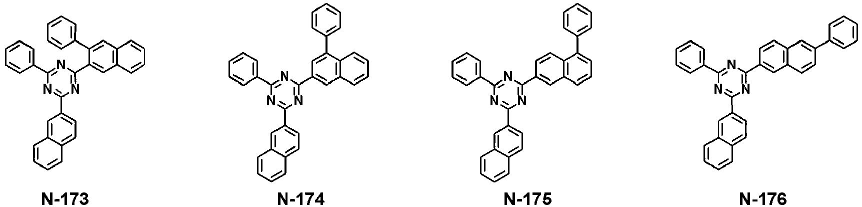

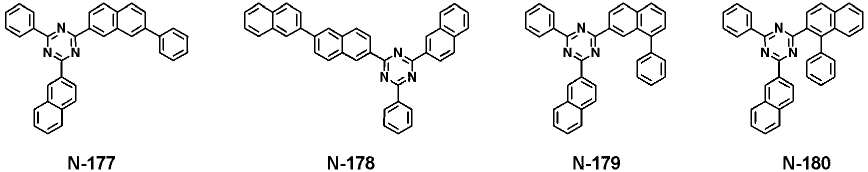

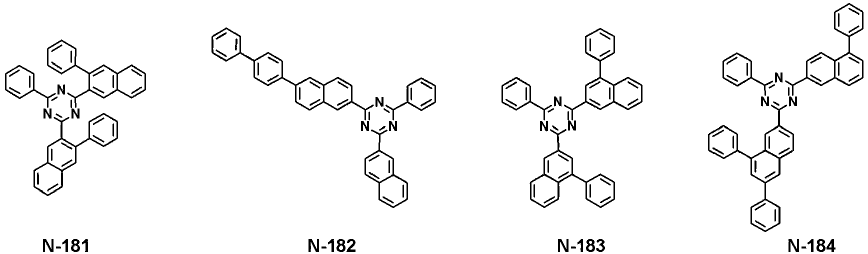

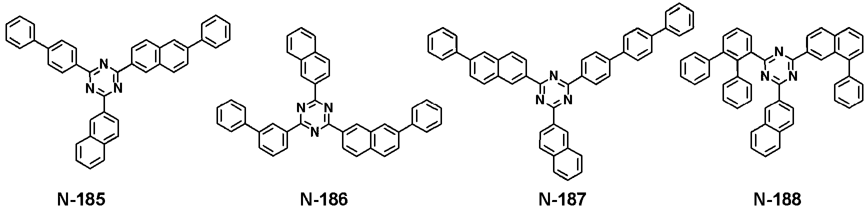

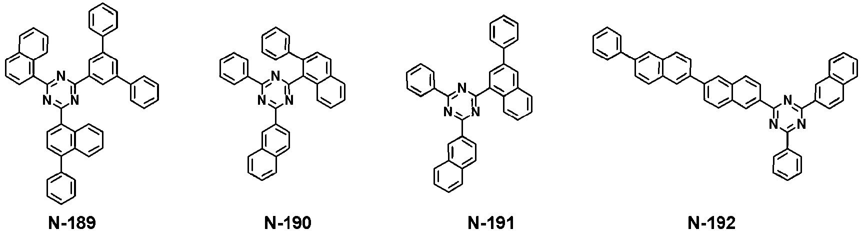

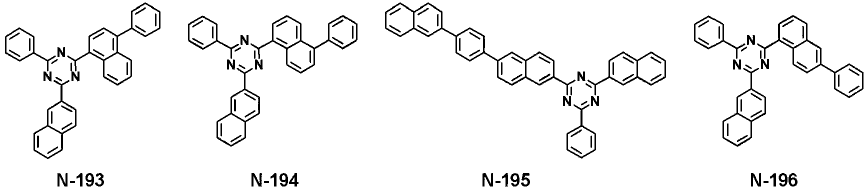

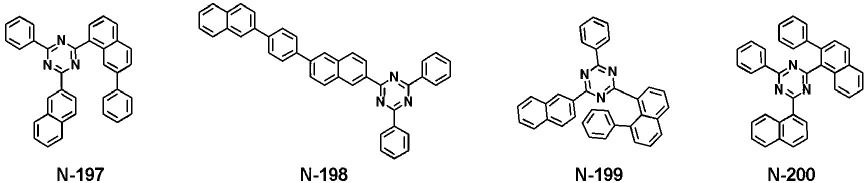

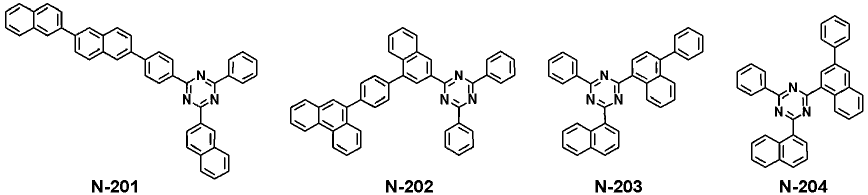

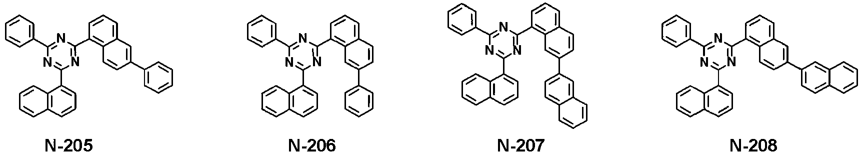

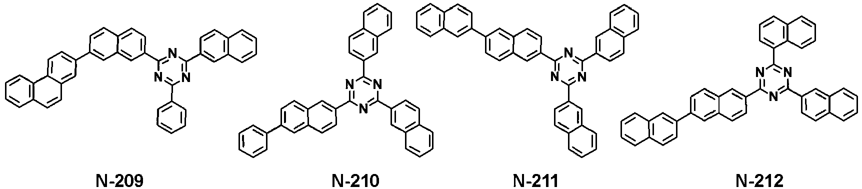

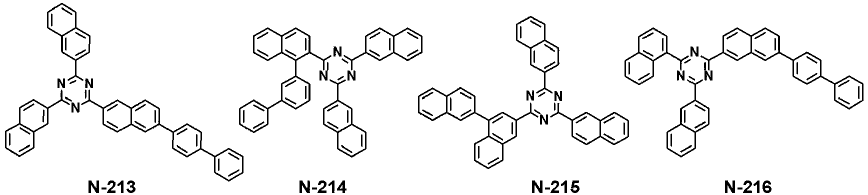

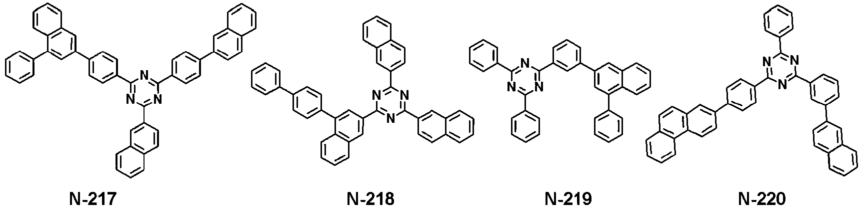

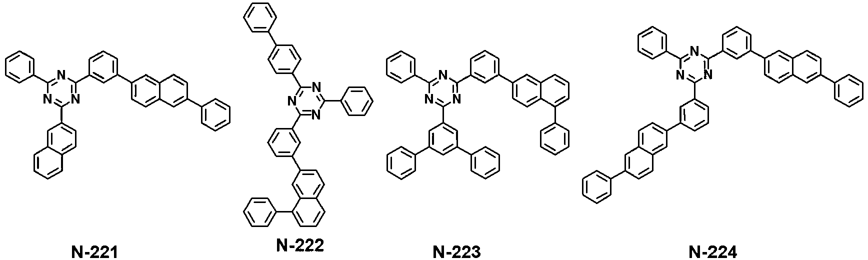

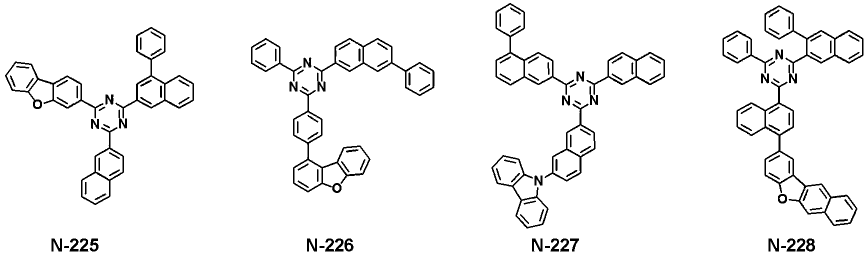

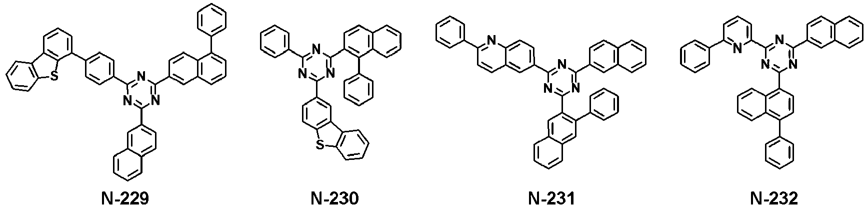

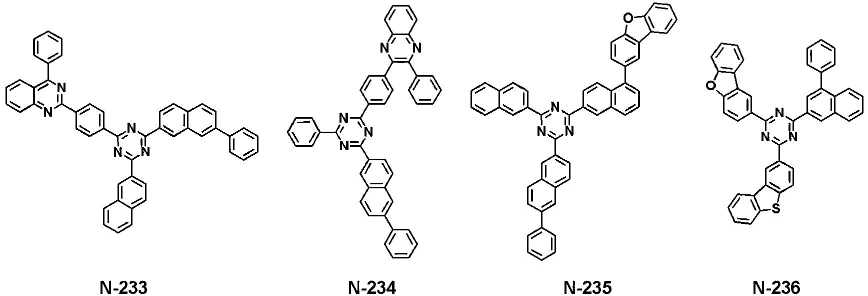

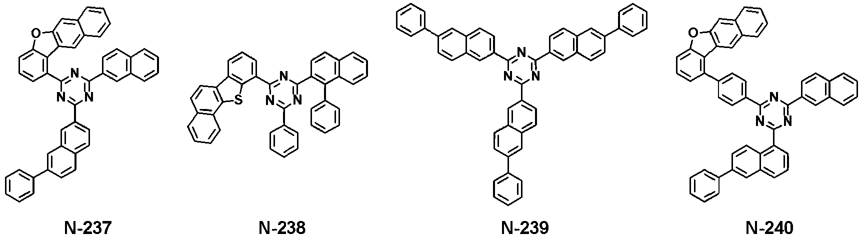

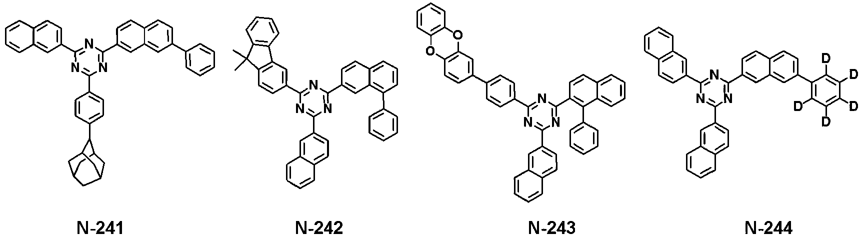

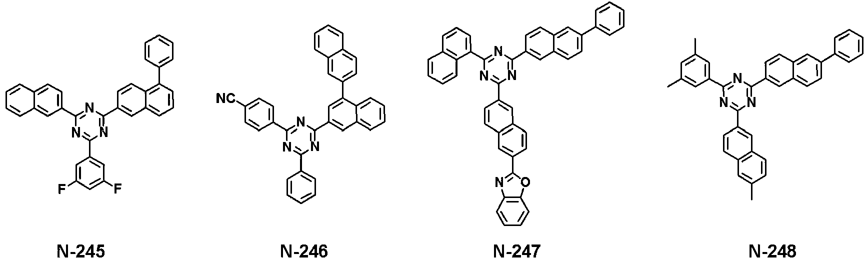

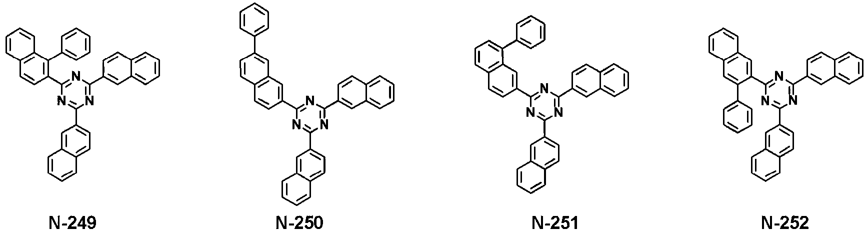

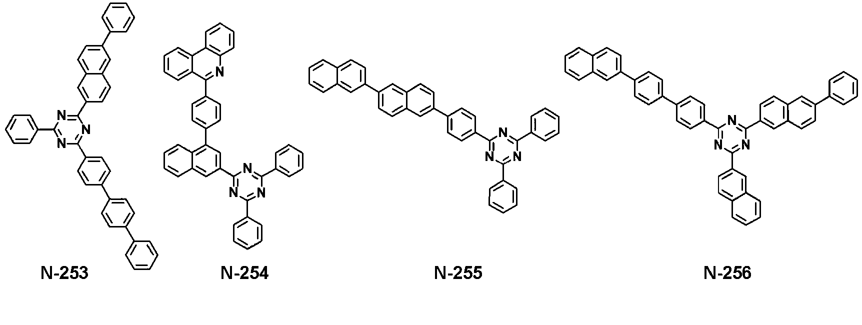

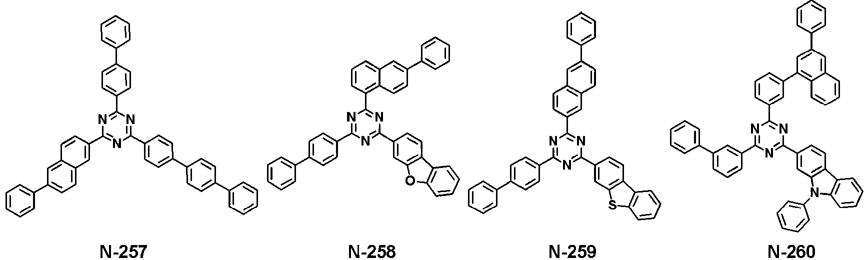

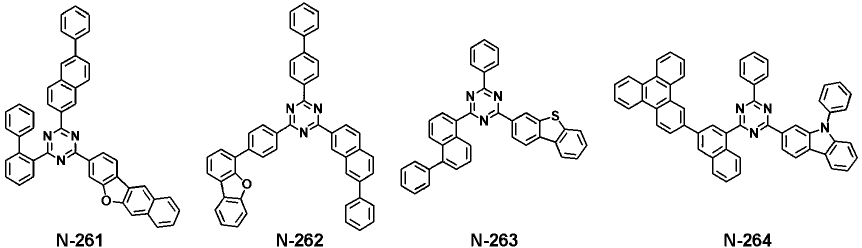

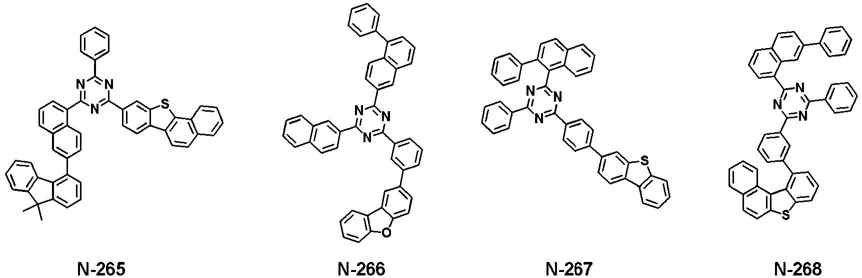

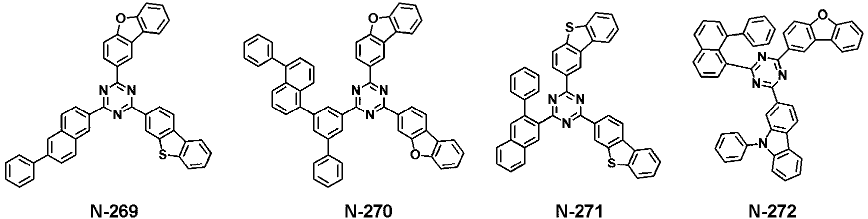

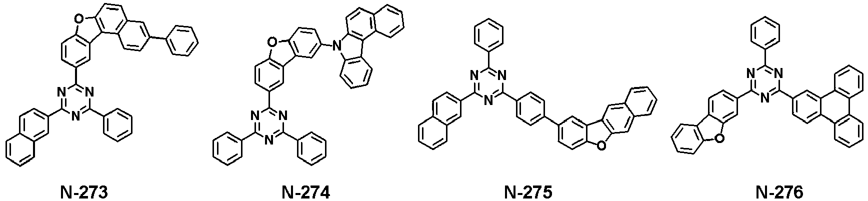

8. The composition for a phosphorescent light-emitting layer of an organic electronic element according to claim 5, wherein the composition is a host for the emitting layer.

9. An organic electronic element comprising a first electrode, a second electrode and an organic material layer between the first electrode and the second electrode; wherein the organic material layer comprises a compound of claim 1 or a composition of claim 5.

10. The organic electronic element according to claim 9, further comprising a light efficiency enhancing layer formed on at least one surface of the first electrode and the second electrode, the surface being opposite to the organic material layer.

11. The organic electronic element according to claim 9, wherein the organic material layer comprises 2 or more stacks including a hole transport layer, an emitting layer, and an electron transport layer sequentially formed on a first electrode.

12. The organic electronic element according to claim 11, wherein the organic material layer further comprises a charge generation layer formed between the 2 or more stacks.

13. An electronic device comprising a display device comprising the organic electronic element of claim 9; and a control unit for driving the display device.

14. The electronic device according to claim 13, wherein the organic electronic element is at least one of an OLED, an organic solar cell, an organic photo conductor(OPC), organic transistor (organic TFT) and an element for monochromic or white illumination.

15. A method for reusing Formula 1 of claim 1 comprising:
recovering a crude organic light emitting material comprising Formula 1 of claim 1 from a deposition apparatus used in the process for depositing the organic emitting material to prepare an organic an organic light emitting device;
removing impurities from the crude organic light emitting material;
recovering the organic light emitting material after the impurities are removed; and
purifying the recovered organic light emitting material to have a purity of 99.9% or higher.
